# EUROPEAN PATENT APPLICATION

(11) **EP 1 676 917 A2**
(43) Date of publication of application: **05.07.2006**
(21) Application number: 06004953.3
(22) Date of filing: 12.12.2001
(51) Int. Cl.: C12N 15/54, C12N 15/09, C12N 5/10, C07K 16/40, C12Q 1/48, A61K 38/45, A61K 39/00, A61K 48/00

(54) **Novel human kinases and uses thereof**

(30) Priority: 12.12.2000 US 255103 P; 08.05.2001 US 289422 P
(62) Divisional of application: 01992174.1
(71) Applicant: Lexicon Genetics Incorporated, The Woodlands, TX 77381-1160 (US)
(72) Inventor: Turner, C. Alexander, Jnr, The Woodlands TX 77381 (US); Mathur, Brian, Nashville TN 37203 (US); Friddle, Carl Johan, The Woodlands TX 77382 (US)
(74) Representative: Hewson, Timothy John

(57) **Abstract**

Novel human polynucleotide and polypeptide sequences are disclosed that can be used in therapeutic, diagnostic, and pharmacogenomic applications.

SEQUENCE LISTING

<110> LEXICON GENETICS INCORPORATED

<120> Novel Human Kinases and Polynucleotides Encoding the Same

<130> 9525(1)/JSvn/TJH

<150> US 60/255,103
<151> 2000-12-12

<150> US 60/289,422
<151> 2001-05-08

<160> 40

<170> FastSEQ for Windows Version 4.0

<210> 1
<211> 2613
<212> DNA
<213> homo sapiens

<400> 1
atgtcagggc tggtgctgat gctggcggcg cggtgcattg tgggcagctc cccgctctgc 60
cgctgccgcc gccgtcgccc aaggaggatc ggggccgggc cgggccggga tgatccgggt 120
cggaaggccg ccgccgccgg agggagcggg tcacccaacg ccgcactgag ccgcccccgc 180
cccgccccgg ccccggggga tgcgccgccc cgagctgctg cctccgccgc cgccgcagcc 240
gcagccgcag cgggcacaga gcaggtagat ggccccctca gggcaggccc ggcggacacc 300
cctccctctg gctggcggat gcagtgccta gcggccgccc ttaaggacga aaccaacatg 360
agtgggggag gggagcaggc cgacatcctg ccggccaact acgtggtcaa ggatcgctgg 420
aaggtgctga aaaagatcgg gggcgggggc tttggtgaga tctacgaggc catggacctg 480
ctgaccaggg agaatgtggc cctcaaggtg gagtcagccc agcagcccaa gcaggtcctc 540
aagatggagg tggccgtgct caagaagttg caagggaagg accatgtgtg caggttcatt 600
ggctgtggca ggaacgagaa gtttaactat gtagtgatgc agctccaggg ccggaacctg 660
gccgacctgc gccgtagcca gccgcgaggc accttcacgc tgagcaccac attgcggctg 720
ggcaagcaga tcttggagtc catcgaggcc atccactctg tgggcttcct gcaccgtgac 780
atcaagcctt caaactttgc catgggcagg ctgccctcca cctacaggaa gtgctatatg 840
ctggacttcg ggctggcccg gcagtacacc aacaccacgg gggatgtgcg gccccctcgg 900
aatgtggccg ggtttcgagg aacggttcgc tatgcctcag tcaatgccca caagaaccgg 960
gagatgggcc gccacgacga cctgtggtcc ctcttctaca tgctggtgga gtttgcagtg 1020
ggccagctgc cctggaggaa gatcaaggac aaggaacagg tagggatgat caaggagaag 1080
tatgagcacc ggatgctgct gaagcacatg ccgtcagagt tccacctctt cctggaccac 1140
attgccagcc tcgactactt caccaagccc gactaccagt tgatcatgtc agtgtttgag 1200
aacagcatga aggagagggg cattgccgag aatgaggcct ttgactggga gaaggcaggc 1260
accgatgccc tcctgtccac gagcacctct accccgcccc agcagaacac ccggcagacg 1320
gcagccatgt ttggggtggt caatgtgacg ccagtgcctg gggacctgct ccgggagaac 1380
accgaggatg tgctacaggg agagcacctg agtgaccagg agaatgcacc cccaattctg 1440
cccgggaggc cctctgaggg gctgggcccc agtccccacc ttgtccccca ccccgggggt 1500
cctgaggctg aagtctggga ggagacagat gtcaaccgga acaaactccg gatcaacatc 1560
ggcaaaagcc cctgtgtgga ggaggaacag agccgaggca tgggggtccc cagctcccca 1620
gtgcgtgccc ccccagactc ccccacaacc ccagtccgtt ctctgcgcta ccggagggtg 1680
aacagccctg agtcagaaag gctgtccacg gcggacgggc gagtggagct acctgagagg 1740
aggtcacgga tggatctgcc tggctcgccc tcgcgccagg cctgctcctc tcagccagcc 1800
cagatgctgt cagtggacac aggccacgct gaccgacagg ccagtggccg catggaygtg 1860
tcagcctctg tggagcagga ggccctgagc aacgccttcc gctcggtgcc gctggctgag 1920
gaggaggatt tcgacagcaa agagtgggtc atcatcgaca aggagacgga gctcaaggac 1980
ttccctccag gggctgagcc cagcacatcg ggcaccacgg atgaggagcc cgaggagctg 2040
cggccactgc ccgaggaggg cgaagagcgg cggcggctgg gggcagagcc caccgtccgg 2100
ccccggggac gcagcatgca ggcgctggcg gaggaggacc tgcagcattt gccgccccag 2160
cccctgccac cccagctgag ccaggscgat ggccgttccg agacgtcaca gccccccacg 2220
cctggcagcc cttcccactc acccctgcac tcgggacccc gccctcgacg gagagagtcg 2280
gaccccacag gcccacagag acagttggag gaggacagac tctcggggca ctccctcccg 2340
cggtacagcc ccctgcgacg actggcgtcc tccgtgttct cctcctccac gctggagacg 2400
gagcattacc ctcaccccgg cggcggcggc tcctcgggct cctccggttc cctcattcag 2460
cgcagccgct cggctgagag cagccctgtg cgggcgcccc accggcgcca cgcgcccctc 2520

gctgctggca accacagact catgccctcg gtgctccgca tctcgcggtc ccagctgcag 2580
caggtgtggg cccggttcac ccacaagacc tag 2613

<210> 2
<211> 870
<212> PRT
<213> homo sapiens

<220>
<221> VARIANT
<222> (1)...(870)
<223> Xaa = Any Amino Acid

<400> 2
Met Ser Gly Leu Val Leu Met Leu Ala Ala Arg Cys Ile Val Gly Ser
1               5                   10                  15
Ser Pro Leu Cys Arg Cys Arg Arg Arg Arg Pro Arg Arg Ile Gly Ala
            20                  25                  30
Gly Pro Gly Arg Asp Asp Pro Gly Arg Lys Ala Ala Ala Ala Gly Gly
        35                  40                  45
Ser Gly Ser Pro Asn Ala Ala Leu Ser Arg Pro Arg Pro Ala Pro Ala
    50                  55                  60
Pro Gly Asp Ala Pro Pro Arg Ala Ala Ala Ser Ala Ala Ala Ala Ala
65                  70                  75                  80
Ala Ala Ala Ala Gly Thr Glu Gln Val Asp Gly Pro Leu Arg Ala Gly
                85                  90                  95
Pro Ala Asp Thr Pro Pro Ser Gly Trp Arg Met Gln Cys Leu Ala Ala
            100                 105                 110
Ala Leu Lys Asp Glu Thr Asn Met Ser Gly Gly Gly Glu Gln Ala Asp
        115                 120                 125
Ile Leu Pro Ala Asn Tyr Val Val Lys Asp Arg Trp Lys Val Leu Lys
    130                 135                 140
Lys Ile Gly Gly Gly Gly Phe Gly Glu Ile Tyr Glu Ala Met Asp Leu
145                 150                 155                 160
Leu Thr Arg Glu Asn Val Ala Leu Lys Val Glu Ser Ala Gln Gln Pro
                165                 170                 175
Lys Gln Val Leu Lys Met Glu Val Ala Val Leu Lys Lys Leu Gln Gly
            180                 185                 190
Lys Asp His Val Cys Arg Phe Ile Gly Cys Gly Arg Asn Glu Lys Phe
        195                 200                 205
Asn Tyr Val Val Met Gln Leu Gln Gly Arg Asn Leu Ala Asp Leu Arg
    210                 215                 220
Arg Ser Gln Pro Arg Gly Thr Phe Thr Leu Ser Thr Thr Leu Arg Leu
225                 230                 235                 240
Gly Lys Gln Ile Leu Glu Ser Ile Glu Ala Ile His Ser Val Gly Phe
                245                 250                 255
Leu His Arg Asp Ile Lys Pro Ser Asn Phe Ala Met Gly Arg Leu Pro
            260                 265                 270
Ser Thr Tyr Arg Lys Cys Tyr Met Leu Asp Phe Gly Leu Ala Arg Gln
        275                 280                 285
Tyr Thr Asn Thr Thr Gly Asp Val Arg Pro Pro Arg Asn Val Ala Gly
    290                 295                 300
Phe Arg Gly Thr Val Arg Tyr Ala Ser Val Asn Ala His Lys Asn Arg
305                 310                 315                 320
Glu Met Gly Arg His Asp Asp Leu Trp Ser Leu Phe Tyr Met Leu Val
                325                 330                 335
Glu Phe Ala Val Gly Gln Leu Pro Trp Arg Lys Ile Lys Asp Lys Glu
            340                 345                 350
Gln Val Gly Met Ile Lys Glu Lys Tyr Glu His Arg Met Leu Leu Lys
        355                 360                 365
His Met Pro Ser Glu Phe His Leu Phe Leu Asp His Ile Ala Ser Leu
    370                 375                 380
Asp Tyr Phe Thr Lys Pro Asp Tyr Gln Leu Ile Met Ser Val Phe Glu
385                 390                 395                 400
Asn Ser Met Lys Glu Arg Gly Ile Ala Glu Asn Glu Ala Phe Asp Trp
                405                 410                 415
Glu Lys Ala Gly Thr Asp Ala Leu Leu Ser Thr Ser Thr Ser Thr Pro
            420                 425                 430

Pro Gln Gln Asn Thr Arg Gln Thr Ala Ala Met Phe Gly Val Val Asn
        435                 440                 445
Val Thr Pro Val Pro Gly Asp Leu Leu Arg Glu Asn Thr Glu Asp Val
    450                 455                 460
Leu Gln Gly Glu His Leu Ser Asp Gln Glu Asn Ala Pro Pro Ile Leu
465                 470                 475                 480
Pro Gly Arg Pro Ser Glu Gly Leu Gly Pro Ser Pro His Leu Val Pro
                485                 490                 495
His Pro Gly Gly Pro Glu Ala Glu Val Trp Glu Glu Thr Asp Val Asn
            500                 505                 510
Arg Asn Lys Leu Arg Ile Asn Ile Gly Lys Ser Pro Cys Val Glu Glu
        515                 520                 525
Glu Gln Ser Arg Gly Met Gly Val Pro Ser Ser Pro Val Arg Ala Pro
    530                 535                 540
Pro Asp Ser Pro Thr Thr Pro Val Arg Ser Leu Arg Tyr Arg Arg Val
545                 550                 555                 560
Asn Ser Pro Glu Ser Glu Arg Leu Ser Thr Ala Asp Gly Arg Val Glu
                565                 570                 575
Leu Pro Glu Arg Arg Ser Arg Met Asp Leu Pro Gly Ser Pro Ser Arg
            580                 585                 590
Gln Ala Cys Ser Ser Gln Pro Ala Gln Met Leu Ser Val Asp Thr Gly
        595                 600                 605
His Ala Asp Arg Gln Ala Ser Gly Arg Met Asp Val Ser Ala Ser Val
    610                 615                 620
Glu Gln Glu Ala Leu Ser Asn Ala Phe Arg Ser Val Pro Leu Ala Glu
625                 630                 635                 640
Glu Glu Asp Phe Asp Ser Lys Glu Trp Val Ile Ile Asp Lys Glu Thr
                645                 650                 655
Glu Leu Lys Asp Phe Pro Pro Gly Ala Glu Pro Ser Thr Ser Gly Thr
            660                 665                 670
Thr Asp Glu Glu Pro Glu Glu Leu Arg Pro Leu Pro Glu Glu Gly Glu
        675                 680                 685
Glu Arg Arg Arg Leu Gly Ala Glu Pro Thr Val Arg Pro Arg Gly Arg
    690                 695                 700
Ser Met Gln Ala Leu Ala Glu Glu Asp Leu Gln His Leu Pro Pro Gln
705                 710                 715                 720
Pro Leu Pro Pro Gln Leu Ser Gln Xaa Asp Gly Arg Ser Glu Thr Ser
                725                 730                 735
Gln Pro Pro Thr Pro Gly Ser Pro Ser His Ser Pro Leu His Ser Gly
            740                 745                 750
Pro Arg Pro Arg Arg Arg Glu Ser Asp Pro Thr Gly Pro Gln Arg Gln
        755                 760                 765
Leu Glu Glu Asp Arg Leu Ser Gly His Ser Leu Pro Arg Tyr Ser Pro
    770                 775                 780
Leu Arg Arg Leu Ala Ser Ser Val Phe Ser Ser Ser Thr Leu Glu Thr
785                 790                 795                 800
Glu His Tyr Pro His Pro Gly Gly Gly Gly Ser Ser Gly Ser Ser Gly
                805                 810                 815
Ser Leu Ile Gln Arg Ser Arg Ser Ala Glu Ser Ser Pro Val Arg Ala
            820                 825                 830
Pro His Arg Arg His Ala Pro Leu Ala Ala Gly Asn His Arg Leu Met
        835                 840                 845
Pro Ser Val Leu Arg Ile Ser Arg Ser Gln Leu Gln Gln Val Trp Ala
    850                 855                 860
Arg Phe Thr His Lys Thr
865                 870


<210> 3
<211> 2595
<212> DNA
<213> homo sapiens

<400> 3
atgctggcgg cgcggtgcat tgtgggcagc tccccgctct gccgctgccg ccgccgtcgc 60
ccaaggagga tcggggccgg gccgggccgg gatgatccgg gtcggaaggc cgccgccgcc 120
ggagggagcg ggtcacccaa cgccgcactg agccgccccc gccccgcccc ggccccgggg 180
gatgcgccgc cccgagctgc tgcctccgcc gccgccgcag ccgcagccgc agcgggcaca 240

gagcaggtag atggccccct cagggcaggc ccggcggaca cccctccctc tggctggcgg 300
atgcagtgcc tagcggccgc ccttaaggac gaaaccaaca tgagtggggg aggggagcag 360
gccgacatcc tgccggccaa ctacgtggtc aaggatcgct ggaaggtgct gaaaaagatc 420
gggggcgggg gctttggtga gatctacgag gccatggacc tgctgaccag ggagaatgtg 480
gccctcaagg tggagtcagc ccagcagccc aagcaggtcc tcaagatgga ggtggccgtg 540
ctcaagaagt tgcaagggaa ggaccatgtg tgcaggttca ttggctgtgg caggaacgag 600
aagtttaact atgtagtgat gcagctccag ggccggaacc tggccgacct gcgccgtagc 660
cagccgcgag gcaccttcac gctgagcacc acattgcggc tgggcaagca gatcttggag 720
tccatcgagg ccatccactc tgtgggcttc ctgcaccgtg acatcaagcc ttcaaacttt 780
gccatgggca ggctgccctc cacctacagg aagtgctata tgctggactt cgggctggcc 840
cggcagtaca ccaacaccac gggggatgtg cggccccctc ggaatgtggc cgggtttcga 900
ggaacggttc gctatgcctc agtcaatgcc cacaagaacc gggagatggg ccgccacgac 960
gacctgtggt ccctcttcta catgctggtg gagtttgcag tgggccagct gccctggagg 1020
aagatcaagg acaaggaaca ggtagggatg atcaaggaga agtatgagca ccggatgctg 1080
ctgaagcaca tgccgtcaga gttccacctc ttcctggacc acattgccag cctcgactac 1140
ttcaccaagc ccgactacca gttgatcatg tcagtgtttg agaacagcat gaaggagagg 1200
ggcattgccg agaatgaggc ctttgactgg gagaaggcag gcaccgatgc cctcctgtcc 1260
acgagcacct ctaccccgcc ccagcagaac acccggcaga cggcagccat gtttgggtg 1320
gtcaatgtga cgccagtgcc tggggacctg ctccgggaga acaccgagga tgtgctacag 1380
ggagagcacc tgagtgacca ggagaatgca cccccaattc tgcccgggag gccctctgag 1440
gggctgggcc ccagtcccca ccttgtcccc caccccgggg gtcctgaggc tgaagtctgg 1500
gaggagacag atgtcaaccg gaacaaactc cggatcaaca tcggcaaaag cccctgtgtg 1560
gaggaggaac agagccgagg catgggggtc cccagctccc cagtgcgtgc ccccccagac 1620
tcccccacaa ccccagtccg ttctctgcgc taccggaggg tgaacagccc tgagtcagaa 1680
aggctgtcca cggcggacgg gcgagtggag ctacctgaga ggaggtcacg gatggatctg 1740
cctggctcgc cctcgcgcca ggcctgctcc tctcagccag cccagatgct gtcagtggac 1800
acaggccacg ctgaccgaca ggccagtggc cgcatggayg tgtcagcctc tgtggagcag 1860
gaggccctga gcaacgcctt ccgctcggtg ccgctggctg aggaggagga tttcgacagc 1920
aaagagtggg tcatcatcga caaggagacg gagctcaagg acttccctcc aggggctgag 1980
cccagcacat cgggcaccac ggatgaggag cccgaggagc tgcggccact gcccgaggag 2040
ggcgaagagc ggcggcggct gggggcagag cccaccgtcc ggccccgggg acgcagcatg 2100
caggcgctgg cggaggagga cctgcagcat ttgccgcccc agcccctgcc accccagctg 2160
agccaggscg atggccgttc cgagacgtca cagcccccca cgcctggcag cccttcccac 2220
tcacccctgc actcggaccc ccgccctcga cggagagagt cggaccccac aggcccacag 2280
agacagttgg aggaggacag actctcgggg cactccctcc cgcggtacag ccccctgcga 2340
cgactggcgt cctccgtgtt ctcctcctcc acgctggaga cggagcatta ccctcacccc 2400
ggcggcggcg gctcctcggg ctcctccggt tccctcattc agcgcagccg ctcggctgag 2460
agcagccctg tgcgggcgcc ccaccggcgc cacgcgcccc tcgctgctgg caaccacaga 2520
ctcatgccct cggtgctccg catctcgcgg tcccagctgc agcaggtgtg ggcccggttc 2580
acccacaaga cctag 2595

<210> 4
<211> 864
<212> PRT
<213> homo sapiens

<220>
<221> VARIANT
<222> (1)...(864)
<223> Xaa = Any Amino Acid

<400> 4
Met Leu Ala Ala Arg Cys Ile Val Gly Ser Ser Pro Leu Cys Arg Cys
1               5                   10                  15
Arg Arg Arg Arg Pro Arg Arg Ile Gly Ala Gly Pro Gly Arg Asp Asp
            20                  25                  30
Pro Gly Arg Lys Ala Ala Ala Ala Gly Gly Ser Gly Ser Pro Asn Ala
        35                  40                  45
Ala Leu Ser Arg Pro Arg Pro Ala Pro Ala Pro Gly Asp Ala Pro Pro
    50                  55                  60
Arg Ala Ala Ala Ser Ala Ala Ala Ala Ala Ala Ala Ala Ala Gly Thr
65                  70                  75                  80
Glu Gln Val Asp Gly Pro Leu Arg Ala Gly Pro Ala Asp Thr Pro Pro
                85                  90                  95
Ser Gly Trp Arg Met Gln Cys Leu Ala Ala Ala Leu Lys Asp Glu Thr
            100                 105                 110
Asn Met Ser Gly Gly Gly Glu Gln Ala Asp Ile Leu Pro Ala Asn Tyr
        115                 120                 125

Val Val Lys Asp Arg Trp Lys Val Leu Lys Lys Ile Gly Gly Gly Gly
    130                 135                 140
Phe Gly Glu Ile Tyr Glu Ala Met Asp Leu Leu Thr Arg Glu Asn Val
145                 150                 155                 160
Ala Leu Lys Val Glu Ser Ala Gln Gln Pro Lys Gln Val Leu Lys Met
                165                 170                 175
Glu Val Ala Val Leu Lys Lys Leu Gln Gly Lys Asp His Val Cys Arg
            180                 185                 190
Phe Ile Gly Cys Gly Arg Asn Glu Lys Phe Asn Tyr Val Val Met Gln
        195                 200                 205
Leu Gln Gly Arg Asn Leu Ala Asp Leu Arg Arg Ser Gln Pro Arg Gly
    210                 215                 220
Thr Phe Thr Leu Ser Thr Thr Leu Arg Leu Gly Lys Gln Ile Leu Glu
225                 230                 235                 240
Ser Ile Glu Ala Ile His Ser Val Gly Phe Leu His Arg Asp Ile Lys
                245                 250                 255
Pro Ser Asn Phe Ala Met Gly Arg Leu Pro Ser Thr Tyr Arg Lys Cys
            260                 265                 270
Tyr Met Leu Asp Phe Gly Leu Ala Arg Gln Tyr Thr Asn Thr Thr Gly
        275                 280                 285
Asp Val Arg Pro Pro Arg Asn Val Ala Gly Phe Arg Gly Thr Val Arg
    290                 295                 300
Tyr Ala Ser Val Asn Ala His Lys Asn Arg Glu Met Gly Arg His Asp
305                 310                 315                 320
Asp Leu Trp Ser Leu Phe Tyr Met Leu Val Glu Phe Ala Val Gly Gln
                325                 330                 335
Leu Pro Trp Arg Lys Ile Lys Asp Lys Glu Gln Val Gly Met Ile Lys
            340                 345                 350
Glu Lys Tyr Glu His Arg Met Leu Leu Lys His Met Pro Ser Glu Phe
        355                 360                 365
His Leu Phe Leu Asp His Ile Ala Ser Leu Asp Tyr Phe Thr Lys Pro
    370                 375                 380
Asp Tyr Gln Leu Ile Met Ser Val Phe Glu Asn Ser Met Lys Glu Arg
385                 390                 395                 400
Gly Ile Ala Glu Asn Glu Ala Phe Asp Trp Glu Lys Ala Gly Thr Asp
                405                 410                 415
Ala Leu Leu Ser Thr Ser Thr Ser Thr Pro Pro Gln Gln Asn Thr Arg
            420                 425                 430
Gln Thr Ala Ala Met Phe Gly Val Val Asn Val Thr Pro Val Pro Gly
        435                 440                 445
Asp Leu Leu Arg Glu Asn Thr Glu Asp Val Leu Gln Gly Glu His Leu
    450                 455                 460
Ser Asp Gln Glu Asn Ala Pro Pro Ile Leu Pro Gly Arg Pro Ser Glu
465                 470                 475                 480
Gly Leu Gly Pro Ser Pro His Leu Val Pro His Pro Gly Gly Pro Glu
                485                 490                 495
Ala Glu Val Trp Glu Glu Thr Asp Val Asn Arg Asn Lys Leu Arg Ile
            500                 505                 510
Asn Ile Gly Lys Ser Pro Cys Val Glu Glu Glu Gln Ser Arg Gly Met
        515                 520                 525
Gly Val Pro Ser Ser Pro Val Arg Ala Pro Pro Asp Ser Pro Thr Thr
    530                 535                 540
Pro Val Arg Ser Leu Arg Tyr Arg Arg Val Asn Ser Pro Glu Ser Glu
545                 550                 555                 560
Arg Leu Ser Thr Ala Asp Gly Arg Val Glu Leu Pro Glu Arg Arg Ser
                565                 570                 575
Arg Met Asp Leu Pro Gly Ser Pro Ser Arg Gln Ala Cys Ser Ser Gln
            580                 585                 590
Pro Ala Gln Met Leu Ser Val Asp Thr Gly His Ala Asp Arg Gln Ala
        595                 600                 605
Ser Gly Arg Met Asp Val Ser Ala Ser Val Glu Gln Glu Ala Leu Ser
    610                 615                 620
Asn Ala Phe Arg Ser Val Pro Leu Ala Glu Glu Glu Asp Phe Asp Ser
625                 630                 635                 640
Lys Glu Trp Val Ile Ile Asp Lys Glu Thr Glu Leu Lys Asp Phe Pro
                645                 650                 655
Pro Gly Ala Glu Pro Ser Thr Ser Gly Thr Thr Asp Glu Glu Pro Glu
            660                 665                 670

Glu Leu Arg Pro Leu Pro Glu Glu Gly Glu Glu Arg Arg Arg Leu Gly
                675                 680                 685
Ala Glu Pro Thr Val Arg Pro Arg Gly Arg Ser Met Gln Ala Leu Ala
    690                 695                 700
Glu Glu Asp Leu Gln His Leu Pro Pro Gln Pro Leu Pro Pro Gln Leu
705                 710                 715                 720
Ser Gln Xaa Asp Gly Arg Ser Glu Thr Ser Gln Pro Pro Thr Pro Gly
                725                 730                 735
Ser Pro Ser His Ser Pro Leu His Ser Gly Pro Arg Pro Arg Arg Arg
            740                 745                 750
Glu Ser Asp Pro Thr Gly Pro Gln Arg Gln Leu Glu Glu Asp Arg Leu
        755                 760                 765
Ser Gly His Ser Leu Pro Arg Tyr Ser Pro Leu Arg Arg Leu Ala Ser
    770                 775                 780
Ser Val Phe Ser Ser Ser Thr Leu Glu Thr Glu His Tyr Pro His Pro
785                 790                 795                 800
Gly Gly Gly Gly Ser Ser Gly Ser Ser Gly Ser Leu Ile Gln Arg Ser
                805                 810                 815
Arg Ser Ala Glu Ser Ser Pro Val Arg Ala Pro His Arg Arg His Ala
            820                 825                 830
Pro Leu Ala Ala Gly Asn His Arg Leu Met Pro Ser Val Leu Arg Ile
        835                 840                 845
Ser Arg Ser Gln Leu Gln Gln Val Trp Ala Arg Phe Thr His Lys Thr
    850                 855                 860

<210> 5
<211> 2295
<212> DNA
<213> homo sapiens

<400> 5
atgcagtgcc tagcggccgc ccttaaggac gaaaccaaca tgagtggggg aggggagcag 60
gccgacatcc tgccggccaa ctacgtggtc aaggatcgct ggaaggtgct gaaaaagatc 120
gggggcgggg gctttggtga gatctacgag gccatggacc tgctgaccag ggagaatgtg 180
gccctcaagg tggagtcagc ccagcagccc aagcaggtcc tcaagatgga ggtggccgtg 240
ctcaagaagt tgcaagggaa ggaccatgtg tgcaggttca ttggctgtgg caggaacgag 300
aagtttaact atgtagtgat gcagctccag ggccggaacc tggccgacct gcgccgtagc 360
cagccgcgag gcaccttcac gctgagcacc acattgcggc tgggcaagca gatcttggag 420
tccatcgagg ccatccactc tgtgggcttc ctgcaccgtg acatcaagcc ttcaaacttt 480
gccatgggca ggctgccctc cacctacagg aagtgctata tgctggactt cgggctggcc 540
cggcagtaca ccaacaccac gggggatgtg cggccccctc ggaatgtggc cgggtttcga 600
ggaacggttc gctatgcctc agtcaatgcc cacaagaacc gggagatggg ccgccacgac 660
gacctgtggt ccctcttcta catgctggtg gagtttgcag tgggccagct gccctggagg 720
aagatcaagg acaaggaaca ggtagggatg atcaaggaga agtatgagca ccggatgctg 780
ctgaagcaca tgccgtcaga gttccacctc ttcctggacc acattgccag cctcgactac 840
ttcaccaagc ccgactacca gttgatcatg tcagtgtttg agaacagcat gaaggagagg 900
ggcattgccg agaatgaggc ctttgactgg gagaaggcag gcaccgatgc cctcctgtcc 960
acgagcacct ctaccccgcc ccagcagaac acccggcaga cggcagccat gtttggggtg 1020
gtcaatgtga cgccagtgcc tggggacctg ctccgggaga acaccgagga tgtgctacag 1080
ggagagcacc tgagtgacca ggagaatgca cccccaattc tgcccgggag gccctctgag 1140
gggctgggcc ccagtcccca ccttgtcccc caccccgggg gtcctgaggc tgaagtctgg 1200
gaggagacag atgtcaaccg gaacaaactc cggatcaaca tcggcaaaag cccctgtgtg 1260
gaggaggaac agagccgagg catgggggtc cccagctccc cagtgcgtgc ccccccagac 1320
tcccccacaa ccccagtccg ttctctgcgc taccggaggg tgaacagccc tgagtcagaa 1380
aggctgtcca cggcggacgg gcgagtggag ctacctgaga ggaggtcacg gatggatctg 1440
cctggctcgc cctcgcgcca ggcctgctcc tctcagccag cccagatgct gtcagtggac 1500
acaggccacg ctgaccgaca ggccagtggc cgcatggayg tgtcagcctc tgtggagcag 1560
gaggccctga gcaacgcctt ccgctcggtg ccgctggctg aggaggagga tttcgacagc 1620
aaagagtggg tcatcatcga caaggagacg gagctcaagg acttccctcc aggggctgag 1680
cccagcacat cgggcaccac ggatgaggag cccgaggagc tgcggccact gcccgaggag 1740
ggcgaagagc ggcggcggct gggggcagag cccaccgtcc ggccccgggg acgcagcatg 1800
caggcgctgg cggaggagga cctgcagcat ttgccgcccc agcccctgcc accccagctg 1860
agccaggscg atggccgttc cgagacgtca cagcccccca cgcctggcag cccttcccac 1920
tcacccctgc actcgggacc ccgccctcga cggagagagt cggaccccac aggcccacag 1980
agacagttgg aggaggacag actctcgggg cactccctcc cgcggtacag ccccctgcga 2040
cgactggcgt cctccgtgtt ctcctcctcc acgctggaga cggagcatta ccctcacccc 2100
ggcggcggcg gctcctcggg ctcctccggt tccctcattc agcgcagccg ctcggctgag 2160

agcagccctg tgcgggcgcc ccaccggcgc cacgcgcccc tcgctgctgg caaccacaga 2220
ctcatgccct cggtgctccg catctcgcgg tcccagctgc agcaggtgtg ggcccggttc 2280
acccacaaga cctag 2295

<210> 6
<211> 764
<212> PRT
<213> homo sapiens

<220>
<221> VARIANT
<222> (1)...(764)
<223> Xaa = Any Amino Acid

<400> 6
Met Gln Cys Leu Ala Ala Ala Leu Lys Asp Glu Thr Asn Met Ser Gly
1               5                   10                  15
Gly Gly Glu Gln Ala Asp Ile Leu Pro Ala Asn Tyr Val Val Lys Asp
            20                  25                  30
Arg Trp Lys Val Leu Lys Lys Ile Gly Gly Gly Gly Phe Gly Glu Ile
        35                  40                  45
Tyr Glu Ala Met Asp Leu Leu Thr Arg Glu Asn Val Ala Leu Lys Val
    50                  55                  60
Glu Ser Ala Gln Gln Pro Lys Gln Val Leu Lys Met Glu Val Ala Val
65                  70                  75                  80
Leu Lys Lys Leu Gln Gly Lys Asp His Val Cys Arg Phe Ile Gly Cys
                85                  90                  95
Gly Arg Asn Glu Lys Phe Asn Tyr Val Val Met Gln Leu Gln Gly Arg
            100                 105                 110
Asn Leu Ala Asp Leu Arg Arg Ser Gln Pro Arg Gly Thr Phe Thr Leu
        115                 120                 125
Ser Thr Thr Leu Arg Leu Gly Lys Gln Ile Leu Glu Ser Ile Glu Ala
    130                 135                 140
Ile His Ser Val Gly Phe Leu His Arg Asp Ile Lys Pro Ser Asn Phe
145                 150                 155                 160
Ala Met Gly Arg Leu Pro Ser Thr Tyr Arg Lys Cys Tyr Met Leu Asp
                165                 170                 175
Phe Gly Leu Ala Arg Gln Tyr Thr Asn Thr Thr Gly Asp Val Arg Pro
            180                 185                 190
Pro Arg Asn Val Ala Gly Phe Arg Gly Thr Val Arg Tyr Ala Ser Val
        195                 200                 205
Asn Ala His Lys Asn Arg Glu Met Gly Arg His Asp Asp Leu Trp Ser
    210                 215                 220
Leu Phe Tyr Met Leu Val Glu Phe Ala Val Gly Gln Leu Pro Trp Arg
225                 230                 235                 240
Lys Ile Lys Asp Lys Glu Gln Val Gly Met Ile Lys Glu Lys Tyr Glu
                245                 250                 255
His Arg Met Leu Leu Lys His Met Pro Ser Glu Phe His Leu Phe Leu
            260                 265                 270
Asp His Ile Ala Ser Leu Asp Tyr Phe Thr Lys Pro Asp Tyr Gln Leu
        275                 280                 285
Ile Met Ser Val Phe Glu Asn Ser Met Lys Glu Arg Gly Ile Ala Glu
    290                 295                 300
Asn Glu Ala Phe Asp Trp Glu Lys Ala Gly Thr Asp Ala Leu Leu Ser
305                 310                 315                 320
Thr Ser Thr Ser Thr Pro Pro Gln Gln Asn Thr Arg Gln Thr Ala Ala
                325                 330                 335
Met Phe Gly Val Val Asn Val Thr Pro Val Pro Gly Asp Leu Leu Arg
            340                 345                 350
Glu Asn Thr Glu Asp Val Leu Gln Gly Glu His Leu Ser Asp Gln Glu
        355                 360                 365
Asn Ala Pro Pro Ile Leu Pro Gly Arg Pro Ser Glu Gly Leu Gly Pro
    370                 375                 380
Ser Pro His Leu Val Pro His Pro Gly Gly Pro Glu Ala Glu Val Trp
385                 390                 395                 400
Glu Glu Thr Asp Val Asn Arg Asn Lys Leu Arg Ile Asn Ile Gly Lys
                405                 410                 415
Ser Pro Cys Val Glu Glu Glu Gln Ser Arg Gly Met Gly Val Pro Ser

420                     425                     430
Ser Pro Val Arg Ala Pro Pro Asp Ser Pro Thr Thr Pro Val Arg Ser
        435                     440                     445
Leu Arg Tyr Arg Arg Val Asn Ser Pro Glu Ser Glu Arg Leu Ser Thr
    450                     455                     460
Ala Asp Gly Arg Val Glu Leu Pro Glu Arg Arg Ser Arg Met Asp Leu
465                     470                     475                     480
Pro Gly Ser Pro Ser Arg Gln Ala Cys Ser Ser Gln Pro Ala Gln Met
                485                     490                     495
Leu Ser Val Asp Thr Gly His Ala Asp Arg Gln Ala Ser Gly Arg Met
            500                     505                     510
Asp Val Ser Ala Ser Val Glu Gln Glu Ala Leu Ser Asn Ala Phe Arg
        515                     520                     525
Ser Val Pro Leu Ala Glu Glu Glu Asp Phe Asp Ser Lys Glu Trp Val
    530                     535                     540
Ile Ile Asp Lys Glu Thr Glu Leu Lys Asp Phe Pro Pro Gly Ala Glu
545                     550                     555                     560
Pro Ser Thr Ser Gly Thr Thr Asp Glu Glu Pro Glu Glu Leu Arg Pro
                565                     570                     575
Leu Pro Glu Glu Gly Glu Glu Arg Arg Arg Leu Gly Ala Glu Pro Thr
            580                     585                     590
Val Arg Pro Arg Gly Arg Ser Met Gln Ala Leu Ala Glu Glu Asp Leu
        595                     600                     605
Gln His Leu Pro Pro Gln Pro Leu Pro Pro Gln Leu Ser Gln Xaa Asp
    610                     615                     620
Gly Arg Ser Glu Thr Ser Gln Pro Pro Thr Pro Gly Ser Pro Ser His
625                     630                     635                     640
Ser Pro Leu His Ser Gly Pro Arg Pro Arg Arg Arg Glu Ser Asp Pro
                645                     650                     655
Thr Gly Pro Gln Arg Gln Leu Glu Glu Asp Arg Leu Ser Gly His Ser
            660                     665                     670
Leu Pro Arg Tyr Ser Pro Leu Arg Arg Leu Ala Ser Ser Val Phe Ser
        675                     680                     685
Ser Ser Thr Leu Glu Thr Glu His Tyr Pro His Pro Gly Gly Gly Gly
    690                     695                     700
Ser Ser Gly Ser Ser Gly Ser Leu Ile Gln Arg Ser Arg Ser Ala Glu
705                     710                     715                     720
Ser Ser Pro Val Arg Ala Pro His Arg Arg His Ala Pro Leu Ala Ala
                725                     730                     735
Gly Asn His Arg Leu Met Pro Ser Val Leu Arg Ile Ser Arg Ser Gln
            740                     745                     750
Leu Gln Gln Val Trp Ala Arg Phe Thr His Lys Thr
        755                     760

<210> 7
<211> 2256
<212> DNA
<213> homo sapiens

<400> 7
   atgagtgggg gaggggagca ggccgacatc ctgccggcca actacgtggt caaggatcgc 60
   tggaaggtgc tgaaaaagat cgggggcggg ggctttggtg agatctacga ggccatggac 120
   ctgctgacca gggagaatgt ggccctcaag gtggagtcag cccagcagcc caagcaggtc 180
   ctcaagatgg aggtggccgt gctcaagaag ttgcaaggga aggaccatgt gtgcaggttc 240
   attggctgtg gcaggaacga gaagtttaac tatgtagtga tgcagctcca gggccggaac 300
   ctggccgacc tgcgccgtag ccagccgcga ggcaccttca cgctgagcac cacattgcgg 360
   ctgggcaagc agatcttgga gtccatcgag gccatccact ctgtgggctt cctgcaccgt 420
   gacatcaagc cttcaaactt tgccatgggc aggctgccct ccacctacag gaagtgctat 480
   atgctggact tcgggctggc ccggcagtac accaacacca cgggggatgt gcggccccct 540
   cggaatgtgg ccgggtttcg aggaacggtt cgctatgcct cagtcaatgc ccacaagaac 600
   cgggagatgg gccgccacga cgacctgtgg tccctcttct acatgctggt ggagtttgca 660
   gtgggccagc tgccctggag gaagatcaag gacaaggaac aggtagggat gatcaaggag 720
   aagtatgagc accggatgct gctgaagcac atgccgtcag agttccacct cttcctggac 780
   cacattgcca gcctcgacta cttcaccaag cccgactacc agttgatcat gtcagtgttt 840
   gagaacagca tgaaggagag gggcattgcc gagaatgagg cctttgactg ggagaaggca 900
   ggcaccgatg ccctcctgtc cacgagcacc tctaccccgc cccagcagaa cacccggcag 960
   acggcagcca tgtttggggt ggtcaatgtg acgccagtgc ctggggacct gctccgggag 1020
   aacaccgagg atgtgctaca gggagagcac ctgagtgacc aggagaatgc acccccaatt 1080
   ctgcccggga ggccctctga ggggctgggc cccagtcccc accttgtccc ccaccccggg 1140
   ggtcctgagg ctgaagtctg ggaggagaca gatgtcaacc ggaacaaact ccggatcaac 1200
   atcggcaaaa gcccctgtgt ggaggaggaa cagagccgag gcatgggggt ccccagctcc 1260
   ccagtgcgtg cccccccaga ctcccccaca accccagtcc gttctctgcg ctaccggagg 1320
   gtgaacagcc ctgagtcaga aaggctgtcc acggcggacg ggcgagtgga gctacctgag 1380
   aggaggtcac ggatggatct gcctggctcg ccctcgcgcc aggcctgctc ctctcagcca 1440
   gcccagatgc tgtcagtgga cacaggccac gctgaccgac aggccagtgg ccgcatggay 1500
   gtgtcagcct ctgtggagca ggaggccctg agcaacgcct tccgctcggt gccgctggct 1560
   gaggaggagg atttcgacag caaagagtgg gtcatcatcg acaaggagac ggagctcaag 1620
   gacttccctc caggggctga gcccagcaca tcgggcacca cggatgagga gcccgaggag 1680
   ctgcggccac tgcccgagga gggcgaagag cggcggcggc tgggggcaga gcccaccgtc 1740
   cggccccggg gacgcagcat gcaggcgctg gcggaggagg acctgcagca tttgccgccc 1800
   cagcccctgc caccccagct gagccaggsc gatggccgtt ccgagacgtc acagcccccc 1860
   acgcctggca gcccttccca ctcacccctg cactcgggac cccgccctcg acggagagag 1920
   tcggacccca caggcccaca gagacagttg gaggaggaca gactctcggg gcactccctc 1980
   ccgcggtaca gccccctgcg acgactggcg tcctccgtgt tctcctcctc cacgctggag 2040
   acggagcatt accctcaccc cggcggcggc ggctcctcgg gctcctccgg ttccctcatt 2100
   cagcgcagcc gctcggctga gagcagccct gtgcgggcgc cccaccggcg ccacgcgccc 2160
   ctcgctgctg gcaaccacag actcatgccc tcggtgctcc gcatctcgcg gtcccagctg 2220
   cagcaggtgt gggcccggtt cacccacaag acctag 2256

<210> 8
<211> 751
<212> PRT
<213> homo sapiens

<220>
<221> VARIANT
<222> (1)...(751)
<223> Xaa = Any Amino Acid

<400> 8
Met Ser Gly Gly Gly Glu Gln Ala Asp Ile Leu Pro Ala Asn Tyr Val
1               5                   10                  15
Val Lys Asp Arg Trp Lys Val Leu Lys Lys Ile Gly Gly Gly Gly Phe
            20                  25                  30
Gly Glu Ile Tyr Glu Ala Met Asp Leu Leu Thr Arg Glu Asn Val Ala
        35                  40                  45
Leu Lys Val Glu Ser Ala Gln Gln Pro Lys Gln Val Leu Lys Met Glu
    50                  55                  60
Val Ala Val Leu Lys Lys Leu Gln Gly Lys Asp His Val Cys Arg Phe
65                  70                  75                  80
Ile Gly Cys Gly Arg Asn Glu Lys Phe Asn Tyr Val Val Met Gln Leu
                85                  90                  95
Gln Gly Arg Asn Leu Ala Asp Leu Arg Arg Ser Gln Pro Arg Gly Thr
            100                 105                 110
Phe Thr Leu Ser Thr Thr Leu Arg Leu Gly Lys Gln Ile Leu Glu Ser
        115                 120                 125
Ile Glu Ala Ile His Ser Val Gly Phe Leu His Arg Asp Ile Lys Pro
    130                 135                 140
Ser Asn Phe Ala Met Gly Arg Leu Pro Ser Thr Tyr Arg Lys Cys Tyr
145                 150                 155                 160
Met Leu Asp Phe Gly Leu Ala Arg Gln Tyr Thr Asn Thr Thr Gly Asp
                165                 170                 175
Val Arg Pro Pro Arg Asn Val Ala Gly Phe Arg Gly Thr Val Arg Tyr
            180                 185                 190
Ala Ser Val Asn Ala His Lys Asn Arg Glu Met Gly Arg His Asp Asp
        195                 200                 205
Leu Trp Ser Leu Phe Tyr Met Leu Val Glu Phe Ala Val Gly Gln Leu
    210                 215                 220
Pro Trp Arg Lys Ile Lys Asp Lys Glu Gln Val Gly Met Ile Lys Glu
225                 230                 235                 240
Lys Tyr Glu His Arg Met Leu Leu Lys His Met Pro Ser Glu Phe His
                245                 250                 255
Leu Phe Leu Asp His Ile Ala Ser Leu Asp Tyr Phe Thr Lys Pro Asp
            260                 265                 270
Tyr Gln Leu Ile Met Ser Val Phe Glu Asn Ser Met Lys Glu Arg Gly

        275                 280                 285
Ile Ala Glu Asn Glu Ala Phe Asp Trp Glu Lys Ala Gly Thr Asp Ala
    290                 295                 300
Leu Leu Ser Thr Ser Thr Ser Thr Pro Pro Gln Gln Asn Thr Arg Gln
305                 310                 315                 320
Thr Ala Ala Met Phe Gly Val Val Asn Val Thr Pro Val Pro Gly Asp
                325                 330                 335
Leu Leu Arg Glu Asn Thr Glu Asp Val Leu Gln Gly Glu His Leu Ser
            340                 345                 350
Asp Gln Glu Asn Ala Pro Pro Ile Leu Pro Gly Arg Pro Ser Glu Gly
        355                 360                 365
Leu Gly Pro Ser Pro His Leu Val Pro His Pro Gly Gly Pro Glu Ala
    370                 375                 380
Glu Val Trp Glu Glu Thr Asp Val Asn Arg Asn Lys Leu Arg Ile Asn
385                 390                 395                 400
Ile Gly Lys Ser Pro Cys Val Glu Glu Glu Gln Ser Arg Gly Met Gly
                405                 410                 415
Val Pro Ser Ser Pro Val Arg Ala Pro Pro Asp Ser Pro Thr Thr Pro
            420                 425                 430
Val Arg Ser Leu Arg Tyr Arg Arg Val Asn Ser Pro Glu Ser Glu Arg
        435                 440                 445
Leu Ser Thr Ala Asp Gly Arg Val Glu Leu Pro Glu Arg Arg Ser Arg
    450                 455                 460
Met Asp Leu Pro Gly Ser Pro Ser Arg Gln Ala Cys Ser Ser Gln Pro
465                 470                 475                 480
Ala Gln Met Leu Ser Val Asp Thr Gly His Ala Asp Arg Gln Ala Ser
                485                 490                 495
Gly Arg Met Asp Val Ser Ala Ser Val Glu Gln Glu Ala Leu Ser Asn
            500                 505                 510
Ala Phe Arg Ser Val Pro Leu Ala Glu Glu Glu Asp Phe Asp Ser Lys
        515                 520                 525
Glu Trp Val Ile Ile Asp Lys Glu Thr Glu Leu Lys Asp Phe Pro Pro
    530                 535                 540
Gly Ala Glu Pro Ser Thr Ser Gly Thr Thr Asp Glu Glu Pro Glu Glu
545                 550                 555                 560
Leu Arg Pro Leu Pro Glu Glu Gly Glu Glu Arg Arg Arg Leu Gly Ala
                565                 570                 575
Glu Pro Thr Val Arg Pro Arg Gly Arg Ser Met Gln Ala Leu Ala Glu
            580                 585                 590
Glu Asp Leu Gln His Leu Pro Pro Gln Pro Leu Pro Pro Gln Leu Ser
        595                 600                 605
Gln Xaa Asp Gly Arg Ser Glu Thr Ser Gln Pro Pro Thr Pro Gly Ser
    610                 615                 620
Pro Ser His Ser Pro Leu His Ser Gly Pro Arg Pro Arg Arg Arg Glu
625                 630                 635                 640
Ser Asp Pro Thr Gly Pro Gln Arg Gln Leu Glu Glu Asp Arg Leu Ser
                645                 650                 655
Gly His Ser Leu Pro Arg Tyr Ser Pro Leu Arg Arg Leu Ala Ser Ser
            660                 665                 670
Val Phe Ser Ser Ser Thr Leu Glu Thr Glu His Tyr Pro His Pro Gly
        675                 680                 685
Gly Gly Gly Ser Ser Gly Ser Ser Gly Ser Leu Ile Gln Arg Ser Arg
    690                 695                 700
Ser Ala Glu Ser Ser Pro Val Arg Ala Pro His Arg Arg His Ala Pro
705                 710                 715                 720
Leu Ala Ala Gly Asn His Arg Leu Met Pro Ser Val Leu Arg Ile Ser
                725                 730                 735
Arg Ser Gln Leu Gln Gln Val Trp Ala Arg Phe Thr His Lys Thr
            740                 745                 750

<210> 9
<211> 1965
<212> DNA
<213> homo sapiens

<400> 9
atgtcagggc tggtgctgat gctggcggcg cggtgcattg tgggcagctc cccgctctgc 60

cgctgccgcc gccgtcgccc aaggaggatc ggggccgggc cgggccggga tgatccgggt 120
cggaaggccg ccgccgccgg agggagcggg tcacccaacg ccgcactgag ccgcccccgc 180
cccgccccgg ccccggggga tgcgccgccc cgagctgctg cctccgccgc cgccgcagcc 240
gcagccgcag cgggcacaga gcaggtagat ggcccccctca gggcaggccc ggcggacacc 300
cctccctctg gctggcggat gcagtgccta gcggccgccc ttaaggacga aaccaacatg 360
agtgggggag gggagcaggc cgacatcctg ccggccaact acgtggtcaa ggatcgctgg 420
aaggtgctga aaaagatcgg gggcgggggc tttggtgaga tctacgaggc catggacctg 480
ctgaccaggg agaatgtggc cctcaaggtg gagtcagccc agcagcccaa gcaggtcctc 540
aagatggagg tggccgtgct caagaagttg caagggaagg accatgtgtg caggttcatt 600
ggctgtggca ggaacgagaa gtttaactat gtagtgatgc agctccaggg ccggaacctg 660
gccgacctgc gccgtagcca gccgcgaggc accttcacgc tgagcaccac attgcggctg 720
ggcaagcaga tcttggagtc catcgaggcc atccactctg tgggcttcct gcaccgtgac 780
atcaagcctt caaactttgc catgggcagg ctgccctcca cctacaggaa gtgctatatg 840
ctggacttcg ggctggcccg gcagtacacc aacaccacgg gggatgtgcg gccccctcgg 900
aatgtggccg ggtttcgagg aacggttcgc tatgcctcag tcaatgccca caagaaccgg 960
gagatgggcc gccacgacga cctgtggtcc ctcttctaca tgctggtgga gtttgcagtg 1020
ggccagctgc cctggaggaa gatcaaggac aaggaacagg tagggatgat caaggagaag 1080
tatgagcacc ggatgctgct gaagcacatg ccgtcagagt tccacctctt cctggaccac 1140
attgccagcc tcgactactt caccaagccc gactaccagt tgatcatgtc agtgtttgag 1200
aacagcatga aggagagggg cattgccgag aatgaggcct ttgactggga gaaggcaggc 1260
accgatgccc tcctgtccac gagcacctct accccgcccc agcagaacac ccggcagacg 1320
gcagccatgt ttggggtggt caatgtgacg ccagtgcctg gggacctgct ccgggagaac 1380
accgaggatg tgctacaggg agagcacctg agtgaccagg agaatgcacc cccaattctg 1440
cccgggaggc cctctgaggg gctgggcccc agtccccacc ttgtccccca ccccgggggt 1500
cctgaggctg aagtctggga ggagacagat gtcaaccgga acaaactccg gatcaacatc 1560
ggcaaaagcc cctgtgtgga ggaggaacag agccgaggca tgggggtccc cagctcccca 1620
gtgcgtgccc ccccagactc ccccacaacc ccagtccgtt ctctgcgcta ccggagggtg 1680
aacagccctg agtcagaaag gctgtccacg gcggacgggc gagtggagct acctgagagg 1740
aggtgggtct ggggccaggg gcatggttgg ggcccaaggc cctctccgcc ttcacgtggc 1800
tggtctggag gaaaagttag atgtgtggcg gaggtgggca gaccctggga agtgctgaga 1860
gggttatact tgggcctggg gtcagactca gttggggcca sagacagggc ctgggaraac 1920
cagtggggga tccagagagg tcccggctca tgccaggaaa cgtaa 1965

<210> 10
<211> 654
<212> PRT
<213> homo sapiens

<220>
<221> VARIANT
<222> (1)...(654)
<223> Xaa = Any Amino Acid

<400> 10
Met Ser Gly Leu Val Leu Met Leu Ala Ala Arg Cys Ile Val Gly Ser
1               5                   10                  15
Ser Pro Leu Cys Arg Cys Arg Arg Arg Arg Pro Arg Arg Ile Gly Ala
            20                  25                  30
Gly Pro Gly Arg Asp Asp Pro Gly Arg Lys Ala Ala Ala Ala Gly Gly
        35                  40                  45
Ser Gly Ser Pro Asn Ala Ala Leu Ser Arg Pro Arg Pro Ala Pro Ala
    50                  55                  60
Pro Gly Asp Ala Pro Pro Arg Ala Ala Ala Ser Ala Ala Ala Ala Ala
65                  70                  75                  80
Ala Ala Ala Ala Gly Thr Glu Gln Val Asp Gly Pro Leu Arg Ala Gly
                85                  90                  95
Pro Ala Asp Thr Pro Pro Ser Gly Trp Arg Met Gln Cys Leu Ala Ala
            100                 105                 110
Ala Leu Lys Asp Glu Thr Asn Met Ser Gly Gly Gly Glu Gln Ala Asp
        115                 120                 125
Ile Leu Pro Ala Asn Tyr Val Val Lys Asp Arg Trp Lys Val Leu Lys
    130                 135                 140
Lys Ile Gly Gly Gly Gly Phe Gly Glu Ile Tyr Glu Ala Met Asp Leu
145                 150                 155                 160
Leu Thr Arg Glu Asn Val Ala Leu Lys Val Glu Ser Ala Gln Gln Pro
                165                 170                 175
Lys Gln Val Leu Lys Met Glu Val Ala Val Leu Lys Lys Leu Gln Gly
            180                 185                 190

Lys Asp His Val Cys Arg Phe Ile Gly Cys Gly Arg Asn Glu Lys Phe
        195                 200                 205
Asn Tyr Val Val Met Gln Leu Gln Gly Arg Asn Leu Ala Asp Leu Arg
    210                 215                 220
Arg Ser Gln Pro Arg Gly Thr Phe Thr Leu Ser Thr Thr Leu Arg Leu
225                 230                 235                 240
Gly Lys Gln Ile Leu Glu Ser Ile Glu Ala Ile His Ser Val Gly Phe
                245                 250                 255
Leu His Arg Asp Ile Lys Pro Ser Asn Phe Ala Met Gly Arg Leu Pro
            260                 265                 270
Ser Thr Tyr Arg Lys Cys Tyr Met Leu Asp Phe Gly Leu Ala Arg Gln
        275                 280                 285
Tyr Thr Asn Thr Thr Gly Asp Val Arg Pro Pro Arg Asn Val Ala Gly
    290                 295                 300
Phe Arg Gly Thr Val Arg Tyr Ala Ser Val Asn Ala His Lys Asn Arg
305                 310                 315                 320
Glu Met Gly Arg His Asp Asp Leu Trp Ser Leu Phe Tyr Met Leu Val
                325                 330                 335
Glu Phe Ala Val Gly Gln Leu Pro Trp Arg Lys Ile Lys Asp Lys Glu
            340                 345                 350
Gln Val Gly Met Ile Lys Glu Lys Tyr Glu His Arg Met Leu Leu Lys
        355                 360                 365
His Met Pro Ser Glu Phe His Leu Phe Leu Asp His Ile Ala Ser Leu
    370                 375                 380
Asp Tyr Phe Thr Lys Pro Asp Tyr Gln Leu Ile Met Ser Val Phe Glu
385                 390                 395                 400
Asn Ser Met Lys Glu Arg Gly Ile Ala Glu Asn Glu Ala Phe Asp Trp
                405                 410                 415
Glu Lys Ala Gly Thr Asp Ala Leu Leu Ser Thr Ser Thr Ser Thr Pro
            420                 425                 430
Pro Gln Gln Asn Thr Arg Gln Thr Ala Ala Met Phe Gly Val Val Asn
        435                 440                 445
Val Thr Pro Val Pro Gly Asp Leu Leu Arg Glu Asn Thr Glu Asp Val
    450                 455                 460
Leu Gln Gly Glu His Leu Ser Asp Gln Glu Asn Ala Pro Pro Ile Leu
465                 470                 475                 480
Pro Gly Arg Pro Ser Glu Gly Leu Gly Pro Ser Pro His Leu Val Pro
                485                 490                 495
His Pro Gly Gly Pro Glu Ala Glu Val Trp Glu Glu Thr Asp Val Asn
            500                 505                 510
Arg Asn Lys Leu Arg Ile Asn Ile Gly Lys Ser Pro Cys Val Glu Glu
        515                 520                 525
Glu Gln Ser Arg Gly Met Gly Val Pro Ser Ser Pro Val Arg Ala Pro
    530                 535                 540
Pro Asp Ser Pro Thr Thr Pro Val Arg Ser Leu Arg Tyr Arg Arg Val
545                 550                 555                 560
Asn Ser Pro Glu Ser Glu Arg Leu Ser Thr Ala Asp Gly Arg Val Glu
                565                 570                 575
Leu Pro Glu Arg Arg Trp Val Trp Gly Gln Gly His Gly Trp Gly Pro
            580                 585                 590
Arg Pro Ser Pro Pro Ser Arg Gly Trp Ser Gly Gly Lys Val Arg Cys
        595                 600                 605
Val Ala Glu Val Gly Arg Pro Trp Glu Val Leu Arg Gly Leu Tyr Leu
    610                 615                 620
Gly Leu Gly Ser Asp Ser Val Gly Ala Xaa Asp Arg Ala Trp Glu Asn
625                 630                 635                 640
Gln Trp Gly Ile Gln Arg Gly Pro Gly Ser Cys Gln Glu Thr
                645                 650

<210> 11
<211> 1947
<212> DNA
<213> homo sapiens

<400> 11
atgctggcgg cgcggtgcat tgtgggcagc tccccgctct gccgctgccg ccgccgtcgc 60
ccaaggagga tcggggccgg gccgggccgg gatgatccgg gtcggaaggc cgccgccgcc 120

ggagggagcg ggtcacccaa cgccgcactg agccgccccc gccccgcccc ggccccgggg 180
gatgcgccgc cccgagctgc tgcctccgcc gccgccgcag ccgcagccgc agcgggcaca 240
gagcaggtag atggccccct cagggcaggc ccggcggaca cccctccctc tggctggcgg 300
atgcagtgcc tagcggccgc ccttaaggac gaaaccaaca tgagtggggg aggggagcag 360
gccgacatcc tgccggccaa ctacgtggtc aaggatcgct ggaaggtgct gaaaaagatc 420
gggggcgggg gctttggtga gatctacgag gccatggacc tgctgaccag ggagaatgtg 480
gccctcaagg tggagtcagc ccagcagccc aagcaggtcc tcaagatgga ggtggccgtg 540
ctcaagaagt tgcaagggaa ggaccatgtg tgcaggttca ttggctgtgg caggaacgag 600
aagtttaact atgtagtgat gcagctccag ggccggaacc tggccgacct gcgccgtagc 660
cagccgcgag gcaccttcac gctgagcacc acattgcggc tgggcaagca gatcttggag 720
tccatcgagg ccatccactc tgtgggcttc ctgcaccgtg acatcaagcc ttcaaacttt 780
gccatgggca ggctgccctc cacctacagg aagtgctata tgctggactt cgggctggcc 840
cggcagtaca ccaacaccac gggggatgtg cggccccctc ggaatgtggc cgggtttcga 900
ggaacggttc gctatgcctc agtcaatgcc cacaagaacc gggagatggg ccgccacgac 960
gacctgtggt ccctcttcta catgctggtg gagtttgcag tgggccagct gccctggagg 1020
aagatcaagg acaaggaaca ggtagggatg atcaaggaga agtatgagca ccggatgctg 1080
ctgaagcaca tgccgtcaga gttccacctc ttcctggacc acattgccag cctcgactac 1140
ttcaccaagc ccgactacca gttgatcatg tcagtgtttg agaacagcat gaaggagagg 1200
ggcattgccg agaatgaggc ctttgactgg gagaaggcag gcaccgatgc cctcctgtcc 1260
acgagcacct ctaccccgcc ccagcagaac acccggcaga cggcagccat gtttggggtg 1320
gtcaatgtga cgccagtgcc tggggacctg ctccgggaga acaccgagga tgtgctacag 1380
ggagagcacc tgagtgacca ggagaatgca cccccaattc tgcccgggag gccctctgag 1440
gggctgggcc ccagtcccca ccttgtcccc caccccgggg gtcctgaggc tgaagtctgg 1500
gaggagacag atgtcaaccg gaacaaactc cggatcaaca tcggcaaaag cccctgtgtg 1560
gaggaggaac agagccgagg catgggggtc cccagctccc cagtgcgtgc ccccccagac 1620
tcccccacaa ccccagtccg ttctctgcgc taccggaggg tgaacagccc tgagtcagaa 1680
aggctgtcca cggcggacgg gcgagtggag ctacctgaga ggaggtgggt ctggggccag 1740
gggcatggtt ggggcccaag gccctctccg ccttcacgtg gctggtctgg aggaaaagtt 1800
agatgtgtgg cggaggtggg cagaccctgg gaagtgctga gagggttata cttgggcctg 1860
gggtcagact cagttggggc casagacagg gcctgggara accagtgggg gatccagaga 1920
ggtcccggct catgccagga aacgtaa 1947

<210> 12
<211> 648
<212> PRT
<213> homo sapiens

<220>
<221> VARIANT
<222> (1)...(648)
<223> Xaa = Any Amino Acid

<400> 12
Met Leu Ala Ala Arg Cys Ile Val Gly Ser Ser Pro Leu Cys Arg Cys
1               5                   10                  15
Arg Arg Arg Arg Pro Arg Arg Ile Gly Ala Gly Pro Gly Arg Asp Asp
            20                  25                  30
Pro Gly Arg Lys Ala Ala Ala Ala Gly Gly Ser Gly Ser Pro Asn Ala
        35                  40                  45
Ala Leu Ser Arg Pro Arg Pro Ala Pro Ala Pro Gly Asp Ala Pro Pro
    50                  55                  60
Arg Ala Ala Ala Ser Ala Ala Ala Ala Ala Ala Ala Ala Ala Gly Thr
65                  70                  75                  80
Glu Gln Val Asp Gly Pro Leu Arg Ala Gly Pro Ala Asp Thr Pro Pro
                85                  90                  95
Ser Gly Trp Arg Met Gln Cys Leu Ala Ala Ala Leu Lys Asp Glu Thr
            100                 105                 110
Asn Met Ser Gly Gly Gly Glu Gln Ala Asp Ile Leu Pro Ala Asn Tyr
        115                 120                 125
Val Val Lys Asp Arg Trp Lys Val Leu Lys Lys Ile Gly Gly Gly Gly
    130                 135                 140
Phe Gly Glu Ile Tyr Glu Ala Met Asp Leu Leu Thr Arg Glu Asn Val
145                 150                 155                 160
Ala Leu Lys Val Glu Ser Ala Gln Gln Pro Lys Gln Val Leu Lys Met
                165                 170                 175
Glu Val Ala Val Leu Lys Lys Leu Gln Gly Lys Asp His Val Cys Arg
            180                 185                 190
Phe Ile Gly Cys Gly Arg Asn Glu Lys Phe Asn Tyr Val Val Met Gln

195                 200                 205
Leu Gln Gly Arg Asn Leu Ala Asp Leu Arg Arg Ser Gln Pro Arg Gly
    210                 215                 220
Thr Phe Thr Leu Ser Thr Thr Leu Arg Leu Gly Lys Gln Ile Leu Glu
225                 230                 235                 240
Ser Ile Glu Ala Ile His Ser Val Gly Phe Leu His Arg Asp Ile Lys
                245                 250                 255
Pro Ser Asn Phe Ala Met Gly Arg Leu Pro Ser Thr Tyr Arg Lys Cys
            260                 265                 270
Tyr Met Leu Asp Phe Gly Leu Ala Arg Gln Tyr Thr Asn Thr Thr Gly
        275                 280                 285
Asp Val Arg Pro Pro Arg Asn Val Ala Gly Phe Arg Gly Thr Val Arg
    290                 295                 300
Tyr Ala Ser Val Asn Ala His Lys Asn Arg Glu Met Gly Arg His Asp
305                 310                 315                 320
Asp Leu Trp Ser Leu Phe Tyr Met Leu Val Glu Phe Ala Val Gly Gln
                325                 330                 335
Leu Pro Trp Arg Lys Ile Lys Asp Lys Glu Gln Val Gly Met Ile Lys
            340                 345                 350
Glu Lys Tyr Glu His Arg Met Leu Leu Lys His Met Pro Ser Glu Phe
        355                 360                 365
His Leu Phe Leu Asp His Ile Ala Ser Leu Asp Tyr Phe Thr Lys Pro
    370                 375                 380
Asp Tyr Gln Leu Ile Met Ser Val Phe Glu Asn Ser Met Lys Glu Arg
385                 390                 395                 400
Gly Ile Ala Glu Asn Glu Ala Phe Asp Trp Glu Lys Ala Gly Thr Asp
                405                 410                 415
Ala Leu Leu Ser Thr Ser Thr Ser Thr Pro Pro Gln Gln Asn Thr Arg
            420                 425                 430
Gln Thr Ala Ala Met Phe Gly Val Val Asn Val Thr Pro Val Pro Gly
        435                 440                 445
Asp Leu Leu Arg Glu Asn Thr Glu Asp Val Leu Gln Gly Glu His Leu
    450                 455                 460
Ser Asp Gln Glu Asn Ala Pro Pro Ile Leu Pro Gly Arg Pro Ser Glu
465                 470                 475                 480
Gly Leu Gly Pro Ser Pro His Leu Val Pro His Pro Gly Gly Pro Glu
                485                 490                 495
Ala Glu Val Trp Glu Glu Thr Asp Val Asn Arg Asn Lys Leu Arg Ile
            500                 505                 510
Asn Ile Gly Lys Ser Pro Cys Val Glu Glu Glu Gln Ser Arg Gly Met
        515                 520                 525
Gly Val Pro Ser Ser Pro Val Arg Ala Pro Pro Asp Ser Pro Thr Thr
    530                 535                 540
Pro Val Arg Ser Leu Arg Tyr Arg Arg Val Asn Ser Pro Glu Ser Glu
545                 550                 555                 560
Arg Leu Ser Thr Ala Asp Gly Arg Val Glu Leu Pro Glu Arg Arg Trp
                565                 570                 575
Val Trp Gly Gln Gly His Gly Trp Gly Pro Arg Pro Ser Pro Pro Ser
            580                 585                 590
Arg Gly Trp Ser Gly Gly Lys Val Arg Cys Val Ala Glu Val Gly Arg
        595                 600                 605
Pro Trp Glu Val Leu Arg Gly Leu Tyr Leu Gly Leu Gly Ser Asp Ser
    610                 615                 620
Val Gly Ala Xaa Asp Arg Ala Trp Glu Asn Gln Trp Gly Ile Gln Arg
625                 630                 635                 640
Gly Pro Gly Ser Cys Gln Glu Thr
                645

<210> 13
<211> 1647
<212> DNA
<213> homo sapiens

<400> 13
atgcagtgcc tagcggccgc ccttaaggac gaaaccaaca tgagtggggg aggggagcag 60
gccgacatcc tgccggccaa ctacgtggtc aaggatcgct ggaaggtgct gaaaaagatc 120
gggggcgggg gctttggtga gatctacgag gccatggacc tgctgaccag ggagaatgtg 180

gccctcaagg tggagtcagc ccagcagccc aagcaggtcc tcaagatgga ggtggccgtg 240
ctcaagaagt tgcaagggaa ggaccatgtg tgcaggttca ttggctgtgg caggaacgag 300
aagtttaact atgtagtgat gcagctccag ggccggaacc tggccgacct gcgccgtagc 360
cagccgcgag gcaccttcac gctgagcacc acattgcggc tgggcaagca gatcttggag 420
tccatcgagg ccatccactc tgtgggcttc ctgcaccgtg acatcaagcc ttcaaacttt 480
gccatgggca ggctgccctc cacctacagg aagtgctata tgctggactt cgggctggcc 540
cggcagtaca ccaacaccac gggggatgtg cggccccctc ggaatgtggc cgggtttcga 600
ggaacggttc gctatgcctc agtcaatgcc cacaagaacc gggagatggg ccgccacgac 660
gacctgtggt ccctcttcta catgctggtg gagtttgcag tgggccagct gccctggagg 720
aagatcaagg acaaggaaca ggtagggatg atcaaggaga agtatgagca ccggatgctg 780
ctgaagcaca tgccgtcaga gttccacctc ttcctggacc acattgccag cctcgactac 840
ttcaccaagc ccgactacca gttgatcatg tcagtgtttg agaacagcat gaaggagagg 900
ggcattgccg agaatgaggc ctttgactgg gagaaggcag gcaccgatgc cctcctgtcc 960
acgagcacct ctaccccgcc ccagcagaac acccggcaga cggcagccat gtttggggtg 1020
gtcaatgtga cgccagtgcc tggggacctg ctccgggaga acaccgagga tgtgctacag 1080
ggagagcacc tgagtgacca ggagaatgca cccccaattc tgcccgggag gccctctgag 1140
gggctgggcc ccagtcccca ccttgtcccc caccccgggg gtcctgaggc tgaagtctgg 1200
gaggagacag atgtcaaccg gaacaaactc cggatcaaca tcggcaaaag cccctgtgtg 1260
gaggaggaac agagccgagg catgggggtc cccagctccc cagtgcgtgc ccccccagac 1320
tcccccacaa ccccagtccg ttctctgcgc taccggaggg tgaacagccc tgagtcagaa 1380
aggctgtcca cggcggacgg gcgagtggag ctacctgaga ggaggtgggt ctggggccag 1440
gggcatggtt ggggcccaag gccctctccg ccttcacgtg gctggtctgg aggaaaagtt 1500
agatgtgtgg cggaggtggg cagaccctgg gaagtgctga gagggttata cttgggcctg 1560
gggtcagact cagttggggc casagacagg gcctgggara accagtgggg gatccagaga 1620
ggtcccggct catgccagga aacgtaa 1647

<210> 14
<211> 548
<212> PRT
<213> homo sapiens

<220>
<221> VARIANT
<222> (1)...(548)
<223> Xaa = Any Amino Acid

<400> 14
Met Gln Cys Leu Ala Ala Ala Leu Lys Asp Glu Thr Asn Met Ser Gly
1               5                   10                  15
Gly Gly Glu Gln Ala Asp Ile Leu Pro Ala Asn Tyr Val Val Lys Asp
            20                  25                  30
Arg Trp Lys Val Leu Lys Lys Ile Gly Gly Gly Gly Phe Gly Glu Ile
        35                  40                  45
Tyr Glu Ala Met Asp Leu Leu Thr Arg Glu Asn Val Ala Leu Lys Val
    50                  55                  60
Glu Ser Ala Gln Gln Pro Lys Gln Val Leu Lys Met Glu Val Ala Val
65                  70                  75                  80
Leu Lys Lys Leu Gln Gly Lys Asp His Val Cys Arg Phe Ile Gly Cys
                85                  90                  95
Gly Arg Asn Glu Lys Phe Asn Tyr Val Val Met Gln Leu Gln Gly Arg
            100                 105                 110
Asn Leu Ala Asp Leu Arg Arg Ser Gln Pro Arg Gly Thr Phe Thr Leu
        115                 120                 125
Ser Thr Thr Leu Arg Leu Gly Lys Gln Ile Leu Glu Ser Ile Glu Ala
    130                 135                 140
Ile His Ser Val Gly Phe Leu His Arg Asp Ile Lys Pro Ser Asn Phe
145                 150                 155                 160
Ala Met Gly Arg Leu Pro Ser Thr Tyr Arg Lys Cys Tyr Met Leu Asp
                165                 170                 175
Phe Gly Leu Ala Arg Gln Tyr Thr Asn Thr Thr Gly Asp Val Arg Pro
            180                 185                 190
Pro Arg Asn Val Ala Gly Phe Arg Gly Thr Val Arg Tyr Ala Ser Val
        195                 200                 205
Asn Ala His Lys Asn Arg Glu Met Gly Arg His Asp Asp Leu Trp Ser
    210                 215                 220
Leu Phe Tyr Met Leu Val Glu Phe Ala Val Gly Gln Leu Pro Trp Arg
225                 230                 235                 240
Lys Ile Lys Asp Lys Glu Gln Val Gly Met Ile Lys Glu Lys Tyr Glu

245                 250                 255
His Arg Met Leu Leu Lys His Met Pro Ser Glu Phe His Leu Phe Leu
            260                 265                 270
Asp His Ile Ala Ser Leu Asp Tyr Phe Thr Lys Pro Asp Tyr Gln Leu
        275                 280                 285
Ile Met Ser Val Phe Glu Asn Ser Met Lys Glu Arg Gly Ile Ala Glu
    290                 295                 300
Asn Glu Ala Phe Asp Trp Glu Lys Ala Gly Thr Asp Ala Leu Leu Ser
305                 310                 315                 320
Thr Ser Thr Ser Thr Pro Pro Gln Gln Asn Thr Arg Gln Thr Ala Ala
                325                 330                 335
Met Phe Gly Val Val Asn Val Thr Pro Val Pro Gly Asp Leu Leu Arg
            340                 345                 350
Glu Asn Thr Glu Asp Val Leu Gln Gly Glu His Leu Ser Asp Gln Glu
        355                 360                 365
Asn Ala Pro Pro Ile Leu Pro Gly Arg Pro Ser Glu Gly Leu Gly Pro
    370                 375                 380
Ser Pro His Leu Val Pro His Pro Gly Gly Pro Glu Ala Glu Val Trp
385                 390                 395                 400
Glu Glu Thr Asp Val Asn Arg Asn Lys Leu Arg Ile Asn Ile Gly Lys
                405                 410                 415
Ser Pro Cys Val Glu Glu Glu Gln Ser Arg Gly Met Gly Val Pro Ser
            420                 425                 430
Ser Pro Val Arg Ala Pro Pro Asp Ser Pro Thr Thr Pro Val Arg Ser
        435                 440                 445
Leu Arg Tyr Arg Arg Val Asn Ser Pro Glu Ser Glu Arg Leu Ser Thr
    450                 455                 460
Ala Asp Gly Arg Val Glu Leu Pro Glu Arg Arg Trp Val Trp Gly Gln
465                 470                 475                 480
Gly His Gly Trp Gly Pro Arg Pro Ser Pro Pro Ser Arg Gly Trp Ser
                485                 490                 495
Gly Gly Lys Val Arg Cys Val Ala Glu Val Gly Arg Pro Trp Glu Val
            500                 505                 510
Leu Arg Gly Leu Tyr Leu Gly Leu Gly Ser Asp Ser Val Gly Ala Xaa
        515                 520                 525
Asp Arg Ala Trp Glu Asn Gln Trp Gly Ile Gln Arg Gly Pro Gly Ser
    530                 535                 540
Cys Gln Glu Thr
545

<210> 15
<211> 1608
<212> DNA
<213> homo sapiens

<400> 15
atgagtgggg gaggggagca ggccgacatc ctgccggcca actacgtggt caaggatcgc 60
tggaaggtgc tgaaaaagat cgggggcggg ggctttggtg agatctacga ggccatggac 120
ctgctgacca gggagaatgt ggccctcaag gtggagtcag cccagcagcc caagcaggtc 180
ctcaagatgg aggtggccgt gctcaagaag ttgcaaggga aggaccatgt gtgcaggttc 240
attggctgtg gcaggaacga gaagtttaac tatgtagtga tgcagctcca gggccggaac 300
ctggccgacc tgcgccgtag ccagccgcga ggcaccttca cgctgagcac cacattgcgg 360
ctgggcaagc agatcttgga gtccatcgag gccatccact ctgtgggctt cctgcaccgt 420
gacatcaagc cttcaaactt tgccatgggc aggctgccct ccacctacag gaagtgctat 480
atgctggact tcgggctggc ccggcagtac accaacacca cgggggatgt gcggcccccct 540
cggaatgtgg ccgggtttcg aggaacggtt cgctatgcct cagtcaatgc ccacaagaac 600
cgggagatgg gccgccacga cgacctgtgg tccctcttct acatgctggt ggagtttgca 660
gtgggccagc tgccctggag gaagatcaag gacaaggaac aggtagggat gatcaaggag 720
aagtatgagc accggatgct gctgaagcac atgccgtcag agttccacct cttcctggac 780
cacattgcca gcctcgacta cttcaccaag cccgactacc agttgatcat gtcagtgttt 840
gagaacagca tgaaggagag gggcattgcc gagaatgagg cctttgactg ggagaaggca 900
ggcaccgatg ccctcctgtc cacgagcacc tctaccccgc cccagcagaa cacccggcag 960
acggcagcca tgtttggggt ggtcaatgtg acgccagtgc ctggggacct gctccgggag 1020
aacaccgagg atgtgctaca gggagagcac ctgagtgacc aggagaatgc acccccaatt 1080
ctgcccggga ggccctctga ggggctgggc cccagtcccc accttgtccc ccaccccggg 1140
ggtcctgagg ctgaagtctg ggaggagaca gatgtcaacc ggaacaaact ccggatcaac 1200
atcggcaaaa gcccctgtgt ggaggaggaa cagagccgag gcatgggggt ccccagctcc 1260

ccagtgcgtg cccccccaga ctcccccaca accccagtcc gttctctgcg ctaccggagg 1320
gtgaacagcc ctgagtcaga aaggctgtcc acggcggacg ggcgagtgga gctacctgag 1380
aggaggtggg tctggggcca ggggcatggt tggggcccaa ggccctctcc gccttcacgt 1440
ggctggtctg gaggaaaagt tagatgtgtg gcggaggtgg gcagaccctg ggaagtgctg 1500
agagggttat acttgggcct ggggtcagac tcagttgggg ccasagacag ggcctgggar 1560
aaccagtggg ggatccagag aggtcccggc tcatgccagg aaacgtaa 1608

<210> 16
<211> 535
<212> PRT
<213> homo sapiens

<220>
<221> VARIANT
<222> (1)...(535)
<223> Xaa = Any Amino Acid

<400> 16
Met Ser Gly Gly Gly Glu Gln Ala Asp Ile Leu Pro Ala Asn Tyr Val
1               5                   10                  15
Val Lys Asp Arg Trp Lys Val Leu Lys Lys Ile Gly Gly Gly Gly Phe
            20                  25                  30
Gly Glu Ile Tyr Glu Ala Met Asp Leu Leu Thr Arg Glu Asn Val Ala
        35                  40                  45
Leu Lys Val Glu Ser Ala Gln Gln Pro Lys Gln Val Leu Lys Met Glu
    50                  55                  60
Val Ala Val Leu Lys Lys Leu Gln Gly Lys Asp His Val Cys Arg Phe
65                  70                  75                  80
Ile Gly Cys Gly Arg Asn Glu Lys Phe Asn Tyr Val Val Met Gln Leu
                85                  90                  95
Gln Gly Arg Asn Leu Ala Asp Leu Arg Arg Ser Gln Pro Arg Gly Thr
            100                 105                 110
Phe Thr Leu Ser Thr Thr Leu Arg Leu Gly Lys Gln Ile Leu Glu Ser
        115                 120                 125
Ile Glu Ala Ile His Ser Val Gly Phe Leu His Arg Asp Ile Lys Pro
    130                 135                 140
Ser Asn Phe Ala Met Gly Arg Leu Pro Ser Thr Tyr Arg Lys Cys Tyr
145                 150                 155                 160
Met Leu Asp Phe Gly Leu Ala Arg Gln Tyr Thr Asn Thr Thr Gly Asp
                165                 170                 175
Val Arg Pro Pro Arg Asn Val Ala Gly Phe Arg Gly Thr Val Arg Tyr
            180                 185                 190
Ala Ser Val Asn Ala His Lys Asn Arg Glu Met Gly Arg His Asp Asp
        195                 200                 205
Leu Trp Ser Leu Phe Tyr Met Leu Val Glu Phe Ala Val Gly Gln Leu
    210                 215                 220
Pro Trp Arg Lys Ile Lys Asp Lys Glu Gln Val Gly Met Ile Lys Glu
225                 230                 235                 240
Lys Tyr Glu His Arg Met Leu Leu Lys His Met Pro Ser Glu Phe His
                245                 250                 255
Leu Phe Leu Asp His Ile Ala Ser Leu Asp Tyr Phe Thr Lys Pro Asp
            260                 265                 270
Tyr Gln Leu Ile Met Ser Val Phe Glu Asn Ser Met Lys Glu Arg Gly
        275                 280                 285
Ile Ala Glu Asn Glu Ala Phe Asp Trp Glu Lys Ala Gly Thr Asp Ala
    290                 295                 300
Leu Leu Ser Thr Ser Thr Ser Thr Pro Pro Gln Gln Asn Thr Arg Gln
305                 310                 315                 320
Thr Ala Ala Met Phe Gly Val Val Asn Val Thr Pro Val Pro Gly Asp
                325                 330                 335
Leu Leu Arg Glu Asn Thr Glu Asp Val Leu Gln Gly Glu His Leu Ser
            340                 345                 350
Asp Gln Glu Asn Ala Pro Pro Ile Leu Pro Gly Arg Pro Ser Glu Gly
        355                 360                 365
Leu Gly Pro Ser Pro His Leu Val Pro His Pro Gly Gly Pro Glu Ala
    370                 375                 380
Glu Val Trp Glu Glu Thr Asp Val Asn Arg Asn Lys Leu Arg Ile Asn
385                 390                 395                 400

Ile Gly Lys Ser Pro Cys Val Glu Glu Glu Gln Ser Arg Gly Met Gly
                405                 410                 415
Val Pro Ser Ser Pro Val Arg Ala Pro Pro Asp Ser Pro Thr Thr Pro
            420                 425                 430
Val Arg Ser Leu Arg Tyr Arg Arg Val Asn Ser Pro Glu Ser Glu Arg
        435                 440                 445
Leu Ser Thr Ala Asp Gly Arg Val Glu Leu Pro Glu Arg Arg Trp Val
    450                 455                 460
Trp Gly Gln Gly His Gly Trp Gly Pro Arg Pro Ser Pro Pro Ser Arg
465                 470                 475                 480
Gly Trp Ser Gly Gly Lys Val Arg Cys Val Ala Glu Val Gly Arg Pro
                485                 490                 495
Trp Glu Val Leu Arg Gly Leu Tyr Leu Gly Leu Gly Ser Asp Ser Val
            500                 505                 510
Gly Ala Xaa Asp Arg Ala Trp Glu Asn Gln Trp Gly Ile Gln Arg Gly
        515                 520                 525
Pro Gly Ser Cys Gln Glu Thr
    530                 535

<210> 17
<211> 2688
<212> DNA
<213> homo sapiens

<400> 17
   atgtcagggc tggtgctgat gctggcggcg cggtgcattg tgggcagctc cccgctctgc 60
   cgctgccgcc gccgtcgccc aaggaggatc ggggccgggc cgggccggga tgatccgggt 120
   cggaaggccg ccgccgccgg agggagcggg tcacccaacg ccgcactgag ccgcccccgc 180
   cccgccccgg ccccggggga tgcgccgccc cgagctgctg cctccgccgc cgccgcagcc 240
   gcagccgcag cgggcacaga gcaggtagat ggccccctca gggcaggccc ggcggacacc 300
   cctccctctg gctggcggat gcagtgccta gcggccgccc ttaaggacga aaccaacatg 360
   agtgggggag gggagcaggc cgacatcctg ccggccaact acgtggtcaa ggatcgctgg 420
   aaggtgctga aaaagatcgg gggcgggggc tttggtgaga tctacgaggc catggacctg 480
   ctgaccaggg agaatgtggc cctcaaggtg gagtcagccc agcagcccaa gcaggtcctc 540
   aagatggagg tggccgtgct caagaagttg caagggaagg accatgtgtg caggttcatt 600
   ggctgtggca ggaacgagaa gtttaactat gtagtgatgc agctccaggg ccggaacctg 660
   gccgacctgc gccgtagcca gccgcgaggc accttcacgc tgagcaccac attgcggctg 720
   ggcaagcaga tcttggagtc catcgaggcc atccactctg tgggcttcct gcaccgtgac 780
   atcaagcctt caaactttgc catgggcagg ctgccctcca cctacaggaa gtgctatatg 840
   ctggacttcg ggctggcccg gcagtacacc aacaccacgg gggatgtgcg gccccctcgg 900
   aatgtggccg ggtttcgagg aacggttcgc tatgcctcag tcaatgccca caagaaccgg 960
   gagatgggcc gccacgacga cctgtggtcc ctcttctaca tgctggtgga gtttgcagtg 1020
   ggccagctgc cctggaggaa gatcaaggac aaggaacagg tagggatgat caaggagaag 1080
   tatgagcacc ggatgctgct gaagcacatg ccgtcagagt tccacctctt cctggaccac 1140
   attgccagcc tcgactactt caccaagccc gactaccagt tgatcatgtc agtgtttgag 1200
   aacagcatga aggagagggg cattgccgag aatgaggcct ttgactggga gaaggcaggc 1260
   accgatgccc tcctgtccac gagcacctct accccgcccc agcagaacac ccggcagacg 1320
   gcagccatgt ttggggtggt caatgtgacg ccagtgcctg gggacctgct ccgggagaac 1380
   accgaggatg tgctacaggg agagcacctg agtgaccagg agaatgcacc cccaattctg 1440
   cccgggaggc cctctgaggg gctgggcccc agtccccacc ttgtccccca ccccgggggt 1500
   cctgaggctg aagtctggga ggagacagat gtcaaccgga acaaactccg gatcaacatc 1560
   ggcaaaagcc cctgtgtgga ggaggaacag agccgaggca tgggggtccc cagctcccca 1620
   gtgcgtgccc ccccagactc ccccacaacc ccagtccgtt ctctgcgcta ccggagggtg 1680
   aacagccctg agtcagaaag gctgtccacg gcggacgggc gagtggagct acctgagagg 1740
   aggtcacgga tggatctgcc tggctcgccc tcgcgccagg cctgctcctc tcagccagcc 1800
   cagatgctgt cagtggacac aggccacgct gaccgacagg ccagtggccg catggaygtg 1860
   tcagcctctg tggagcagga ggccctgagc aacgccttcc gctcggtgcc gctggctgag 1920
   gaggaggatt tcgacagcaa agagtgggtc atcatcgaca aggagacgga gctcaaggac 1980
   ttccctccag gggctgagcc cagcacatcg ggcaccacgg atgaggagcc cgaggagctg 2040
   cggccactgc ccgaggaggg cgaagagcgg cggcggctgg gggcagagcc caccgtccgg 2100
   ccccggggac gcagcatgca ggcgctggcg gaggaggacc tgcagcattt gccgccccag 2160
   cccctgccac cccagctgag ccaggscgat ggccgttccg agacgtcaca gccccccacg 2220
   cctggcagcc cttcccactc acccctgcac tcgggacccc gccctcgacg gagagagtcg 2280
   gaccccacag gcccacagag acaggctgga gtgcaatggc gtgatctcgg ctcactgcaa 2340
   cctccacctc ccaggttcaa gcaattctcc tgcctcagcc tcccgagaag ctgggattac 2400
   aggcatgcac caccaccaca cccggctaat tttgtatttt tagtagagac ggggtttctc 2460
   catgttgagg ctggtcttga gctcctgacc tcaggtgatc tgcctgcctc ggcctcccaa 2520
   attgctggga ttacaggcgt gagccatcgc gcccagcctg aggtctgtga gtttaacaga 2580
   aaacatacag gccagagaga gcagatggtt tgtgcaggat cagagagagc ctggagcatg 2640
   cgtgacctgc ccgggcggcc gctcgagccc tatagtgagt cgtattag 2688

<210> 18
<211> 895
<212> PRT
<213> homo sapiens

<220>
<221> VARIANT
<222> (1)...(895)
<223> Xaa = Any Amino Acid

<400> 18
Met Ser Gly Leu Val Leu Met Leu Ala Ala Arg Cys Ile Val Gly Ser
1               5                   10                  15
Ser Pro Leu Cys Arg Cys Arg Arg Arg Arg Pro Arg Arg Ile Gly Ala
            20                  25                  30
Gly Pro Gly Arg Asp Asp Pro Gly Arg Lys Ala Ala Ala Ala Gly Gly
        35                  40                  45
Ser Gly Ser Pro Asn Ala Ala Leu Ser Arg Pro Arg Pro Ala Pro Ala
    50                  55                  60
Pro Gly Asp Ala Pro Pro Arg Ala Ala Ala Ser Ala Ala Ala Ala Ala
65                  70                  75                  80
Ala Ala Ala Ala Gly Thr Glu Gln Val Asp Gly Pro Leu Arg Ala Gly
                85                  90                  95
Pro Ala Asp Thr Pro Pro Ser Gly Trp Arg Met Gln Cys Leu Ala Ala
            100                 105                 110
Ala Leu Lys Asp Glu Thr Asn Met Ser Gly Gly Gly Glu Gln Ala Asp
        115                 120                 125
Ile Leu Pro Ala Asn Tyr Val Val Lys Asp Arg Trp Lys Val Leu Lys
    130                 135                 140
Lys Ile Gly Gly Gly Gly Phe Gly Glu Ile Tyr Glu Ala Met Asp Leu
145                 150                 155                 160
Leu Thr Arg Glu Asn Val Ala Leu Lys Val Glu Ser Ala Gln Gln Pro
                165                 170                 175
Lys Gln Val Leu Lys Met Glu Val Ala Val Leu Lys Lys Leu Gln Gly
            180                 185                 190
Lys Asp His Val Cys Arg Phe Ile Gly Cys Gly Arg Asn Glu Lys Phe
        195                 200                 205
Asn Tyr Val Val Met Gln Leu Gln Gly Arg Asn Leu Ala Asp Leu Arg
    210                 215                 220
Arg Ser Gln Pro Arg Gly Thr Phe Thr Leu Ser Thr Thr Leu Arg Leu
225                 230                 235                 240
Gly Lys Gln Ile Leu Glu Ser Ile Glu Ala Ile His Ser Val Gly Phe
                245                 250                 255
Leu His Arg Asp Ile Lys Pro Ser Asn Phe Ala Met Gly Arg Leu Pro
            260                 265                 270
Ser Thr Tyr Arg Lys Cys Tyr Met Leu Asp Phe Gly Leu Ala Arg Gln
        275                 280                 285
Tyr Thr Asn Thr Thr Gly Asp Val Arg Pro Pro Arg Asn Val Ala Gly
    290                 295                 300
Phe Arg Gly Thr Val Arg Tyr Ala Ser Val Asn Ala His Lys Asn Arg
305                 310                 315                 320
Glu Met Gly Arg His Asp Asp Leu Trp Ser Leu Phe Tyr Met Leu Val
                325                 330                 335
Glu Phe Ala Val Gly Gln Leu Pro Trp Arg Lys Ile Lys Asp Lys Glu
            340                 345                 350
Gln Val Gly Met Ile Lys Glu Lys Tyr Glu His Arg Met Leu Leu Lys
        355                 360                 365
His Met Pro Ser Glu Phe His Leu Phe Leu Asp His Ile Ala Ser Leu
    370                 375                 380
Asp Tyr Phe Thr Lys Pro Asp Tyr Gln Leu Ile Met Ser Val Phe Glu
385                 390                 395                 400
Asn Ser Met Lys Glu Arg Gly Ile Ala Glu Asn Glu Ala Phe Asp Trp
                405                 410                 415
Glu Lys Ala Gly Thr Asp Ala Leu Leu Ser Thr Ser Thr Ser Thr Pro

            420                 425                 430
Pro Gln Gln Asn Thr Arg Gln Thr Ala Ala Met Phe Gly Val Val Asn
        435                 440                 445
Val Thr Pro Val Pro Gly Asp Leu Leu Arg Glu Asn Thr Glu Asp Val
    450                 455                 460
Leu Gln Gly Glu His Leu Ser Asp Gln Glu Asn Ala Pro Pro Ile Leu
465                 470                 475                 480
Pro Gly Arg Pro Ser Glu Gly Leu Gly Pro Ser Pro His Leu Val Pro
                485                 490                 495
His Pro Gly Gly Pro Glu Ala Glu Val Trp Glu Glu Thr Asp Val Asn
            500                 505                 510
Arg Asn Lys Leu Arg Ile Asn Ile Gly Lys Ser Pro Cys Val Glu Glu
        515                 520                 525
Glu Gln Ser Arg Gly Met Gly Val Pro Ser Ser Pro Val Arg Ala Pro
    530                 535                 540
Pro Asp Ser Pro Thr Thr Pro Val Arg Ser Leu Arg Tyr Arg Arg Val
545                 550                 555                 560
Asn Ser Pro Glu Ser Glu Arg Leu Ser Thr Ala Asp Gly Arg Val Glu
                565                 570                 575
Leu Pro Glu Arg Arg Ser Arg Met Asp Leu Pro Gly Ser Pro Ser Arg
            580                 585                 590
Gln Ala Cys Ser Ser Gln Pro Ala Gln Met Leu Ser Val Asp Thr Gly
        595                 600                 605
His Ala Asp Arg Gln Ala Ser Gly Arg Met Asp Val Ser Ala Ser Val
    610                 615                 620
Glu Gln Glu Ala Leu Ser Asn Ala Phe Arg Ser Val Pro Leu Ala Glu
625                 630                 635                 640
Glu Glu Asp Phe Asp Ser Lys Glu Trp Val Ile Ile Asp Lys Glu Thr
                645                 650                 655
Glu Leu Lys Asp Phe Pro Pro Gly Ala Glu Pro Ser Thr Ser Gly Thr
            660                 665                 670
Thr Asp Glu Glu Pro Glu Glu Leu Arg Pro Leu Pro Glu Glu Gly Glu
        675                 680                 685
Glu Arg Arg Arg Leu Gly Ala Glu Pro Thr Val Arg Pro Arg Gly Arg
    690                 695                 700
Ser Met Gln Ala Leu Ala Glu Glu Asp Leu Gln His Leu Pro Pro Gln
705                 710                 715                 720
Pro Leu Pro Pro Gln Leu Ser Gln Xaa Asp Gly Arg Ser Glu Thr Ser
                725                 730                 735
Gln Pro Pro Thr Pro Gly Ser Pro Ser His Ser Pro Leu His Ser Gly
            740                 745                 750
Pro Arg Pro Arg Arg Arg Glu Ser Asp Pro Thr Gly Pro Gln Arg Gln
        755                 760                 765
Ala Gly Val Gln Trp Arg Asp Leu Gly Ser Leu Gln Pro Pro Pro Pro
    770                 775                 780
Arg Phe Lys Gln Phe Ser Cys Leu Ser Leu Pro Arg Ser Trp Asp Tyr
785                 790                 795                 800
Arg His Ala Pro Pro Pro His Pro Ala Asn Phe Val Phe Leu Val Glu
                805                 810                 815
Thr Gly Phe Leu His Val Glu Ala Gly Leu Glu Leu Leu Thr Ser Gly
            820                 825                 830
Asp Leu Pro Ala Ser Ala Ser Gln Ile Ala Gly Ile Thr Gly Val Ser
        835                 840                 845
His Arg Ala Gln Pro Glu Val Cys Glu Phe Asn Arg Lys His Thr Gly
    850                 855                 860
Gln Arg Glu Gln Met Val Cys Ala Gly Ser Glu Arg Ala Trp Ser Met
865                 870                 875                 880
Arg Asp Leu Pro Gly Arg Pro Leu Glu Pro Tyr Ser Glu Ser Tyr
                885                 890                 895

<210> 19
<211> 2670
<212> DNA
<213> homo sapiens

<400> 19
   atgctggcgg cgcggtgcat tgtgggcagc tccccgctct gccgctgccg ccgccgtcgc 60
   ccaaggagga tcggggccgg gccgggccgg gatgatccgg gtcggaaggc cgccgccgcc 120
   ggagggagcg ggtcacccaa cgccgcactg agccgccccc gccccgcccc ggccccgggg 180
   gatgcgccgc cccgagctgc tgcctccgcc gccgccgcag ccgcagccgc agcgggcaca 240
   gagcaggtag atggccccct cagggcaggc ccggcggaca cccctccctc tggctggcgg 300
   atgcagtgcc tagcggccgc ccttaaggac gaaaccaaca tgagtggggg aggggagcag 360
   gccgacatcc tgccggccaa ctacgtggtc aaggatcgct ggaaggtgct gaaaaagatc 420
   gggggcgggg gctttggtga gatctacgag gccatggacc tgctgaccag ggagaatgtg 480
   gccctcaagg tggagtcagc ccagcagccc aagcaggtcc tcaagatgga ggtggccgtg 540
   ctcaagaagt tgcaagggaa ggaccatgtg tgcaggttca ttggctgtgg caggaacgag 600
   aagtttaact atgtagtgat gcagctccag ggccggaacc tggccgacct gcgccgtagc 660
   cagccgcgag gcaccttcac gctgagcacc acattgcggc tgggcaagca gatcttggag 720
   tccatcgagg ccatccactc tgtgggcttc ctgcaccgtg acatcaagcc ttcaaacttt 780
   gccatgggca ggctgccctc cacctacagg aagtgctata tgctggactt cgggctggcc 840
   cggcagtaca ccaacaccac gggggatgtg cggccccctc ggaatgtggc cgggtttcga 900
   ggaacggttc gctatgcctc agtcaatgcc cacaagaacc gggagatggg ccgccacgac 960
   gacctgtggt ccctcttcta catgctggtg gagtttgcag tgggccagct gccctggagg 1020
   aagatcaagg acaaggaaca ggtagggatg atcaaggaga agtatgagca ccggatgctg 1080
   ctgaagcaca tgccgtcaga gttccacctc ttcctggacc acattgccag cctcgactac 1140
   ttcaccaagc ccgactacca gttgatcatg tcagtgtttg agaacagcat gaaggagagg 1200
   ggcattgccg agaatgaggc ctttgactgg gagaaggcag gcaccgatgc cctcctgtcc 1260
   acgagcacct ctaccccgcc ccagcagaac acccggcaga cggcagccat gtttggggtg 1320
   gtcaatgtga cgccagtgcc tggggacctg ctccgggaga acaccgagga tgtgctacag 1380
   ggagagcacc tgagtgacca ggagaatgca cccccaattc tgcccggagg gccctctgag 1440
   gggctgggcc ccagtcccca ccttgtcccc caccccgggg gtcctgaggc tgaagtctgg 1500
   gaggagacag atgtcaaccg gaacaaactc cggatcaaca tcggcaaaag cccctgtgtg 1560
   gaggaggaac agagccgagg catgggggtc cccagctccc cagtgcgtgc ccccccagac 1620
   tcccccacaa ccccagtccg ttctctgcgc taccggaggg tgaacagccc tgagtcagaa 1680
   aggctgtcca cggcggacgg gcgagtggag ctacctgaga ggaggtcacg gatggatctg 1740
   cctggctcgc cctcgcgcca ggcctgctcc tctcagccag cccagatgct gtcagtggac 1800
   acaggccacg ctgaccgaca ggccagtggc cgcatggayg tgtcagcctc tgtggagcag 1860
   gaggccctga gcaacgcctt ccgctcggtg ccgctggctg aggaggagga tttcgacagc 1920
   aaagagtggg tcatcatcga caaggagacg gagctcaagg acttccctcc aggggctgag 1980
   cccagcacat cgggcaccac ggatgaggag cccgaggagc tgcggccact gcccgaggag 2040
   ggcgaagagc ggcggcggct gggggcagag cccaccgtcc ggccccgggg acgcagcatg 2100
   caggcgctgg cggaggagga cctgcagcat ttgccgcccc agcccctgcc accccagctg 2160
   agccaggscg atggccgttc cgagacgtca cagcccccca cgcctggcag cccttcccac 2220
   tcacccctgc actcgggacc ccgccctcga cggagagagt cggaccccac aggcccacag 2280
   agacaggctg gagtgcaatg gcgtgatctc ggctcactgc aacctccacc tcccaggttc 2340
   aagcaattct cctgcctcag cctcccgaga agctgggatt acaggcatgc accaccacca 2400
   cacccggcta attttgtatt tttagtagag acggggtttc tccatgttga ggctggtctt 2460
   gagctcctga cctcaggtga tctgcctgcc tcggcctccc aaattgctgg gattacaggc 2520
   gtgagccatc gcgcccagcc tgaggtctgt gagtttaaca gaaaacatac aggccagaga 2580
   gagcagatgg tttgtgcagg atcagagaga gcctggagca tgcgtgacct gcccgggcgg 2640
   ccgctcgagc cctatagtga gtcgtattag 2670

<210> 20
<211> 889
<212> PRT
<213> homo sapiens

<220>
<221> VARIANT
<222> (1)...(889)
<223> Xaa = Any Amino Acid

<400> 20
Met Leu Ala Ala Arg Cys Ile Val Gly Ser Ser Pro Leu Cys Arg Cys
1               5                   10                  15
Arg Arg Arg Arg Pro Arg Arg Ile Gly Ala Gly Pro Gly Arg Asp Asp
            20                  25                  30
Pro Gly Arg Lys Ala Ala Ala Ala Gly Gly Ser Gly Ser Pro Asn Ala
        35                  40                  45
Ala Leu Ser Arg Pro Arg Pro Ala Pro Ala Pro Gly Asp Ala Pro Pro
    50                  55                  60
Arg Ala Ala Ala Ser Ala Ala Ala Ala Ala Ala Ala Ala Ala Gly Thr
65                  70                  75                  80
Glu Gln Val Asp Gly Pro Leu Arg Ala Gly Pro Ala Asp Thr Pro Pro
                85                  90                  95

Ser Gly Trp Arg Met Gln Cys Leu Ala Ala Ala Leu Lys Asp Glu Thr
            100                 105                 110
Asn Met Ser Gly Gly Gly Glu Gln Ala Asp Ile Leu Pro Ala Asn Tyr
        115                 120                 125
Val Val Lys Asp Arg Trp Lys Val Leu Lys Lys Ile Gly Gly Gly Gly
    130                 135                 140
Phe Gly Glu Ile Tyr Glu Ala Met Asp Leu Leu Thr Arg Glu Asn Val
145                 150                 155                 160
Ala Leu Lys Val Glu Ser Ala Gln Gln Pro Lys Gln Val Leu Lys Met
                165                 170                 175
Glu Val Ala Val Leu Lys Lys Leu Gln Gly Lys Asp His Val Cys Arg
            180                 185                 190
Phe Ile Gly Cys Gly Arg Asn Glu Lys Phe Asn Tyr Val Val Met Gln
        195                 200                 205
Leu Gln Gly Arg Asn Leu Ala Asp Leu Arg Arg Ser Gln Pro Arg Gly
    210                 215                 220
Thr Phe Thr Leu Ser Thr Thr Leu Arg Leu Gly Lys Gln Ile Leu Glu
225                 230                 235                 240
Ser Ile Glu Ala Ile His Ser Val Gly Phe Leu His Arg Asp Ile Lys
                245                 250                 255
Pro Ser Asn Phe Ala Met Gly Arg Leu Pro Ser Thr Tyr Arg Lys Cys
            260                 265                 270
Tyr Met Leu Asp Phe Gly Leu Ala Arg Gln Tyr Thr Asn Thr Thr Gly
        275                 280                 285
Asp Val Arg Pro Pro Arg Asn Val Ala Gly Phe Arg Gly Thr Val Arg
    290                 295                 300
Tyr Ala Ser Val Asn Ala His Lys Asn Arg Glu Met Gly Arg His Asp
305                 310                 315                 320
Asp Leu Trp Ser Leu Phe Tyr Met Leu Val Glu Phe Ala Val Gly Gln
                325                 330                 335
Leu Pro Trp Arg Lys Ile Lys Asp Lys Glu Gln Val Gly Met Ile Lys
            340                 345                 350
Glu Lys Tyr Glu His Arg Met Leu Leu Lys His Met Pro Ser Glu Phe
        355                 360                 365
His Leu Phe Leu Asp His Ile Ala Ser Leu Asp Tyr Phe Thr Lys Pro
    370                 375                 380
Asp Tyr Gln Leu Ile Met Ser Val Phe Glu Asn Ser Met Lys Glu Arg
385                 390                 395                 400
Gly Ile Ala Glu Asn Glu Ala Phe Asp Trp Glu Lys Ala Gly Thr Asp
                405                 410                 415
Ala Leu Leu Ser Thr Ser Thr Ser Thr Pro Pro Gln Gln Asn Thr Arg
            420                 425                 430
Gln Thr Ala Ala Met Phe Gly Val Val Asn Val Thr Pro Val Pro Gly
        435                 440                 445
Asp Leu Leu Arg Glu Asn Thr Glu Asp Val Leu Gln Gly Glu His Leu
    450                 455                 460
Ser Asp Gln Glu Asn Ala Pro Pro Ile Leu Pro Gly Arg Pro Ser Glu
465                 470                 475                 480
Gly Leu Gly Pro Ser Pro His Leu Val Pro His Pro Gly Gly Pro Glu
                485                 490                 495
Ala Glu Val Trp Glu Glu Thr Asp Val Asn Arg Asn Lys Leu Arg Ile
            500                 505                 510
Asn Ile Gly Lys Ser Pro Cys Val Glu Glu Glu Gln Ser Arg Gly Met
        515                 520                 525
Gly Val Pro Ser Ser Pro Val Arg Ala Pro Pro Asp Ser Pro Thr Thr
    530                 535                 540
Pro Val Arg Ser Leu Arg Tyr Arg Arg Val Asn Ser Pro Glu Ser Glu
545                 550                 555                 560
Arg Leu Ser Thr Ala Asp Gly Arg Val Glu Leu Pro Glu Arg Arg Ser
                565                 570                 575
Arg Met Asp Leu Pro Gly Ser Pro Ser Arg Gln Ala Cys Ser Ser Gln
            580                 585                 590
Pro Ala Gln Met Leu Ser Val Asp Thr Gly His Ala Asp Arg Gln Ala
        595                 600                 605
Ser Gly Arg Met Asp Val Ser Ala Ser Val Glu Gln Glu Ala Leu Ser
    610                 615                 620
Asn Ala Phe Arg Ser Val Pro Leu Ala Glu Glu Glu Asp Phe Asp Ser
625                 630                 635                 640

Lys Glu Trp Val Ile Ile Asp Lys Glu Thr Glu Leu Lys Asp Phe Pro
                645                 650                 655
Pro Gly Ala Glu Pro Ser Thr Ser Gly Thr Thr Asp Glu Glu Pro Glu
            660                 665                 670
Glu Leu Arg Pro Leu Pro Glu Glu Gly Glu Glu Arg Arg Arg Leu Gly
        675                 680                 685
Ala Glu Pro Thr Val Arg Pro Arg Gly Arg Ser Met Gln Ala Leu Ala
    690                 695                 700
Glu Glu Asp Leu Gln His Leu Pro Pro Gln Pro Leu Pro Pro Gln Leu
705                 710                 715                 720
Ser Gln Xaa Asp Gly Arg Ser Glu Thr Ser Gln Pro Pro Thr Pro Gly
                725                 730                 735
Ser Pro Ser His Ser Pro Leu His Ser Gly Pro Arg Pro Arg Arg Arg
            740                 745                 750
Glu Ser Asp Pro Thr Gly Pro Gln Arg Gln Ala Gly Val Gln Trp Arg
        755                 760                 765
Asp Leu Gly Ser Leu Gln Pro Pro Pro Pro Arg Phe Lys Gln Phe Ser
    770                 775                 780
Cys Leu Ser Leu Pro Arg Ser Trp Asp Tyr Arg His Ala Pro Pro Pro
785                 790                 795                 800
His Pro Ala Asn Phe Val Phe Leu Val Glu Thr Gly Phe Leu His Val
                805                 810                 815
Glu Ala Gly Leu Glu Leu Leu Thr Ser Gly Asp Leu Pro Ala Ser Ala
            820                 825                 830
Ser Gln Ile Ala Gly Ile Thr Gly Val Ser His Arg Ala Gln Pro Glu
        835                 840                 845
Val Cys Glu Phe Asn Arg Lys His Thr Gly Gln Arg Glu Gln Met Val
    850                 855                 860
Cys Ala Gly Ser Glu Arg Ala Trp Ser Met Arg Asp Leu Pro Gly Arg
865                 870                 875                 880
Pro Leu Glu Pro Tyr Ser Glu Ser Tyr
                885

<210> 21
<211> 2370
<212> DNA
<213> homo sapiens

<400> 21
atgcagtgcc tagcggccgc ccttaaggac gaaaccaaca tgagtggggg aggggagcag 60
gccgacatcc tgccggccaa ctacgtggtc aaggatcgct ggaaggtgct gaaaaagatc 120
gggggcgggg gctttggtga gatctacgag gccatggacc tgctgaccag ggagaatgtg 180
gccctcaagg tggagtcagc ccagcagccc aagcaggtcc tcaagatgga ggtggccgtg 240
ctcaagaagt tgcaagggaa ggaccatgtg tgcaggttca ttggctgtgg caggaacgag 300
aagtttaact atgtagtgat gcagctccag ggccggaacc tggccgacct gcgccgtagc 360
cagccgcgag gcaccttcac gctgagcacc acattgcggc tgggcaagca gatcttggag 420
tccatcgagg ccatccactc tgtgggcttc ctgcaccgtg acatcaagcc ttcaaacttt 480
gccatgggca ggctgccctc cacctacagg aagtgctata tgctggactt cgggctggcc 540
cggcagtaca ccaacaccac gggggatgtg cggccccctc ggaatgtggc cgggtttcga 600
ggaacggttc gctatgcctc agtcaatgcc cacaagaacc gggagatggg ccgccacgac 660
gacctgtggt ccctcttcta catgctggtg gagtttgcag tgggccagct gccctggagg 720
aagatcaagg acaaggaaca ggtagggatg atcaaggaga agtatgagca ccggatgctg 780
ctgaagcaca tgccgtcaga gttccacctc ttcctggacc acattgccag cctcgactac 840
ttcaccaagc ccgactacca gttgatcatg tcagtgtttg agaacagcat gaaggagagg 900
ggcattgccg agaatgaggc ctttgactgg gagaaggcag gcaccgatgc cctcctgtcc 960
acgagcacct ctaccccgcc ccagcagaac acccggcaga cggcagccat gtttggggtg 1020
gtcaatgtga cgccagtgcc tggggacctg ctccgggaga acaccgagga tgtgctacag 1080
ggagagcacc tgagtgacca ggagaatgca cccccaattc tgcccgggag gccctctgag 1140
gggctgggcc ccagtcccca ccttgtcccc caccccgggg gtcctgaggc tgaagtctgg 1200
gaggagacag atgtcaaccg gaacaaactc cggatcaaca tcggcaaaag cccctgtgtg 1260
gaggaggaac agagccgagg catgggggtc cccagctccc cagtgcgtgc ccccccagac 1320
tcccccacaa ccccagtccg ttctctgcgc taccggaggg tgaacagccc tgagtcagaa 1380
aggctgtcca cggcggacgg gcgagtggag ctacctgaga ggaggtcacg gatggatctg 1440
cctggctcgc cctcgcgcca ggcctgctcc tctcagccag cccagatgct gtcagtggac 1500
acaggccacg ctgaccgaca ggccagtggc cgcatggayg tgtcagcctc tgtggagcag 1560
gaggccctga gcaacgcctt ccgctcggtg ccgctggctg aggaggagga tttcgacagc 1620
aaagagtggg tcatcatcga caaggagacg gagctcaagg acttccctcc aggggctgag 1680

cccagcacat cgggcaccac ggatgaggag cccgaggagc tgcggccact gcccgaggag 1740
ggcgaagagc ggcggcggct gggggcagag cccaccgtcc ggccccgggg acgcagcatg 1800
caggcgctgg cggaggagga cctgcagcat ttgccgcccc agcccctgcc accccagctg 1860
agccaggscg atggccgttc cgagacgtca cagcccccca cgcctggcag cccttcccac 1920
tcacccctgc actcgggacc ccgccctcga cggagagagt cggaccccac aggcccacag 1980
agacaggctg gagtgcaatg gcgtgatctc ggctcactgc aacctccacc tcccaggttc 2040
aagcaattct cctgcctcag cctcccgaga agctgggatt acaggcatgc accaccacca 2100
cacccggcta attttgtatt tttagtagag acggggtttc tccatgttga ggctggtctt 2160
gagctcctga cctcaggtga tctgcctgcc tcggcctccc aaattgctgg gattacaggc 2220
gtgagccatc gcgcccagcc tgaggtctgt gagtttaaca gaaaacatac aggccagaga 2280
gagcagatgg tttgtgcagg atcagagaga gcctggagca tgcgtgacct gcccgggcgg 2340
ccgctcgagc cctatagtga gtcgtattag 2370

<210> 22
<211> 789
<212> PRT
<213> homo sapiens

<220>
<221> VARIANT
<222> (1)...(789)
<223> Xaa = Any Amino Acid

<400> 22
Met Gln Cys Leu Ala Ala Ala Leu Lys Asp Glu Thr Asn Met Ser Gly
1               5                   10                  15
Gly Gly Glu Gln Ala Asp Ile Leu Pro Ala Asn Tyr Val Val Lys Asp
            20                  25                  30
Arg Trp Lys Val Leu Lys Lys Ile Gly Gly Gly Gly Phe Gly Glu Ile
        35                  40                  45
Tyr Glu Ala Met Asp Leu Leu Thr Arg Glu Asn Val Ala Leu Lys Val
    50                  55                  60
Glu Ser Ala Gln Gln Pro Lys Gln Val Leu Lys Met Glu Val Ala Val
65                  70                  75                  80
Leu Lys Lys Leu Gln Gly Lys Asp His Val Cys Arg Phe Ile Gly Cys
                85                  90                  95
Gly Arg Asn Glu Lys Phe Asn Tyr Val Val Met Gln Leu Gln Gly Arg
            100                 105                 110
Asn Leu Ala Asp Leu Arg Arg Ser Gln Pro Arg Gly Thr Phe Thr Leu
        115                 120                 125
Ser Thr Thr Leu Arg Leu Gly Lys Gln Ile Leu Glu Ser Ile Glu Ala
    130                 135                 140
Ile His Ser Val Gly Phe Leu His Arg Asp Ile Lys Pro Ser Asn Phe
145                 150                 155                 160
Ala Met Gly Arg Leu Pro Ser Thr Tyr Arg Lys Cys Tyr Met Leu Asp
                165                 170                 175
Phe Gly Leu Ala Arg Gln Tyr Thr Asn Thr Thr Gly Asp Val Arg Pro
            180                 185                 190
Pro Arg Asn Val Ala Gly Phe Arg Gly Thr Val Arg Tyr Ala Ser Val
        195                 200                 205
Asn Ala His Lys Asn Arg Glu Met Gly Arg His Asp Asp Leu Trp Ser
    210                 215                 220
Leu Phe Tyr Met Leu Val Glu Phe Ala Val Gly Gln Leu Pro Trp Arg
225                 230                 235                 240
Lys Ile Lys Asp Lys Glu Gln Val Gly Met Ile Lys Glu Lys Tyr Glu
                245                 250                 255
His Arg Met Leu Leu Lys His Met Pro Ser Glu Phe His Leu Phe Leu
            260                 265                 270
Asp His Ile Ala Ser Leu Asp Tyr Phe Thr Lys Pro Asp Tyr Gln Leu
        275                 280                 285
Ile Met Ser Val Phe Glu Asn Ser Met Lys Glu Arg Gly Ile Ala Glu
    290                 295                 300
Asn Glu Ala Phe Asp Trp Glu Lys Ala Gly Thr Asp Ala Leu Leu Ser
305                 310                 315                 320
Thr Ser Thr Ser Thr Pro Pro Gln Gln Asn Thr Arg Gln Thr Ala Ala
                325                 330                 335
Met Phe Gly Val Val Asn Val Thr Pro Val Pro Gly Asp Leu Leu Arg
            340                 345                 350

Glu Asn Thr Glu Asp Val Leu Gln Gly Glu His Leu Ser Asp Gln Glu
        355                 360                 365
Asn Ala Pro Pro Ile Leu Pro Gly Arg Pro Ser Glu Gly Leu Gly Pro
    370                 375                 380
Ser Pro His Leu Val Pro His Pro Gly Gly Pro Glu Ala Glu Val Trp
385                 390                 395                 400
Glu Glu Thr Asp Val Asn Arg Asn Lys Leu Arg Ile Asn Ile Gly Lys
                405                 410                 415
Ser Pro Cys Val Glu Glu Glu Gln Ser Arg Gly Met Gly Val Pro Ser
            420                 425                 430
Ser Pro Val Arg Ala Pro Pro Asp Ser Pro Thr Thr Pro Val Arg Ser
        435                 440                 445
Leu Arg Tyr Arg Arg Val Asn Ser Pro Glu Ser Glu Arg Leu Ser Thr
    450                 455                 460
Ala Asp Gly Arg Val Glu Leu Pro Glu Arg Arg Ser Arg Met Asp Leu
465                 470                 475                 480
Pro Gly Ser Pro Ser Arg Gln Ala Cys Ser Ser Gln Pro Ala Gln Met
                485                 490                 495
Leu Ser Val Asp Thr Gly His Ala Asp Arg Gln Ala Ser Gly Arg Met
            500                 505                 510
Asp Val Ser Ala Ser Val Glu Gln Glu Ala Leu Ser Asn Ala Phe Arg
        515                 520                 525
Ser Val Pro Leu Ala Glu Glu Glu Asp Phe Asp Ser Lys Glu Trp Val
    530                 535                 540
Ile Ile Asp Lys Glu Thr Glu Leu Lys Asp Phe Pro Pro Gly Ala Glu
545                 550                 555                 560
Pro Ser Thr Ser Gly Thr Thr Asp Glu Glu Pro Glu Glu Leu Arg Pro
                565                 570                 575
Leu Pro Glu Glu Gly Glu Glu Arg Arg Arg Leu Gly Ala Glu Pro Thr
            580                 585                 590
Val Arg Pro Arg Gly Arg Ser Met Gln Ala Leu Ala Glu Glu Asp Leu
        595                 600                 605
Gln His Leu Pro Pro Gln Pro Leu Pro Pro Gln Leu Ser Gln Xaa Asp
    610                 615                 620
Gly Arg Ser Glu Thr Ser Gln Pro Pro Thr Pro Gly Ser Pro Ser His
625                 630                 635                 640
Ser Pro Leu His Ser Gly Pro Arg Pro Arg Arg Arg Glu Ser Asp Pro
                645                 650                 655
Thr Gly Pro Gln Arg Gln Ala Gly Val Gln Trp Arg Asp Leu Gly Ser
            660                 665                 670
Leu Gln Pro Pro Pro Pro Arg Phe Lys Gln Phe Ser Cys Leu Ser Leu
        675                 680                 685
Pro Arg Ser Trp Asp Tyr Arg His Ala Pro Pro Pro His Pro Ala Asn
    690                 695                 700
Phe Val Phe Leu Val Glu Thr Gly Phe Leu His Val Glu Ala Gly Leu
705                 710                 715                 720
Glu Leu Leu Thr Ser Gly Asp Leu Pro Ala Ser Ala Ser Gln Ile Ala
                725                 730                 735
Gly Ile Thr Gly Val Ser His Arg Ala Gln Pro Glu Val Cys Glu Phe
            740                 745                 750
Asn Arg Lys His Thr Gly Gln Arg Glu Gln Met Val Cys Ala Gly Ser
        755                 760                 765
Glu Arg Ala Trp Ser Met Arg Asp Leu Pro Gly Arg Pro Leu Glu Pro
    770                 775                 780
Tyr Ser Glu Ser Tyr
785

<210> 23
<211> 2331
<212> DNA
<213> homo sapiens

<400> 23
   atgagtgggg gaggggagca ggccgacatc ctgccggcca actacgtggt caaggatcgc 60
   tggaaggtgc tgaaaaagat cgggggcggg ggctttggtg agatctacga ggccatggac 120
   ctgctgacca gggagaatgt ggccctcaag gtggagtcag cccagcagcc caagcaggtc 180
   ctcaagatgg aggtggccgt gctcaagaag ttgcaaggga aggaccatgt gtgcaggttc 240
   attggctgtg gcaggaacga gaagtttaac tatgtagtga tgcagctcca gggccggaac 300
   ctggccgacc tgcgccgtag ccagccgcga ggcaccttca cgctgagcac cacattgcgg 360
   ctgggcaagc agatcttgga gtccatcgag gccatccact ctgtgggctt cctgcaccgt 420
   gacatcaagc cttcaaactt tgccatgggc aggctgccct ccacctacag gaagtgctat 480
   atgctggact tcgggctggc ccggcagtac accaacacca cgggggatgt gcggccccct 540
   cggaatgtgg ccgggtttcg aggaacggtt cgctatgcct cagtcaatgc ccacaagaac 600
   cgggagatgg gccgccacga cgacctgtgg tccctcttct acatgctggt ggagtttgca 660
   gtgggccagc tgccctggag gaagatcaag gacaaggaac aggtagggat gatcaaggag 720
   aagtatgagc accggatgct gctgaagcac atgccgtcag agttccacct cttcctggac 780
   cacattgcca gcctcgacta cttcaccaag cccgactacc agttgatcat gtcagtgttt 840
   gagaacagca tgaaggagag gggcattgcc gagaatgagg cctttgactg ggagaaggca 900
   ggcaccgatg ccctcctgtc cacgagcacc tctaccccgc cccagcagaa cacccggcag 960
   acggcagcca tgtttggggt ggtcaatgtg acgccagtgc ctggggacct gctccgggag 1020
   aacaccgagg atgtgctaca gggagagcac ctgagtgacc aggagaatgc acccccaatt 1080
   ctgcccggga ggccctctga ggggctgggc cccagtcccc accttgtccc ccaccccggg 1140
   ggtcctgagg ctgaagtctg ggaggagaca gatgtcaacc ggaacaaact ccggatcaac 1200
   atcggcaaaa gcccctgtgt ggaggaggaa cagagccgag gcatgggggt ccccagctcc 1260
   ccagtgcgtg cccccccaga ctcccccaca accccagtcc gttctctgcg ctaccggagg 1320
   gtgaacagcc ctgagtcaga aaggctgtcc acggcggacg ggcgagtgga gctacctgag 1380
   aggaggtcac ggatggatct gcctggctcg ccctcgcgcc aggcctgctc ctctcagcca 1440
   gcccagatgc tgtcagtgga cacaggccac gctgaccgac aggccagtgg ccgcatggay 1500
   gtgtcagcct ctgtggagca ggaggccctg agcaacgcct tccgctcggt gccgctggct 1560
   gaggaggagg atttcgacag caaagagtgg gtcatcatcg acaaggagac ggagctcaag 1620
   gacttccctc caggggctga gcccagcaca tcgggcacca cggatgagga gcccgaggag 1680
   ctgcggccac tgcccgagga gggcgaagag cggcggcggc tgggggcaga gcccaccgtc 1740
   cggccccggg gacgcagcat gcaggcgctg gcggaggagg acctgcagca tttgccgccc 1800
   cagcccctgc caccccagct gagccaggsc gatggccgtt ccgagacgtc acagcccccc 1860
   acgcctggca gcccttccca ctcacccctg cactcgggac ccgccctcgc acggagagag 1920
   tcggacccca caggcccaca gagacaggct ggagtgcaat ggcgtgatct cggctcactg 1980
   caacctccac ctcccaggtt caagcaattc tcctgcctca gcctcccgag aagctgggat 2040
   tacaggcatg caccaccacc acacccggct aattttgtat ttttagtaga gacggggttt 2100
   ctccatgttg aggctggtct tgagctcctg acctcaggtg atctgcctgc ctcggcctcc 2160
   caaattgctg ggattacagg cgtgagccat cgcgcccagc ctgaggtctg tgagtttaac 2220
   agaaaacata caggccagag agagcagatg gtttgtgcag gatcagagag agcctggagc 2280
   atgcgtgacc tgcccgggcg gccgctcgag ccctatagtg agtcgtatta g 2331

<210> 24
<211> 776
<212> PRT
<213> homo sapiens

<220>
<221> VARIANT
<222> (1)...(776)
<223> Xaa = Any Amino Acid

<400> 24
Met Ser Gly Gly Gly Glu Gln Ala Asp Ile Leu Pro Ala Asn Tyr Val
1               5                   10                  15
Val Lys Asp Arg Trp Lys Val Leu Lys Lys Ile Gly Gly Gly Gly Phe
            20                  25                  30
Gly Glu Ile Tyr Glu Ala Met Asp Leu Leu Thr Arg Glu Asn Val Ala
        35                  40                  45
Leu Lys Val Glu Ser Ala Gln Gln Pro Lys Gln Val Leu Lys Met Glu
    50                  55                  60
Val Ala Val Leu Lys Lys Leu Gln Gly Lys Asp His Val Cys Arg Phe
65                  70                  75                  80
Ile Gly Cys Gly Arg Asn Glu Lys Phe Asn Tyr Val Val Met Gln Leu
                85                  90                  95
Gln Gly Arg Asn Leu Ala Asp Leu Arg Arg Ser Gln Pro Arg Gly Thr
            100                 105                 110
Phe Thr Leu Ser Thr Thr Leu Arg Leu Gly Lys Gln Ile Leu Glu Ser
        115                 120                 125
Ile Glu Ala Ile His Ser Val Gly Phe Leu His Arg Asp Ile Lys Pro
    130                 135                 140
Ser Asn Phe Ala Met Gly Arg Leu Pro Ser Thr Tyr Arg Lys Cys Tyr
145                 150                 155                 160
Met Leu Asp Phe Gly Leu Ala Arg Gln Tyr Thr Asn Thr Thr Gly Asp

                165                 170                 175
Val Arg Pro Pro Arg Asn Val Ala Gly Phe Arg Gly Thr Val Arg Tyr
            180                 185                 190
Ala Ser Val Asn Ala His Lys Asn Arg Glu Met Gly Arg His Asp Asp
        195                 200                 205
Leu Trp Ser Leu Phe Tyr Met Leu Val Glu Phe Ala Val Gly Gln Leu
    210                 215                 220
Pro Trp Arg Lys Ile Lys Asp Lys Glu Gln Val Gly Met Ile Lys Glu
225                 230                 235                 240
Lys Tyr Glu His Arg Met Leu Leu Lys His Met Pro Ser Glu Phe His
                245                 250                 255
Leu Phe Leu Asp His Ile Ala Ser Leu Asp Tyr Phe Thr Lys Pro Asp
            260                 265                 270
Tyr Gln Leu Ile Met Ser Val Phe Glu Asn Ser Met Lys Glu Arg Gly
        275                 280                 285
Ile Ala Glu Asn Glu Ala Phe Asp Trp Glu Lys Ala Gly Thr Asp Ala
    290                 295                 300
Leu Leu Ser Thr Ser Thr Ser Thr Pro Pro Gln Gln Asn Thr Arg Gln
305                 310                 315                 320
Thr Ala Ala Met Phe Gly Val Val Asn Val Thr Pro Val Pro Gly Asp
                325                 330                 335
Leu Leu Arg Glu Asn Thr Glu Asp Val Leu Gln Gly Glu His Leu Ser
            340                 345                 350
Asp Gln Glu Asn Ala Pro Pro Ile Leu Pro Gly Arg Pro Ser Glu Gly
        355                 360                 365
Leu Gly Pro Ser Pro His Leu Val Pro His Pro Gly Gly Pro Glu Ala
    370                 375                 380
Glu Val Trp Glu Glu Thr Asp Val Asn Arg Asn Lys Leu Arg Ile Asn
385                 390                 395                 400
Ile Gly Lys Ser Pro Cys Val Glu Glu Glu Gln Ser Arg Gly Met Gly
                405                 410                 415
Val Pro Ser Ser Pro Val Arg Ala Pro Pro Asp Ser Pro Thr Thr Pro
            420                 425                 430
Val Arg Ser Leu Arg Tyr Arg Arg Val Asn Ser Pro Glu Ser Glu Arg
        435                 440                 445
Leu Ser Thr Ala Asp Gly Arg Val Glu Leu Pro Glu Arg Arg Ser Arg
    450                 455                 460
Met Asp Leu Pro Gly Ser Pro Ser Arg Gln Ala Cys Ser Ser Gln Pro
465                 470                 475                 480
Ala Gln Met Leu Ser Val Asp Thr Gly His Ala Asp Arg Gln Ala Ser
                485                 490                 495
Gly Arg Met Asp Val Ser Ala Ser Val Glu Gln Glu Ala Leu Ser Asn
            500                 505                 510
Ala Phe Arg Ser Val Pro Leu Ala Glu Glu Glu Asp Phe Asp Ser Lys
        515                 520                 525
Glu Trp Val Ile Ile Asp Lys Glu Thr Glu Leu Lys Asp Phe Pro Pro
    530                 535                 540
Gly Ala Glu Pro Ser Thr Ser Gly Thr Thr Asp Glu Glu Pro Glu Glu
545                 550                 555                 560
Leu Arg Pro Leu Pro Glu Glu Gly Glu Glu Arg Arg Arg Leu Gly Ala
                565                 570                 575
Glu Pro Thr Val Arg Pro Arg Gly Arg Ser Met Gln Ala Leu Ala Glu
            580                 585                 590
Glu Asp Leu Gln His Leu Pro Pro Gln Pro Leu Pro Pro Gln Leu Ser
        595                 600                 605
Gln Xaa Asp Gly Arg Ser Glu Thr Ser Gln Pro Pro Thr Pro Gly Ser
    610                 615                 620
Pro Ser His Ser Pro Leu His Ser Gly Pro Arg Pro Arg Arg Arg Glu
625                 630                 635                 640
Ser Asp Pro Thr Gly Pro Gln Arg Gln Ala Gly Val Gln Trp Arg Asp
                645                 650                 655
Leu Gly Ser Leu Gln Pro Pro Pro Pro Arg Phe Lys Gln Phe Ser Cys
            660                 665                 670
Leu Ser Leu Pro Arg Ser Trp Asp Tyr Arg His Ala Pro Pro Pro His
        675                 680                 685
Pro Ala Asn Phe Val Phe Leu Val Glu Thr Gly Phe Leu His Val Glu
    690                 695                 700
Ala Gly Leu Glu Leu Leu Thr Ser Gly Asp Leu Pro Ala Ser Ala Ser

705                 710                 715                 720
Gln Ile Ala Gly Ile Thr Gly Val Ser His Arg Ala Gln Pro Glu Val
                725                 730                 735
Cys Glu Phe Asn Arg Lys His Thr Gly Gln Arg Glu Gln Met Val Cys
            740                 745                 750
Ala Gly Ser Glu Arg Ala Trp Ser Met Arg Asp Leu Pro Gly Arg Pro
        755                 760                 765
Leu Glu Pro Tyr Ser Glu Ser Tyr
    770                 775

<210> 25
<211> 2949
<212> DNA
<213> homo sapiens

<400> 25
atgtcagggc tggtgctgat gctggcggcg cggtgcattg tgggcagctc cccgctctgc 60
cgctgccgcc gccgtcgccc aaggaggatc ggggccgggc cgggccggga tgatccgggt 120
cggaaggccg ccgccgccgg agggagcggg tcacccaacg ccgcactgag ccgcccccgc 180
cccgccccgg ccccggggga tgcgccgccc cgagctgctg cctccgccgc cgccgcagcc 240
gcagccgcag cgggcacaga gcaggtagat ggcccccctca gggcaggccc ggcggacacc 300
cctccctctg gctggcggat gcagtgccta gcggccgccc ttaaggacga aaccaacatg 360
agtgggggag gggagcaggc cgacatcctg ccggccaact acgtggtcaa ggatcgctgg 420
aaggtgctga aaaagatcgg gggcgggggc tttggtgaga tctacgaggc catggacctg 480
ctgaccaggg agaatgtggc cctcaaggtg gagtcagccc agcagcccaa gcaggtcctc 540
aagatggagg tggccgtgct caagaagttg caagggaagg accatgtgtg caggttcatt 600
ggctgtggca ggaacgagaa gtttaactat gtagtgatgc agctccaggg ccggaacctg 660
gccgacctgc gccgtagcca gccgcgaggc accttcacgc tgagcaccac attgcggctg 720
ggcaagcaga tcttggagtc catcgaggcc atccactctg tgggcttcct gcaccgtgac 780
atcaagcctt caaactttgc catgggcagg ctgccctcca cctacaggaa gtgctatatg 840
ctggacttcg ggctggcccg gcagtacacc aacaccacgg gggatgtgcg gccccctcgg 900
aatgtggccg ggtttcgagg aacggttcgc tatgcctcag tcaatgccca caagaaccgg 960
gagatgggcc gccacgacga cctgtggtcc ctcttctaca tgctggtgga gtttgcagtg 1020
ggccagctgc cctggaggaa gatcaaggac aaggaacagg tagggatgat caaggagaag 1080
tatgagcacc ggatgctgct gaagcacatg ccgtcagagt tccacctctt cctggaccac 1140
attgccagcc tcgactactt caccaagccc gactaccagt tgatcatgtc agtgtttgag 1200
aacagcatga aggagagggg cattgccgag aatgaggcct ttgactggga gaaggcaggc 1260
accgatgccc tcctgtccac gagcacctct accccgcccc agcagaacac ccggcagacg 1320
gcagccatgt ttggggtggt caatgtgacg ccagtgcctg gggacctgct ccgggagaac 1380
accgaggatg tgctacaggg agagcacctg agtgaccagg agaatgcacc cccaattctg 1440
cccgggaggc cctctgaggg gctgggcccc agtccccacc ttgtccccca ccccgggggt 1500
cctgaggctg aagtctggga ggagacagat gtcaaccgga acaaactccg gatcaacatc 1560
ggcaaagtaa ctgccgccag ggcgaagggc gtgggtggcc ttttctctca cccccgattc 1620
ccagccttgt gcccctgccc tgttcctcct aagcaccctg tccccggcca tctgcctgct 1680
tgccctgcct ctgtttcccg gtccctcccc gcactagcct cgctgtgtct tccatcatca 1740
tcatcctctg tctccttcac cctgaggaga ccatccgccc acagccgcct catcagcccc 1800
agctcatggc actcccctct cctgcagagc ccctgtgtgg aggaggaaca gagccgaggc 1860
atgggggtcc ccagctcccc agtgcgtgcc cccccagact cccccacaac cccagtccgt 1920
tctctgcgct accggagggt gaacagccct gagtcagaaa ggctgtccac ggcggacggg 1980
cgagtggagc tacctgagag gaggtcacgg atggatctgc ctggctcgcc ctcgcgccag 2040
gcctgctcct ctcagccagc ccagatgctg tcagtggaca caggccacgc tgaccgacag 2100
gccagtggcc gcatggaygt gtcagcctct gtggagcagg aggccctgag caacgccttc 2160
cgctcggtgc cgctggctga ggaggaggat ttcgacagca aagagtgggt catcatcgac 2220
aaggagacgg agctcaagga cttccctcca ggggctgagc ccagcacatc gggcaccacg 2280
gatgaggagc ccgaggagct gcggccactg cccgaggagg gcgaagagcg gcggcggctg 2340
ggggcagagc ccaccgtccg gccccgggga cgcagcatgc aggcgctggc ggaggaggac 2400
ctgcagcatt tgccgcccca gcccctgcca ccccagctga gccaggscga tggccgttcc 2460
gagacgtcac agcccccccac gcctggcagc ccttcccact cacccctgca ctcgggaccc 2520
cgccctcgac ggagagagtc ggaccccaca ggcccacaga gacaggctgg agtgcaatgg 2580
cgtgatctcg gctcactgca acctccacct cccaggttca agcaattctc ctgcctcagc 2640
ctcccgagaa gctgggatta caggcatgca ccaccaccac acccggctaa ttttgtattt 2700
ttagtagaga cggggtttct ccatgttgag gctggtcttg agctcctgac ctcaggtgat 2760
ctgcctgcct cggcctccca aattgctggg attacaggcg tgagccatcg cgcccagcct 2820
gaggtctgtg agtttaacag aaaacataca ggccagagag agcagatggt ttgtgcagga 2880
tcagagagag cctggagcat gcgtgacctg cccgggcggc cgctcgagcc ctatagtgag 2940
tcgtattag 2949

<210> 26
<211> 982
<212> PRT
<213> homo sapiens

<220>
<221> VARIANT
<222> (1)...(982)
<223> Xaa = Any Amino Acid

<400> 26
Met Ser Gly Leu Val Leu Met Leu Ala Ala Arg Cys Ile Val Gly Ser
1               5                   10                  15
Ser Pro Leu Cys Arg Cys Arg Arg Arg Arg Pro Arg Arg Ile Gly Ala
            20                  25                  30
Gly Pro Gly Arg Asp Asp Pro Gly Arg Lys Ala Ala Ala Ala Gly Gly
        35                  40                  45
Ser Gly Ser Pro Asn Ala Ala Leu Ser Arg Pro Arg Pro Ala Pro Ala
    50                  55                  60
Pro Gly Asp Ala Pro Pro Arg Ala Ala Ala Ser Ala Ala Ala Ala Ala
65                  70                  75                  80
Ala Ala Ala Ala Gly Thr Glu Gln Val Asp Gly Pro Leu Arg Ala Gly
                85                  90                  95
Pro Ala Asp Thr Pro Pro Ser Gly Trp Arg Met Gln Cys Leu Ala Ala
            100                 105                 110
Ala Leu Lys Asp Glu Thr Asn Met Ser Gly Gly Gly Glu Gln Ala Asp
        115                 120                 125
Ile Leu Pro Ala Asn Tyr Val Val Lys Asp Arg Trp Lys Val Leu Lys
    130                 135                 140
Lys Ile Gly Gly Gly Gly Phe Gly Glu Ile Tyr Glu Ala Met Asp Leu
145                 150                 155                 160
Leu Thr Arg Glu Asn Val Ala Leu Lys Val Glu Ser Ala Gln Gln Pro
                165                 170                 175
Lys Gln Val Leu Lys Met Glu Val Ala Val Leu Lys Lys Leu Gln Gly
            180                 185                 190
Lys Asp His Val Cys Arg Phe Ile Gly Cys Gly Arg Asn Glu Lys Phe
        195                 200                 205
Asn Tyr Val Val Met Gln Leu Gln Gly Arg Asn Leu Ala Asp Leu Arg
    210                 215                 220
Arg Ser Gln Pro Arg Gly Thr Phe Thr Leu Ser Thr Thr Leu Arg Leu
225                 230                 235                 240
Gly Lys Gln Ile Leu Glu Ser Ile Glu Ala Ile His Ser Val Gly Phe
                245                 250                 255
Leu His Arg Asp Ile Lys Pro Ser Asn Phe Ala Met Gly Arg Leu Pro
            260                 265                 270
Ser Thr Tyr Arg Lys Cys Tyr Met Leu Asp Phe Gly Leu Ala Arg Gln
        275                 280                 285
Tyr Thr Asn Thr Thr Gly Asp Val Arg Pro Pro Arg Asn Val Ala Gly
    290                 295                 300
Phe Arg Gly Thr Val Arg Tyr Ala Ser Val Asn Ala His Lys Asn Arg
305                 310                 315                 320
Glu Met Gly Arg His Asp Asp Leu Trp Ser Leu Phe Tyr Met Leu Val
                325                 330                 335
Glu Phe Ala Val Gly Gln Leu Pro Trp Arg Lys Ile Lys Asp Lys Glu
            340                 345                 350
Gln Val Gly Met Ile Lys Glu Lys Tyr Glu His Arg Met Leu Leu Lys
        355                 360                 365
His Met Pro Ser Glu Phe His Leu Phe Leu Asp His Ile Ala Ser Leu
    370                 375                 380
Asp Tyr Phe Thr Lys Pro Asp Tyr Gln Leu Ile Met Ser Val Phe Glu
385                 390                 395                 400
Asn Ser Met Lys Glu Arg Gly Ile Ala Glu Asn Glu Ala Phe Asp Trp
                405                 410                 415
Glu Lys Ala Gly Thr Asp Ala Leu Leu Ser Thr Ser Thr Ser Thr Pro
            420                 425                 430
Pro Gln Gln Asn Thr Arg Gln Thr Ala Ala Met Phe Gly Val Val Asn
        435                 440                 445
Val Thr Pro Val Pro Gly Asp Leu Leu Arg Glu Asn Thr Glu Asp Val

450                 455                 460
Leu Gln Gly Glu His Leu Ser Asp Gln Glu Asn Ala Pro Pro Ile Leu
465                 470                 475                 480
Pro Gly Arg Pro Ser Glu Gly Leu Gly Pro Ser Pro His Leu Val Pro
                485                 490                 495
His Pro Gly Gly Pro Glu Ala Glu Val Trp Glu Glu Thr Asp Val Asn
            500                 505                 510
Arg Asn Lys Leu Arg Ile Asn Ile Gly Lys Val Thr Ala Ala Arg Ala
        515                 520                 525
Lys Gly Val Gly Gly Leu Phe Ser His Pro Arg Phe Pro Ala Leu Cys
    530                 535                 540
Pro Cys Pro Val Pro Pro Lys His Pro Val Pro Gly His Leu Pro Ala
545                 550                 555                 560
Cys Pro Ala Ser Val Ser Arg Ser Leu Pro Ala Leu Ala Ser Leu Cys
                565                 570                 575
Leu Pro Ser Ser Ser Ser Ser Val Ser Phe Thr Leu Arg Arg Pro Ser
            580                 585                 590
Ala His Ser Arg Leu Ile Ser Pro Ser Ser Trp His Ser Pro Leu Leu
        595                 600                 605
Gln Ser Pro Cys Val Glu Glu Glu Gln Ser Arg Gly Met Gly Val Pro
    610                 615                 620
Ser Ser Pro Val Arg Ala Pro Pro Asp Ser Pro Thr Thr Pro Val Arg
625                 630                 635                 640
Ser Leu Arg Tyr Arg Arg Val Asn Ser Pro Glu Ser Glu Arg Leu Ser
                645                 650                 655
Thr Ala Asp Gly Arg Val Glu Leu Pro Glu Arg Arg Ser Arg Met Asp
            660                 665                 670
Leu Pro Gly Ser Pro Ser Arg Gln Ala Cys Ser Ser Gln Pro Ala Gln
        675                 680                 685
Met Leu Ser Val Asp Thr Gly His Ala Asp Arg Gln Ala Ser Gly Arg
    690                 695                 700
Met Asp Val Ser Ala Ser Val Glu Gln Glu Ala Leu Ser Asn Ala Phe
705                 710                 715                 720
Arg Ser Val Pro Leu Ala Glu Glu Glu Asp Phe Asp Ser Lys Glu Trp
                725                 730                 735
Val Ile Ile Asp Lys Glu Thr Glu Leu Lys Asp Phe Pro Pro Gly Ala
            740                 745                 750
Glu Pro Ser Thr Ser Gly Thr Thr Asp Glu Glu Pro Glu Glu Leu Arg
        755                 760                 765
Pro Leu Pro Glu Glu Gly Glu Glu Arg Arg Arg Leu Gly Ala Glu Pro
    770                 775                 780
Thr Val Arg Pro Arg Gly Arg Ser Met Gln Ala Leu Ala Glu Glu Asp
785                 790                 795                 800
Leu Gln His Leu Pro Pro Gln Pro Leu Pro Pro Gln Leu Ser Gln Xaa
                805                 810                 815
Asp Gly Arg Ser Glu Thr Ser Gln Pro Pro Thr Pro Gly Ser Pro Ser
            820                 825                 830
His Ser Pro Leu His Ser Gly Pro Arg Pro Arg Arg Arg Glu Ser Asp
        835                 840                 845
Pro Thr Gly Pro Gln Arg Gln Ala Gly Val Gln Trp Arg Asp Leu Gly
    850                 855                 860
Ser Leu Gln Pro Pro Pro Pro Arg Phe Lys Gln Phe Ser Cys Leu Ser
865                 870                 875                 880
Leu Pro Arg Ser Trp Asp Tyr Arg His Ala Pro Pro Pro His Pro Ala
                885                 890                 895
Asn Phe Val Phe Leu Val Glu Thr Gly Phe Leu His Val Glu Ala Gly
            900                 905                 910
Leu Glu Leu Leu Thr Ser Gly Asp Leu Pro Ala Ser Ala Ser Gln Ile
        915                 920                 925
Ala Gly Ile Thr Gly Val Ser His Arg Ala Gln Pro Glu Val Cys Glu
    930                 935                 940
Phe Asn Arg Lys His Thr Gly Gln Arg Glu Gln Met Val Cys Ala Gly
945                 950                 955                 960
Ser Glu Arg Ala Trp Ser Met Arg Asp Leu Pro Gly Arg Pro Leu Glu
                965                 970                 975
Pro Tyr Ser Glu Ser Tyr
            980

<210> 27
<211> 2931
<212> DNA
<213> homo sapiens

<400> 27
   atgctggcgg cgcggtgcat tgtgggcagc tccccgctct gccgctgccg ccgccgtcgc 60
   ccaaggagga tcggggccgg gccgggccgg gatgatccgg gtcggaaggc cgccgccgcc 120
   ggagggagcg ggtcacccaa cgccgcactg agccgccccc gccccgcccc ggccccgggg 180
   gatgcgccgc cccgagctgc tgcctccgcc gccgccgcag ccgcagccgc agcgggcaca 240
   gagcaggtag atggccccct cagggcaggc ccggcggaca cccctccctc tggctggcgg 300
   atgcagtgcc tagcggccgc ccttaaggac gaaaccaaca tgagtggggg aggggagcag 360
   gccgacatcc tgccggccaa ctacgtggtc aaggatcgct ggaaggtgct gaaaaagatc 420
   gggggcgggg gctttggtga gatctacgag gccatggacc tgctgaccag ggagaatgtg 480
   gccctcaagg tggagtcagc ccagcagccc aagcaggtcc tcaagatgga ggtggccgtg 540
   ctcaagaagt tgcaagggaa ggaccatgtg tgcaggttca ttggctgtgg caggaacgag 600
   aagtttaact atgtagtgat gcagctccag ggccggaacc tggccgacct gcgccgtagc 660
   cagccgcgag gcaccttcac gctgagcacc acattgcggc tgggcaagca gatcttggag 720
   tccatcgagg ccatccactc tgtgggcttc ctgcaccgtg acatcaagcc ttcaaacttt 780
   gccatgggca ggctgccctc cacctacagg aagtgctata tgctggactt cgggctggcc 840
   cggcagtaca ccaacaccac gggggatgtg cggccccctc ggaatgtggc cgggtttcga 900
   ggaacggttc gctatgcctc agtcaatgcc cacaagaacc gggagatggg ccgccacgac 960
   gacctgtggt ccctcttcta catgctggtg gagtttgcag tgggccagct gccctggagg 1020
   aagatcaagg acaaggaaca ggtagggatg atcaaggaga agtatgagca ccggatgctg 1080
   ctgaagcaca tgccgtcaga gttccacctc ttcctggacc acattgccag cctcgactac 1140
   ttcaccaagc ccgactacca gttgatcatg tcagtgtttg agaacagcat gaaggagagg 1200
   ggcattgccg agaatgaggc ctttgactgg gagaaggcag gcaccgatgc cctcctgtcc 1260
   acgagcacct ctaccccgcc ccagcagaac acccggcaga cggcagccat gtttggggtg 1320
   gtcaatgtga cgccagtgcc tggggacctg ctccgggaga acaccgagga tgtgctacag 1380
   ggagagcacc tgagtgacca ggagaatgca cccccaattc tgcccgggag gccctctgag 1440
   gggctgggcc ccagtcccca ccttgtcccc caccccgggg gtcctgaggc tgaagtctgg 1500
   gaggagacag atgtcaaccg gaacaaactc cggatcaaca tcggcaaagt aactgccgcc 1560
   agggcgaagg gcgtgggtgg ccttttctct cacccccgat tcccagcctt gtgcccctgc 1620
   cctgttcctc ctaagcaccc tgtccccggc catctgcctg cttgccctgc ctctgtttcc 1680
   cggtccctcc ccgcactagc ctcgctgtgt cttccatcat catcatcctc tgtctccttc 1740
   accctgagga gaccatccgc ccacagccgc ctcatcagcc ccagctcatg gcactcccct 1800
   ctcctgcaga gcccctgtgt ggaggaggaa cagagccgag gcatgggggt ccccagctcc 1860
   ccagtgcgtg cccccccaga ctcccccaca accccagtcc gttctctgcg ctaccggagg 1920
   gtgaacagcc ctgagtcaga aaggctgtcc acggcggacg ggcgagtgga gctacctgag 1980
   aggaggtcac ggatggatct gcctggctcg ccctcgcgcc aggcctgctc ctctcagcca 2040
   gcccagatgc tgtcagtgga cacaggccac gctgaccgac aggccagtgg ccgcatggay 2100
   gtgtcagcct ctgtggagca ggaggccctg agcaacgcct tccgctcggt gccgctggct 2160
   gaggaggagg atttcgacag caaagagtgg gtcatcatcg acaaggagac ggagctcaag 2220
   gacttccctc caggggctga gcccagcaca tcgggcacca cggatgagga gcccgaggag 2280
   ctgcggccac tgcccgagga gggcgaagag cggcggcggc tgggggcaga gcccaccgtc 2340
   cggccccggg gacgcagcat gcaggcgctg gcggaggagg acctgcagca tttgccgccc 2400
   cagcccctgc caccccagct gagccaggsc gatggccgtt ccgagacgtc acagcccccc 2460
   acgcctggca gcccttccca ctcacccctg cactcgggac cccgccctcg acggagagag 2520
   tcggacccca caggcccaca gagacaggct ggagtgcaat ggcgtgatct cggctcactg 2580
   caacctccac ctcccaggtt caagcaattc tcctgcctca gcctcccgag aagctgggat 2640
   tacaggcatg caccaccacc acacccggct aattttgtat ttttagtaga gacggggttt 2700
   ctccatgttg aggctggtct tgagctcctg acctcaggtg atctgcctgc ctcggcctcc 2760
   caaattgctg ggattacagg cgtgagccat cgcgcccagc ctgaggtctg tgagtttaac 2820
   agaaaacata caggccagag agagcagatg gtttgtgcag gatcagagag agcctggagc 2880
   atgcgtgacc tgcccgggcg gccgctcgag ccctatagtg agtcgtatta g 2931

<210> 28
<211> 976
<212> PRT
<213> homo sapiens

<220>
<221> VARIANT
<222> (1)...(976)
<223> Xaa = Any Amino Acid

<400> 28

Met Leu Ala Ala Arg Cys Ile Val Gly Ser Ser Pro Leu Cys Arg Cys
1               5                   10                  15
Arg Arg Arg Arg Pro Arg Arg Ile Gly Ala Gly Pro Gly Arg Asp Asp
            20                  25                  30
Pro Gly Arg Lys Ala Ala Ala Ala Gly Gly Ser Gly Ser Pro Asn Ala
        35                  40                  45
Ala Leu Ser Arg Pro Arg Pro Ala Pro Ala Pro Gly Asp Ala Pro Pro
    50                  55                  60
Arg Ala Ala Ala Ser Ala Ala Ala Ala Ala Ala Ala Ala Ala Gly Thr
65                  70                  75                  80
Glu Gln Val Asp Gly Pro Leu Arg Ala Gly Pro Ala Asp Thr Pro Pro
                85                  90                  95
Ser Gly Trp Arg Met Gln Cys Leu Ala Ala Ala Leu Lys Asp Glu Thr
            100                 105                 110
Asn Met Ser Gly Gly Gly Glu Gln Ala Asp Ile Leu Pro Ala Asn Tyr
        115                 120                 125
Val Val Lys Asp Arg Trp Lys Val Leu Lys Lys Ile Gly Gly Gly Gly
    130                 135                 140
Phe Gly Glu Ile Tyr Glu Ala Met Asp Leu Leu Thr Arg Glu Asn Val
145                 150                 155                 160
Ala Leu Lys Val Glu Ser Ala Gln Gln Pro Lys Gln Val Leu Lys Met
                165                 170                 175
Glu Val Ala Val Leu Lys Lys Leu Gln Gly Lys Asp His Val Cys Arg
            180                 185                 190
Phe Ile Gly Cys Gly Arg Asn Glu Lys Phe Asn Tyr Val Val Met Gln
        195                 200                 205
Leu Gln Gly Arg Asn Leu Ala Asp Leu Arg Arg Ser Gln Pro Arg Gly
    210                 215                 220
Thr Phe Thr Leu Ser Thr Thr Leu Arg Leu Gly Lys Gln Ile Leu Glu
225                 230                 235                 240
Ser Ile Glu Ala Ile His Ser Val Gly Phe Leu His Arg Asp Ile Lys
                245                 250                 255
Pro Ser Asn Phe Ala Met Gly Arg Leu Pro Ser Thr Tyr Arg Lys Cys
            260                 265                 270
Tyr Met Leu Asp Phe Gly Leu Ala Arg Gln Tyr Thr Asn Thr Thr Gly
        275                 280                 285
Asp Val Arg Pro Pro Arg Asn Val Ala Gly Phe Arg Gly Thr Val Arg
    290                 295                 300
Tyr Ala Ser Val Asn Ala His Lys Asn Arg Glu Met Gly Arg His Asp
305                 310                 315                 320
Asp Leu Trp Ser Leu Phe Tyr Met Leu Val Glu Phe Ala Val Gly Gln
                325                 330                 335
Leu Pro Trp Arg Lys Ile Lys Asp Lys Glu Gln Val Gly Met Ile Lys
            340                 345                 350
Glu Lys Tyr Glu His Arg Met Leu Leu Lys His Met Pro Ser Glu Phe
        355                 360                 365
His Leu Phe Leu Asp His Ile Ala Ser Leu Asp Tyr Phe Thr Lys Pro
    370                 375                 380
Asp Tyr Gln Leu Ile Met Ser Val Phe Glu Asn Ser Met Lys Glu Arg
385                 390                 395                 400
Gly Ile Ala Glu Asn Glu Ala Phe Asp Trp Glu Lys Ala Gly Thr Asp
                405                 410                 415
Ala Leu Leu Ser Thr Ser Thr Ser Thr Pro Pro Gln Gln Asn Thr Arg
            420                 425                 430
Gln Thr Ala Ala Met Phe Gly Val Val Asn Val Thr Pro Val Pro Gly
        435                 440                 445
Asp Leu Leu Arg Glu Asn Thr Glu Asp Val Leu Gln Gly Glu His Leu
    450                 455                 460
Ser Asp Gln Glu Asn Ala Pro Pro Ile Leu Pro Gly Arg Pro Ser Glu
465                 470                 475                 480
Gly Leu Gly Pro Ser Pro His Leu Val Pro His Pro Gly Gly Pro Glu
                485                 490                 495
Ala Glu Val Trp Glu Glu Thr Asp Val Asn Arg Asn Lys Leu Arg Ile
            500                 505                 510
Asn Ile Gly Lys Val Thr Ala Ala Arg Ala Lys Gly Val Gly Gly Leu
        515                 520                 525
Phe Ser His Pro Arg Phe Pro Ala Leu Cys Pro Cys Pro Val Pro Pro
    530                 535                 540

Lys His Pro Val Pro Gly His Leu Pro Ala Cys Pro Ala Ser Val Ser
545                 550                 555                 560
Arg Ser Leu Pro Ala Leu Ala Ser Leu Cys Leu Pro Ser Ser Ser Ser
                565                 570                 575
Ser Val Ser Phe Thr Leu Arg Arg Pro Ser Ala His Ser Arg Leu Ile
            580                 585                 590
Ser Pro Ser Ser Trp His Ser Pro Leu Leu Gln Ser Pro Cys Val Glu
        595                 600                 605
Glu Glu Gln Ser Arg Gly Met Gly Val Pro Ser Ser Pro Val Arg Ala
    610                 615                 620
Pro Pro Asp Ser Pro Thr Thr Pro Val Arg Ser Leu Arg Tyr Arg Arg
625                 630                 635                 640
Val Asn Ser Pro Glu Ser Glu Arg Leu Ser Thr Ala Asp Gly Arg Val
                645                 650                 655
Glu Leu Pro Glu Arg Arg Ser Arg Met Asp Leu Pro Gly Ser Pro Ser
            660                 665                 670
Arg Gln Ala Cys Ser Ser Gln Pro Ala Gln Met Leu Ser Val Asp Thr
        675                 680                 685
Gly His Ala Asp Arg Gln Ala Ser Gly Arg Met Asp Val Ser Ala Ser
    690                 695                 700
Val Glu Gln Glu Ala Leu Ser Asn Ala Phe Arg Ser Val Pro Leu Ala
705                 710                 715                 720
Glu Glu Glu Asp Phe Asp Ser Lys Glu Trp Val Ile Ile Asp Lys Glu
                725                 730                 735
Thr Glu Leu Lys Asp Phe Pro Pro Gly Ala Glu Pro Ser Thr Ser Gly
            740                 745                 750
Thr Thr Asp Glu Glu Pro Glu Glu Leu Arg Pro Leu Pro Glu Glu Gly
        755                 760                 765
Glu Glu Arg Arg Arg Leu Gly Ala Glu Pro Thr Val Arg Pro Arg Gly
    770                 775                 780
Arg Ser Met Gln Ala Leu Ala Glu Glu Asp Leu Gln His Leu Pro Pro
785                 790                 795                 800
Gln Pro Leu Pro Pro Gln Leu Ser Gln Xaa Asp Gly Arg Ser Glu Thr
                805                 810                 815
Ser Gln Pro Pro Thr Pro Gly Ser Pro Ser His Ser Pro Leu His Ser
            820                 825                 830
Gly Pro Arg Pro Arg Arg Arg Glu Ser Asp Pro Thr Gly Pro Gln Arg
        835                 840                 845
Gln Ala Gly Val Gln Trp Arg Asp Leu Gly Ser Leu Gln Pro Pro Pro
    850                 855                 860
Pro Arg Phe Lys Gln Phe Ser Cys Leu Ser Leu Pro Arg Ser Trp Asp
865                 870                 875                 880
Tyr Arg His Ala Pro Pro Pro His Pro Ala Asn Phe Val Phe Leu Val
                885                 890                 895
Glu Thr Gly Phe Leu His Val Glu Ala Gly Leu Glu Leu Leu Thr Ser
            900                 905                 910
Gly Asp Leu Pro Ala Ser Ala Ser Gln Ile Ala Gly Ile Thr Gly Val
        915                 920                 925
Ser His Arg Ala Gln Pro Glu Val Cys Glu Phe Asn Arg Lys His Thr
    930                 935                 940
Gly Gln Arg Glu Gln Met Val Cys Ala Gly Ser Glu Arg Ala Trp Ser
945                 950                 955                 960
Met Arg Asp Leu Pro Gly Arg Pro Leu Glu Pro Tyr Ser Glu Ser Tyr
                965                 970                 975

<210> 29
<211> 2631
<212> DNA
<213> homo sapiens

<400> 29
atgcagtgcc tagcggccgc ccttaaggac gaaaccaaca tgagtggggg aggggagcag 60
gccgacatcc tgccggccaa ctacgtggtc aaggatcgct ggaaggtgct gaaaaagatc 120
gggggcgggg gctttggtga gatctacgag gccatggacc tgctgaccag ggagaatgtg 180
gccctcaagg tggagtcagc ccagcagccc aagcaggtcc tcaagatgga ggtggccgtg 240
ctcaagaagt tgcaagggaa ggaccatgtg tgcaggttca ttggctgtgg caggaacgag 300
aagtttaact atgtagtgat gcagctccag ggccggaacc tggccgacct gcgccgtagc 360

cagccgcgag gcaccttcac gctgagcacc acattgcggc tgggcaagca gatcttggag 420
tccatcgagg ccatccactc tgtgggcttc ctgcaccgtg acatcaagcc ttcaaacttt 480
gccatgggca ggctgccctc cacctacagg aagtgctata tgctggactt cgggctggcc 540
cggcagtaca ccaacaccac gggggatgtg cggccccctc ggaatgtggc cgggtttcga 600
ggaacggttc gctatgcctc agtcaatgcc cacaagaacc gggagatggg ccgccacgac 660
gacctgtggt ccctcttcta catgctggtg gagtttgcag tgggccagct gccctggagg 720
aagatcaagg acaaggaaca ggtagggatg atcaaggaga agtatgagca ccggatgctg 780
ctgaagcaca tgccgtcaga gttccacctc ttcctggacc acattgccag cctcgactac 840
ttcaccaagc ccgactacca gttgatcatg tcagtgtttg agaacagcat gaaggagagg 900
ggcattgccg agaatgaggc ctttgactgg gagaaggcag gcaccgatgc cctcctgtcc 960
acgagcacct ctaccccgcc ccagcagaac acccggcaga cggcagccat gtttggggtg 1020
gtcaatgtga cgccagtgcc tggggacctg ctccgggaga acaccgagga tgtgctacag 1080
ggagagcacc tgagtgacca ggagaatgca cccccaattc tgcccgggag gccctctgag 1140
gggctgggcc ccagtcccca ccttgtcccc caccccgggg gtcctgaggc tgaagtctgg 1200
gaggagacag atgtcaaccg gaacaaactc cggatcaaca tcggcaaagt aactgccgcc 1260
agggcgaagg gcgtgggtgg cctttttctct cacccccgat tcccagcctt gtgcccctgc 1320
cctgttcctc ctaagcaccc tgtccccggc catctgcctg cttgccctgc ctctgtttcc 1380
cggtccctcc ccgcactagc ctcgctgtgt cttccatcat catcatcctc tgtctccttc 1440
accctgagga gaccatccgc ccacagccgc ctcatcagcc ccagctcatg gcactcccct 1500
ctcctgcaga gcccctgtgt ggaggaggaa cagagccgag gcatgggggt ccccagctcc 1560
ccagtgcgtg cccccccaga ctcccccaca accccagtcc gttctctgcg ctaccggagg 1620
gtgaacagcc ctgagtcaga aaggctgtcc acggcggacg ggcgagtgga gctacctgag 1680
aggaggtcac ggatggatct gcctggctcg ccctcgcgcc aggcctgctc ctctcagcca 1740
gcccagatgc tgtcagtgga cacaggccac gctgaccgac aggccagtgg ccgcatggay 1800
gtgtcagcct ctgtggagca ggaggccctg agcaacgcct tccgctcggt gccgctggct 1860
gaggaggagg atttcgacag caaagagtgg gtcatcatcg acaaggagac ggagctcaag 1920
gacttccctc caggggctga gcccagcaca tcgggcacca cggatgagga gcccgaggag 1980
ctgcggccac tgcccgagga gggcgaagag cggcggcggc tgggggcaga gcccaccgtc 2040
cggccccggg gacgcagcat gcaggcgctg gcggaggagg acctgcagca tttgccgccc 2100
cagcccctgc caccccagct gagccaggsc gatggccgtt ccgagacgtc acagcccccc 2160
acgcctggca gcccttccca ctcacccctg cactcgggac cccgccctcg acggagagag 2220
tcggacccca caggcccaca gagacaggct ggagtgcaat ggcgtgatct cggctcactg 2280
caacctccac ctcccaggtt caagcaattc tcctgcctca gcctcccgag aagctgggat 2340
tacaggcatg caccaccacc acacccggct aatttgtat ttttagtaga gacggggttt 2400
ctccatgttg aggctggtct tgagctcctg acctcaggtg atctgcctgc ctcggcctcc 2460
caaattgctg ggattacagg cgtgagccat cgcgcccagc ctgaggtctg tgagtttaac 2520
agaaaacata caggccagag agagcagatg gtttgtgcag gatcagagag agcctggagc 2580
atgcgtgacc tgcccgggcg gccgctcgag ccctatagtg agtcgtatta g 2631

<210> 30
<211> 876
<212> PRT
<213> homo sapiens

<220>
<221> VARIANT
<222> (1)...(876)
<223> Xaa = Any Amino Acid

<400> 30
   Met Gln Cys Leu Ala Ala Ala Leu Lys Asp Glu Thr Asn Met Ser Gly
   1               5                   10                  15
   Gly Gly Glu Gln Ala Asp Ile Leu Pro Ala Asn Tyr Val Val Lys Asp
               20                  25                  30
   Arg Trp Lys Val Leu Lys Lys Ile Gly Gly Gly Gly Phe Gly Glu Ile
           35                  40                  45
   Tyr Glu Ala Met Asp Leu Leu Thr Arg Glu Asn Val Ala Leu Lys Val
       50                  55                  60
   Glu Ser Ala Gln Gln Pro Lys Gln Val Leu Lys Met Glu Val Ala Val
   65                  70                  75                  80
   Leu Lys Lys Leu Gln Gly Lys Asp His Val Cys Arg Phe Ile Gly Cys
                   85                  90                  95
   Gly Arg Asn Glu Lys Phe Asn Tyr Val Val Met Gln Leu Gln Gly Arg
               100                 105                 110
   Asn Leu Ala Asp Leu Arg Arg Ser Gln Pro Arg Gly Thr Phe Thr Leu
           115                 120                 125
   Ser Thr Thr Leu Arg Leu Gly Lys Gln Ile Leu Glu Ser Ile Glu Ala
       130                 135                 140
   Ile His Ser Val Gly Phe Leu His Arg Asp Ile Lys Pro Ser Asn Phe
   145                 150                 155                 160
   Ala Met Gly Arg Leu Pro Ser Thr Tyr Arg Lys Cys Tyr Met Leu Asp
                   165                 170                 175
   Phe Gly Leu Ala Arg Gln Tyr Thr Asn Thr Thr Gly Asp Val Arg Pro
               180                 185                 190
   Pro Arg Asn Val Ala Gly Phe Arg Gly Thr Val Arg Tyr Ala Ser Val
           195                 200                 205
   Asn Ala His Lys Asn Arg Glu Met Gly Arg His Asp Asp Leu Trp Ser
       210                 215                 220
   Leu Phe Tyr Met Leu Val Glu Phe Ala Val Gly Gln Leu Pro Trp Arg
   225                 230                 235                 240
   Lys Ile Lys Asp Lys Glu Gln Val Gly Met Ile Lys Glu Lys Tyr Glu
                   245                 250                 255
   His Arg Met Leu Leu Lys His Met Pro Ser Glu Phe His Leu Phe Leu
               260                 265                 270
   Asp His Ile Ala Ser Leu Asp Tyr Phe Thr Lys Pro Asp Tyr Gln Leu
           275                 280                 285
   Ile Met Ser Val Phe Glu Asn Ser Met Lys Glu Arg Gly Ile Ala Glu
       290                 295                 300
   Asn Glu Ala Phe Asp Trp Glu Lys Ala Gly Thr Asp Ala Leu Leu Ser
   305                 310                 315                 320
   Thr Ser Thr Ser Thr Pro Pro Gln Gln Asn Thr Arg Gln Thr Ala Ala
                   325                 330                 335
   Met Phe Gly Val Val Asn Val Thr Pro Val Pro Gly Asp Leu Leu Arg
               340                 345                 350
   Glu Asn Thr Glu Asp Val Leu Gln Gly Glu His Leu Ser Asp Gln Glu
           355                 360                 365
   Asn Ala Pro Pro Ile Leu Pro Gly Arg Pro Ser Glu Gly Leu Gly Pro
       370                 375                 380
   Ser Pro His Leu Val Pro His Pro Gly Gly Pro Glu Ala Glu Val Trp
   385                 390                 395                 400
   Glu Glu Thr Asp Val Asn Arg Asn Lys Leu Arg Ile Asn Ile Gly Lys
                   405                 410                 415
   Val Thr Ala Ala Arg Ala Lys Gly Val Gly Gly Leu Phe Ser His Pro
               420                 425                 430
   Arg Phe Pro Ala Leu Cys Pro Cys Pro Val Pro Pro Lys His Pro Val
           435                 440                 445
   Pro Gly His Leu Pro Ala Cys Pro Ala Ser Val Ser Arg Ser Leu Pro
       450                 455                 460
   Ala Leu Ala Ser Leu Cys Leu Pro Ser Ser Ser Ser Ser Val Ser Phe
   465                 470                 475                 480
   Thr Leu Arg Arg Pro Ser Ala His Ser Arg Leu Ile Ser Pro Ser Ser
                   485                 490                 495
   Trp His Ser Pro Leu Leu Gln Ser Pro Cys Val Glu Glu Glu Gln Ser
               500                 505                 510
   Arg Gly Met Gly Val Pro Ser Ser Pro Val Arg Ala Pro Pro Asp Ser
           515                 520                 525
   Pro Thr Thr Pro Val Arg Ser Leu Arg Tyr Arg Arg Val Asn Ser Pro
       530                 535                 540
   Glu Ser Glu Arg Leu Ser Thr Ala Asp Gly Arg Val Glu Leu Pro Glu
   545                 550                 555                 560
   Arg Arg Ser Arg Met Asp Leu Pro Gly Ser Pro Ser Arg Gln Ala Cys
                   565                 570                 575
   Ser Ser Gln Pro Ala Gln Met Leu Ser Val Asp Thr Gly His Ala Asp
               580                 585                 590
   Arg Gln Ala Ser Gly Arg Met Asp Val Ser Ala Ser Val Glu Gln Glu
           595                 600                 605
   Ala Leu Ser Asn Ala Phe Arg Ser Val Pro Leu Ala Glu Glu Glu Asp
       610                 615                 620
   Phe Asp Ser Lys Glu Trp Val Ile Ile Asp Lys Glu Thr Glu Leu Lys
   625                 630                 635                 640
   Asp Phe Pro Pro Gly Ala Glu Pro Ser Thr Ser Gly Thr Thr Asp Glu
                   645                 650                 655
   Glu Pro Glu Glu Leu Arg Pro Leu Pro Glu Glu Gly Glu Glu Arg Arg
               660                 665                 670
   Arg Leu Gly Ala Glu Pro Thr Val Arg Pro Arg Gly Arg Ser Met Gln
           675                 680                 685
   Ala Leu Ala Glu Glu Asp Leu Gln His Leu Pro Pro Gln Pro Leu Pro
       690                 695                 700
   Pro Gln Leu Ser Gln Xaa Asp Gly Arg Ser Glu Thr Ser Gln Pro Pro
   705                 710                 715                 720
   Thr Pro Gly Ser Pro Ser His Ser Pro Leu His Ser Gly Pro Arg Pro
                   725                 730                 735
   Arg Arg Arg Glu Ser Asp Pro Thr Gly Pro Gln Arg Gln Ala Gly Val
               740                 745                 750
   Gln Trp Arg Asp Leu Gly Ser Leu Gln Pro Pro Pro Pro Arg Phe Lys
           755                 760                 765
   Gln Phe Ser Cys Leu Ser Leu Pro Arg Ser Trp Asp Tyr Arg His Ala
       770                 775                 780
   Pro Pro Pro His Pro Ala Asn Phe Val Phe Leu Val Glu Thr Gly Phe
   785                 790                 795                 800
   Leu His Val Glu Ala Gly Leu Glu Leu Leu Thr Ser Gly Asp Leu Pro
                   805                 810                 815
   Ala Ser Ala Ser Gln Ile Ala Gly Ile Thr Gly Val Ser His Arg Ala
               820                 825                 830
   Gln Pro Glu Val Cys Glu Phe Asn Arg Lys His Thr Gly Gln Arg Glu
           835                 840                 845
   Gln Met Val Cys Ala Gly Ser Glu Arg Ala Trp Ser Met Arg Asp Leu
       850                 855                 860
   Pro Gly Arg Pro Leu Glu Pro Tyr Ser Glu Ser Tyr
   865                 870                 875

<210> 31
<211> 2592
<212> DNA
<213> homo sapiens

<400> 31
atgagtgggg gaggggagca ggccgacatc ctgccggcca actacgtggt caaggatcgc 60
tggaaggtgc tgaaaaagat cgggggcggg ggctttggtg agatctacga ggccatggac 120
ctgctgacca gggagaatgt ggccctcaag gtggagtcag cccagcagcc caagcaggtc 180
ctcaagatgg aggtggccgt gctcaagaag ttgcaaggga aggaccatgt gtgcaggttc 240
attggctgtg gcaggaacga gaagtttaac tatgtagtga tgcagctcca gggccggaac 300
ctggccgacc tgcgccgtag ccagccgcga ggcaccttca cgctgagcac cacattgcgg 360
ctgggcaagc agatcttgga gtccatcgag gccatccact ctgtgggctt cctgcaccgt 420
gacatcaagc cttcaaactt tgccatgggc aggctgccct ccacctacag gaagtgctat 480
atgctggact tcgggctggc ccggcagtac accaacacca cgggggatgt gcggccccct 540
cggaatgtgg ccgggtttcg aggaacggtt cgctatgcct cagtcaatgc ccacaagaac 600
cgggagatgg gccgccacga cgacctgtgg tccctcttct acatgctggt ggagtttgca 660
gtgggccagc tgccctggag gaagatcaag gacaaggaac aggtagggat gatcaaggag 720
aagtatgagc accggatgct gctgaagcac atgccgtcag agttccacct cttcctggac 780
cacattgcca gcctcgacta cttcaccaag cccgactacc agttgatcat gtcagtgttt 840
gagaacagca tgaaggagag gggcattgcc gagaatgagg cctttgactg ggagaaggca 900
ggcaccgatg ccctcctgtc cacgagcacc tctaccccgc cccagcagaa cacccggcag 960
acggcagcca tgtttggggt ggtcaatgtg acgccagtgc ctggggacct gctccgggag 1020
aacaccgagg atgtgctaca gggagagcac ctgagtgacc aggagaatgc acccccaatt 1080
ctgcccggga ggccctctga ggggctgggc cccagtcccc accttgtccc ccaccccggg 1140
ggtcctgagg ctgaagtctg ggaggagaca gatgtcaacc ggaacaaact ccggatcaac 1200
atcggcaaag taactgccgc cagggcgaag ggcgtgggtg gcctttctc tcacccccga 1260
ttcccagcct tgtgcccctg ccctgttcct cctaagcacc ctgtccccgg ccatctgcct 1320
gcttgccctg cctctgtttc ccggtccctc cccgcactag cctcgctgtg tcttccatca 1380
tcatcatcct ctgtctcctt caccctgagg agaccatccg cccacagccg cctcatcagc 1440
cccagctcat ggcactcccc tctcctgcag agcccctgtg tggaggagga acagagccga 1500
ggcatggggg tccccagctc cccagtgcgt gcccccccag actcccccac aaccccagtc 1560
cgttctctgc gctaccggag ggtgaacagc cctgagtcag aaaggctgtc cacggcggac 1620
gggcgagtgg agctacctga gaggaggtca cggatggatc tgcctggctc gccctcgcgc 1680
caggcctgct cctctcagcc agcccagatg ctgtcagtgg acacaggcca cgctgaccga 1740
caggccagtg gccgcatgga ygtgtcagcc tctgtggagc aggaggccct gagcaacgcc 1800
ttccgctcgg tgccgctggc tgaggaggag gatttcgaca gcaaagagtg ggtcatcatc 1860
gacaaggaga cggagctcaa ggacttccct ccaggggctg agcccagcac atcgggcacc 1920
acggatgagg agcccgagga gctgcggcca ctgcccgagg agggcgaaga gcggcggcgg 1980
ctgggggcag agcccaccgt ccggccccgg ggacgcagca tgcaggcgct ggcggaggag 2040
gacctgcagc atttgccgcc ccagcccctg ccaccccagc tgagccaggs cgatggccgt 2100
tccgagacgt cacagccccc cacgcctggc agcccttccc actcacccct gcactcggga 2160

ccccgccctc gacggagaga gtcggacccc acaggcccac agagacaggc tggagtgcaa 2220
tggcgtgatc tcggctcact gcaacctcca cctcccaggt tcaagcaatt ctcctgcctc 2280
agcctcccga gaagctggga ttacaggcat gcaccaccac cacacccggc taattttgta 2340
tttttagtag agacggggtt tctccatgtt gaggctggtc ttgagctcct gacctcaggt 2400
gatctgcctg cctcggcctc ccaaattgct gggattacag gcgtgagcca tcgcgcccag 2460
cctgaggtct gtgagtttaa cagaaaacat acaggccaga gagagcagat ggtttgtgca 2520
ggatcagaga gagcctggag catgcgtgac ctgcccgggc ggccgctcga gccctatagt 2580
gagtcgtatt ag 2592

<210> 32
<211> 863
<212> PRT
<213> homo sapiens

<220>
<221> VARIANT
<222> (1)...(863)
<223> Xaa = Any Amino Acid

<400> 32
Met Ser Gly Gly Gly Glu Gln Ala Asp Ile Leu Pro Ala Asn Tyr Val
1               5                   10                  15
Val Lys Asp Arg Trp Lys Val Leu Lys Lys Ile Gly Gly Gly Gly Phe
            20                  25                  30
Gly Glu Ile Tyr Glu Ala Met Asp Leu Leu Thr Arg Glu Asn Val Ala
        35                  40                  45
Leu Lys Val Glu Ser Ala Gln Gln Pro Lys Gln Val Leu Lys Met Glu
    50                  55                  60
Val Ala Val Leu Lys Lys Leu Gln Gly Lys Asp His Val Cys Arg Phe
65                  70                  75                  80
Ile Gly Cys Gly Arg Asn Glu Lys Phe Asn Tyr Val Val Met Gln Leu
                85                  90                  95
Gln Gly Arg Asn Leu Ala Asp Leu Arg Arg Ser Gln Pro Arg Gly Thr
            100                 105                 110
Phe Thr Leu Ser Thr Thr Leu Arg Leu Gly Lys Gln Ile Leu Glu Ser
        115                 120                 125
Ile Glu Ala Ile His Ser Val Gly Phe Leu His Arg Asp Ile Lys Pro
    130                 135                 140
Ser Asn Phe Ala Met Gly Arg Leu Pro Ser Thr Tyr Arg Lys Cys Tyr
145                 150                 155                 160
Met Leu Asp Phe Gly Leu Ala Arg Gln Tyr Thr Asn Thr Thr Gly Asp
                165                 170                 175
Val Arg Pro Pro Arg Asn Val Ala Gly Phe Arg Gly Thr Val Arg Tyr
            180                 185                 190
Ala Ser Val Asn Ala His Lys Asn Arg Glu Met Gly Arg His Asp Asp
        195                 200                 205
Leu Trp Ser Leu Phe Tyr Met Leu Val Glu Phe Ala Val Gly Gln Leu
    210                 215                 220
Pro Trp Arg Lys Ile Lys Asp Lys Glu Gln Val Gly Met Ile Lys Glu
225                 230                 235                 240
Lys Tyr Glu His Arg Met Leu Leu Lys His Met Pro Ser Glu Phe His
                245                 250                 255
Leu Phe Leu Asp His Ile Ala Ser Leu Asp Tyr Phe Thr Lys Pro Asp
            260                 265                 270
Tyr Gln Leu Ile Met Ser Val Phe Glu Asn Ser Met Lys Glu Arg Gly
        275                 280                 285
Ile Ala Glu Asn Glu Ala Phe Asp Trp Glu Lys Ala Gly Thr Asp Ala
    290                 295                 300
Leu Leu Ser Thr Ser Thr Ser Thr Pro Pro Gln Gln Asn Thr Arg Gln
305                 310                 315                 320
Thr Ala Ala Met Phe Gly Val Val Asn Val Thr Pro Val Pro Gly Asp
                325                 330                 335
Leu Leu Arg Glu Asn Thr Glu Asp Val Leu Gln Gly Glu His Leu Ser
            340                 345                 350
Asp Gln Glu Asn Ala Pro Pro Ile Leu Pro Gly Arg Pro Ser Glu Gly
        355                 360                 365
Leu Gly Pro Ser Pro His Leu Val Pro His Pro Gly Gly Pro Glu Ala
    370                 375                 380

Glu Val Trp Glu Glu Thr Asp Val Asn Arg Asn Lys Leu Arg Ile Asn
385                 390                 395                 400
Ile Gly Lys Val Thr Ala Ala Arg Ala Lys Gly Val Gly Gly Leu Phe
                405                 410                 415
Ser His Pro Arg Phe Pro Ala Leu Cys Pro Cys Pro Val Pro Pro Lys
            420                 425                 430
His Pro Val Pro Gly His Leu Pro Ala Cys Pro Ala Ser Val Ser Arg
        435                 440                 445
Ser Leu Pro Ala Leu Ala Ser Leu Cys Leu Pro Ser Ser Ser Ser Ser
    450                 455                 460
Val Ser Phe Thr Leu Arg Arg Pro Ser Ala His Ser Arg Leu Ile Ser
465                 470                 475                 480
Pro Ser Ser Trp His Ser Pro Leu Leu Gln Ser Pro Cys Val Glu Glu
                485                 490                 495
Glu Gln Ser Arg Gly Met Gly Val Pro Ser Ser Pro Val Arg Ala Pro
            500                 505                 510
Pro Asp Ser Pro Thr Thr Pro Val Arg Ser Leu Arg Tyr Arg Arg Val
        515                 520                 525
Asn Ser Pro Glu Ser Glu Arg Leu Ser Thr Ala Asp Gly Arg Val Glu
    530                 535                 540
Leu Pro Glu Arg Arg Ser Arg Met Asp Leu Pro Gly Ser Pro Ser Arg
545                 550                 555                 560
Gln Ala Cys Ser Ser Gln Pro Ala Gln Met Leu Ser Val Asp Thr Gly
                565                 570                 575
His Ala Asp Arg Gln Ala Ser Gly Arg Met Asp Val Ser Ala Ser Val
            580                 585                 590
Glu Gln Glu Ala Leu Ser Asn Ala Phe Arg Ser Val Pro Leu Ala Glu
        595                 600                 605
Glu Glu Asp Phe Asp Ser Lys Glu Trp Val Ile Ile Asp Lys Glu Thr
    610                 615                 620
Glu Leu Lys Asp Phe Pro Pro Gly Ala Glu Pro Ser Thr Ser Gly Thr
625                 630                 635                 640
Thr Asp Glu Glu Pro Glu Glu Leu Arg Pro Leu Pro Glu Glu Gly Glu
                645                 650                 655
Glu Arg Arg Arg Leu Gly Ala Glu Pro Thr Val Arg Pro Arg Gly Arg
            660                 665                 670
Ser Met Gln Ala Leu Ala Glu Glu Asp Leu Gln His Leu Pro Pro Gln
        675                 680                 685
Pro Leu Pro Pro Gln Leu Ser Gln Xaa Asp Gly Arg Ser Glu Thr Ser
    690                 695                 700
Gln Pro Pro Thr Pro Gly Ser Pro Ser His Ser Pro Leu His Ser Gly
705                 710                 715                 720
Pro Arg Pro Arg Arg Arg Glu Ser Asp Pro Thr Gly Pro Gln Arg Gln
                725                 730                 735
Ala Gly Val Gln Trp Arg Asp Leu Gly Ser Leu Gln Pro Pro Pro Pro
            740                 745                 750
Arg Phe Lys Gln Phe Ser Cys Leu Ser Leu Pro Arg Ser Trp Asp Tyr
        755                 760                 765
Arg His Ala Pro Pro Pro His Pro Ala Asn Phe Val Phe Leu Val Glu
    770                 775                 780
Thr Gly Phe Leu His Val Glu Ala Gly Leu Glu Leu Leu Thr Ser Gly
785                 790                 795                 800
Asp Leu Pro Ala Ser Ala Ser Gln Ile Ala Gly Ile Thr Gly Val Ser
                805                 810                 815
His Arg Ala Gln Pro Glu Val Cys Glu Phe Asn Arg Lys His Thr Gly
            820                 825                 830
Gln Arg Glu Gln Met Val Cys Ala Gly Ser Glu Arg Ala Trp Ser Met
        835                 840                 845
Arg Asp Leu Pro Gly Arg Pro Leu Glu Pro Tyr Ser Glu Ser Tyr
    850                 855                 860

<210> 33
<211> 2874
<212> DNA
<213> homo sapiens

<400> 33

atgtcagggc tggtgctgat gctggcggcg cggtgcattg tgggcagctc cccgctctgc 60
cgctgccgcc gccgtcgccc aaggaggatc ggggccgggc cgggccggga tgatccgggt 120
cggaaggccg ccgccgccgg agggagcggg tcacccaacg ccgcactgag ccgcccccgc 180
cccgccccgg ccccggggga tgcgccgccc cgagctgctg cctccgccgc cgccgcagcc 240
gcagccgcag cgggcacaga gcaggtagat ggccccctca gggcaggccc ggcggacacc 300
cctccctctg gctggcggat gcagtgccta gcggccgccc ttaaggacga aaccaacatg 360
agtgggggag gggagcaggc cgacatcctg ccggccaact acgtggtcaa ggatcgctgg 420
aaggtgctga aaaagatcgg gggcgggggc tttggtgaga tctacgaggc catggacctg 480
ctgaccaggg agaatgtggc cctcaaggtg gagtcagccc agcagcccaa gcaggtcctc 540
aagatggagg tggccgtgct caagaagttg caagggaagg accatgtgtg caggttcatt 600
ggctgtggca ggaacgagaa gtttaactat gtagtgatgc agctccaggg ccggaacctg 660
gccgacctgc gccgtagcca gccgcgaggc accttcacgc tgagcaccac attgcggctg 720
ggcaagcaga tcttggagtc catcgaggcc atccactctg tgggcttcct gcaccgtgac 780
atcaagcctt caaactttgc catgggcagg ctgccctcca cctacaggaa gtgctatatg 840
ctggacttcg ggctggcccg gcagtacacc aacaccacgg gggatgtgcg gccccctcgg 900
aatgtggccg ggtttcgagg aacggttcgc tatgcctcag tcaatgccca caagaaccgg 960
gagatgggcc gccacgacga cctgtggtcc ctcttctaca tgctggtgga gtttgcagtg 1020
ggccagctgc cctggaggaa gatcaaggac aaggaacagg tagggatgat caaggagaag 1080
tatgagcacc ggatgctgct gaagcacatg ccgtcagagt tccacctctt cctggaccac 1140
attgccagcc tcgactactt caccaagccc gactaccagt tgatcatgtc agtgtttgag 1200
aacagcatga aggagagggg cattgccgag aatgaggcct ttgactggga gaaggcaggc 1260
accgatgccc tcctgtccac gagcacctct accccgcccc agcagaacac ccggcagacg 1320
gcagccatgt ttggggtggt caatgtgacg ccagtgcctg gggacctgct ccgggagaac 1380
accgaggatg tgctacaggg agagcacctg agtgaccagg agaatgcacc cccaattctg 1440
cccgggaggc cctctgaggg gctgggcccc agtccccacc ttgtccccca ccccgggggt 1500
cctgaggctg aagtctggga ggagacagat gtcaaccgga acaaactccg gatcaacatc 1560
ggcaaagtaa ctgccgccag ggcgaagggc gtgggtggcc ttttctctca cccccgattc 1620
ccagccttgt gcccctgccc tgttcctcct aagcaccctg tccccggcca tctgcctgct 1680
tgccctgcct ctgtttcccg gtccctcccc gcactagcct cgctgtgtct tccatcatca 1740
tcatcctctg tctccttcac cctgaggaga ccatccgccc acagccgcct catcagcccc 1800
agctcatggc actcccctct cctgcagagc ccctgtgtgg aggaggaaca gagccgaggc 1860
atgggggtcc ccagctcccc agtgcgtgcc cccccagact cccccacaac cccagtccgt 1920
tctctgcgct accggagggt gaacagccct gagtcagaaa ggctgtccac ggcggacggg 1980
cgagtggagc tacctgagag gaggtcacgg atggatctgc ctggctcgcc ctcgcgccag 2040
gcctgctcct ctcagccagc ccagatgctg tcagtggaca caggccacgc tgaccgacag 2100
gccagtggcc gcatggaygt gtcagcctct gtggagcagg aggccctgag caacgccttc 2160
cgctcggtgc cgctggctga ggaggaggat ttcgacagca aagagtgggt catcatcgac 2220
aaggagacgg agctcaagga cttccctcca ggggctgagc ccagcacatc gggcaccacg 2280
gatgaggagc ccgaggagct gcggccactg cccgaggagg gcgaagagcg gcggcggctg 2340
ggggcagagc ccaccgtccg gccccgggga cgcagcatgc aggcgctggc ggaggaggac 2400
ctgcagcatt tgccgcccca gcccctgcca ccccagctga gccaggscga tggccgttcc 2460
gagacgtcac agccccccac gcctggcagc ccttcccact cacccctgca ctcgggaccc 2520
cgccctcgac ggagagagtc ggaccccaca ggcccacaga gacagttgga ggaggacaga 2580
ctctcggggc actccctccc gcggtacagc cccctgcgac gactggcgtc ctccgtgttc 2640
tcctcctcca cgctggagac ggagcattac cctcaccccg gcggcggcgg ctcctcgggc 2700
tcctccggtt ccctcattca gcgcagccgc tcggctgaga gcagccctgt gcgggcgccc 2760
caccggcgcc acgcgcccct cgctgctggc aaccacagac tcatgccctc ggtgctccgc 2820
atctcgcggt cccagctgca gcaggtgtgg gcccggttca cccacaagac ctag 2874

<210> 34
<211> 957
<212> PRT
<213> homo sapiens

<220>
<221> VARIANT
<222> (1)...(957)
<223> Xaa = Any Amino Acid

<400> 34
Met Ser Gly Leu Val Leu Met Leu Ala Ala Arg Cys Ile Val Gly Ser
1               5                   10                  15
Ser Pro Leu Cys Arg Cys Arg Arg Arg Arg Pro Arg Arg Ile Gly Ala
            20                  25                  30
Gly Pro Gly Arg Asp Asp Pro Gly Arg Lys Ala Ala Ala Ala Gly Gly
        35                  40                  45
Ser Gly Ser Pro Asn Ala Ala Leu Ser Arg Pro Arg Pro Ala Pro Ala
    50                  55                  60

Pro Gly Asp Ala Pro Pro Arg Ala Ala Ala Ser Ala Ala Ala Ala Ala
65                  70                  75                  80
Ala Ala Ala Ala Gly Thr Glu Gln Val Asp Gly Pro Leu Arg Ala Gly
                85                  90                  95
Pro Ala Asp Thr Pro Pro Ser Gly Trp Arg Met Gln Cys Leu Ala Ala
            100                 105                 110
Ala Leu Lys Asp Glu Thr Asn Met Ser Gly Gly Gly Glu Gln Ala Asp
        115                 120                 125
Ile Leu Pro Ala Asn Tyr Val Val Lys Asp Arg Trp Lys Val Leu Lys
    130                 135                 140
Lys Ile Gly Gly Gly Gly Phe Gly Glu Ile Tyr Glu Ala Met Asp Leu
145                 150                 155                 160
Leu Thr Arg Glu Asn Val Ala Leu Lys Val Glu Ser Ala Gln Gln Pro
                165                 170                 175
Lys Gln Val Leu Lys Met Glu Val Ala Val Leu Lys Lys Leu Gln Gly
            180                 185                 190
Lys Asp His Val Cys Arg Phe Ile Gly Cys Gly Arg Asn Glu Lys Phe
        195                 200                 205
Asn Tyr Val Val Met Gln Leu Gln Gly Arg Asn Leu Ala Asp Leu Arg
    210                 215                 220
Arg Ser Gln Pro Arg Gly Thr Phe Thr Leu Ser Thr Thr Leu Arg Leu
225                 230                 235                 240
Gly Lys Gln Ile Leu Glu Ser Ile Glu Ala Ile His Ser Val Gly Phe
                245                 250                 255
Leu His Arg Asp Ile Lys Pro Ser Asn Phe Ala Met Gly Arg Leu Pro
            260                 265                 270
Ser Thr Tyr Arg Lys Cys Tyr Met Leu Asp Phe Gly Leu Ala Arg Gln
        275                 280                 285
Tyr Thr Asn Thr Thr Gly Asp Val Arg Pro Pro Arg Asn Val Ala Gly
    290                 295                 300
Phe Arg Gly Thr Val Arg Tyr Ala Ser Val Asn Ala His Lys Asn Arg
305                 310                 315                 320
Glu Met Gly Arg His Asp Asp Leu Trp Ser Leu Phe Tyr Met Leu Val
                325                 330                 335
Glu Phe Ala Val Gly Gln Leu Pro Trp Arg Lys Ile Lys Asp Lys Glu
            340                 345                 350
Gln Val Gly Met Ile Lys Glu Lys Tyr Glu His Arg Met Leu Leu Lys
        355                 360                 365
His Met Pro Ser Glu Phe His Leu Phe Leu Asp His Ile Ala Ser Leu
    370                 375                 380
Asp Tyr Phe Thr Lys Pro Asp Tyr Gln Leu Ile Met Ser Val Phe Glu
385                 390                 395                 400
Asn Ser Met Lys Glu Arg Gly Ile Ala Glu Asn Glu Ala Phe Asp Trp
                405                 410                 415
Glu Lys Ala Gly Thr Asp Ala Leu Leu Ser Thr Ser Thr Ser Thr Pro
            420                 425                 430
Pro Gln Gln Asn Thr Arg Gln Thr Ala Ala Met Phe Gly Val Val Asn
        435                 440                 445
Val Thr Pro Val Pro Gly Asp Leu Leu Arg Glu Asn Thr Glu Asp Val
    450                 455                 460
Leu Gln Gly Glu His Leu Ser Asp Gln Glu Asn Ala Pro Pro Ile Leu
465                 470                 475                 480
Pro Gly Arg Pro Ser Glu Gly Leu Gly Pro Ser Pro His Leu Val Pro
                485                 490                 495
His Pro Gly Gly Pro Glu Ala Glu Val Trp Glu Glu Thr Asp Val Asn
            500                 505                 510
Arg Asn Lys Leu Arg Ile Asn Ile Gly Lys Val Thr Ala Ala Arg Ala
        515                 520                 525
Lys Gly Val Gly Gly Leu Phe Ser His Pro Arg Phe Pro Ala Leu Cys
    530                 535                 540
Pro Cys Pro Val Pro Pro Lys His Pro Val Pro Gly His Leu Pro Ala
545                 550                 555                 560
Cys Pro Ala Ser Val Ser Arg Ser Leu Pro Ala Leu Ala Ser Leu Cys
                565                 570                 575
Leu Pro Ser Ser Ser Ser Ser Val Ser Phe Thr Leu Arg Arg Pro Ser
            580                 585                 590
Ala His Ser Arg Leu Ile Ser Pro Ser Ser Trp His Ser Pro Leu Leu
        595                 600                 605

Gln Ser Pro Cys Val Glu Glu Glu Gln Ser Arg Gly Met Gly Val Pro
    610                 615                 620
Ser Ser Pro Val Arg Ala Pro Pro Asp Ser Pro Thr Thr Pro Val Arg
625                 630                 635                 640
Ser Leu Arg Tyr Arg Arg Val Asn Ser Pro Glu Ser Glu Arg Leu Ser
                645                 650                 655
Thr Ala Asp Gly Arg Val Glu Leu Pro Glu Arg Arg Ser Arg Met Asp
            660                 665                 670
Leu Pro Gly Ser Pro Ser Arg Gln Ala Cys Ser Ser Gln Pro Ala Gln
        675                 680                 685
Met Leu Ser Val Asp Thr Gly His Ala Asp Arg Gln Ala Ser Gly Arg
    690                 695                 700
Met Asp Val Ser Ala Ser Val Glu Gln Glu Ala Leu Ser Asn Ala Phe
705                 710                 715                 720
Arg Ser Val Pro Leu Ala Glu Glu Glu Asp Phe Asp Ser Lys Glu Trp
                725                 730                 735
Val Ile Ile Asp Lys Glu Thr Glu Leu Lys Asp Phe Pro Pro Gly Ala
            740                 745                 750
Glu Pro Ser Thr Ser Gly Thr Thr Asp Glu Glu Pro Glu Glu Leu Arg
        755                 760                 765
Pro Leu Pro Glu Glu Gly Glu Glu Arg Arg Arg Leu Gly Ala Glu Pro
    770                 775                 780
Thr Val Arg Pro Arg Gly Arg Ser Met Gln Ala Leu Ala Glu Glu Asp
785                 790                 795                 800
Leu Gln His Leu Pro Pro Gln Pro Leu Pro Pro Gln Leu Ser Gln Xaa
                805                 810                 815
Asp Gly Arg Ser Glu Thr Ser Gln Pro Pro Thr Pro Gly Ser Pro Ser
            820                 825                 830
His Ser Pro Leu His Ser Gly Pro Arg Pro Arg Arg Arg Glu Ser Asp
        835                 840                 845
Pro Thr Gly Pro Gln Arg Gln Leu Glu Glu Asp Arg Leu Ser Gly His
    850                 855                 860
Ser Leu Pro Arg Tyr Ser Pro Leu Arg Arg Leu Ala Ser Ser Val Phe
865                 870                 875                 880
Ser Ser Ser Thr Leu Glu Thr Glu His Tyr Pro His Pro Gly Gly Gly
                885                 890                 895
Gly Ser Ser Gly Ser Ser Gly Ser Leu Ile Gln Arg Ser Arg Ser Ala
            900                 905                 910
Glu Ser Ser Pro Val Arg Ala Pro His Arg Arg His Ala Pro Leu Ala
        915                 920                 925
Ala Gly Asn His Arg Leu Met Pro Ser Val Leu Arg Ile Ser Arg Ser
    930                 935                 940
Gln Leu Gln Gln Val Trp Ala Arg Phe Thr His Lys Thr
945                 950                 955

<210> 35
<211> 2856
<212> DNA
<213> homo sapiens

<400> 35
atgctggcgg cgcggtgcat tgtgggcagc tccccgctct gccgctgccg ccgccgtcgc 60
ccaaggagga tcggggccgg gccgggccgg gatgatccgg gtcggaaggc cgccgccgcc 120
ggagggagcg ggtcacccaa cgccgcactg agccgccccc gccccgcccc ggccccgggg 180
gatgcgccgc cccgagctgc tgcctccgcc gccgccgcag ccgcagccgc agcgggcaca 240
gagcaggtag atggccccct cagggcaggc ccggcggaca cccctccctc tggctggcgg 300
atgcagtgcc tagcggccgc ccttaaggac gaaaccaaca tgagtggggg aggggagcag 360
gccgacatcc tgccggccaa ctacgtggtc aaggatcgct ggaaggtgct gaaaaagatc 420
gggggcgggg gctttggtga gatctacgag gccatggacc tgctgaccag ggagaatgtg 480
gccctcaagg tggagtcagc ccagcagccc aagcaggtcc tcaagatgga ggtggccgtg 540
ctcaagaagt tgcaagggaa ggaccatgtg tgcaggttca ttggctgtgg caggaacgag 600
aagtttaact atgtagtgat gcagctccag ggccggaacc tggccgacct gcgccgtagc 660
cagccgcgag gcaccttcac gctgagcacc acattgcggc tgggcaagca gatcttggag 720
tccatcgagg ccatccactc tgtgggcttc ctgcaccgtg acatcaagcc ttcaaacttt 780
gccatgggca ggctgccctc cacctacagg aagtgctata tgctggactt cgggctggcc 840
cggcagtaca ccaacaccac gggggatgtg cggccccctc ggaatgtggc cgggtttcga 900
ggaacggttc gctatgcctc agtcaatgcc cacaagaacc gggagatggg ccgccacgac 960

gacctgtggt ccctcttcta catgctggtg gagtttgcag tgggccagct gccctggagg 1020
aagatcaagg acaaggaaca ggtagggatg atcaaggaga agtatgagca ccggatgctg 1080
ctgaagcaca tgccgtcaga gttccacctc ttcctggacc acattgccag cctcgactac 1140
ttcaccaagc ccgactacca gttgatcatg tcagtgtttg agaacagcat gaaggagagg 1200
ggcattgccg agaatgaggc ctttgactgg gagaaggcag gcaccgatgc cctcctgtcc 1260
acgagcacct ctaccccgcc ccagcagaac acccggcaga cggcagccat gtttggggtg 1320
gtcaatgtga cgccagtgcc tggggacctg ctccgggaga acaccgagga tgtgctacag 1380
ggagagcacc tgagtgacca ggagaatgca cccccaattc tgcccgggag gccctctgag 1440
gggctgggcc ccagtcccca ccttgtcccc caccccgggg gtcctgaggc tgaagtctgg 1500
gaggagacag atgtcaaccg gaacaaactc cggatcaaca tcggcaaagt aactgccgcc 1560
agggcgaagg gcgtgggtgg ccttttctct cacccccgat tcccagcctt gtgcccctgc 1620
cctgttcctc ctaagcaccc tgtccccggc catctgcctg cttgccctgc ctctgtttcc 1680
cggtccctcc ccgcactagc ctcgctgtgt cttccatcat catcatcctc tgtctccttc 1740
accctgagga gaccatccgc ccacagccgc ctcatcagcc ccagctcatg gcactcccct 1800
ctcctgcaga gcccctgtgt ggaggaggaa cagagccgag gcatgggggt ccccagctcc 1860
ccagtgcgtg cccccccaga ctcccccaca accccagtcc gttctctgcg ctaccggagg 1920
gtgaacagcc ctgagtcaga aaggctgtcc acggcggacg ggcgagtgga gctacctgag 1980
aggaggtcac ggatggatct gcctggctcg ccctcgcgcc aggcctgctc ctctcagcca 2040
gcccagatgc tgtcagtgga cacaggccac gctgaccgac aggccagtgg ccgcatggay 2100
gtgtcagcct ctgtggagca ggaggccctg agcaacgcct tccgctcggt gccgctggct 2160
gaggaggagg atttcgacag caaagagtgg gtcatcatcg acaaggagac ggagctcaag 2220
gacttccctc caggggctga gcccagcaca tcgggcacca cggatgagga gcccgaggag 2280
ctgcggccac tgcccgagga gggcgaagag cggcggcggc tgggggcaga gcccaccgtc 2340
cggccccggg gacgcagcat gcaggcgctg gcggaggagg acctgcagca tttgccgccc 2400
cagcccctgc caccccagct gagccaggsc gatggccgtt ccgagacgtc acagcccccc 2460
acgcctggca gcccttccca ctcacccctg cactcgggac ccgccctcg acggagagag 2520
tcggacccca caggcccaca gagacagttg gaggaggaca gactctcggg gcactccctc 2580
ccgcggtaca gccccctgcg acgactggcg tcctccgtgt tctcctcctc cacgctggag 2640
acggagcatt accctcaccc cggcggcggc ggctcctcgg gctcctccgg ttccctcatt 2700
cagcgcagcc gctcggctga gagcagccct gtgcgggcgc cccaccggcg ccacgcgccc 2760
ctcgctgctg gcaaccacag actcatgccc tcggtgctcc gcatctcgcg gtcccagctg 2820
cagcaggtgt gggcccggtt cacccacaag acctag 2856

<210> 36
<211> 951
<212> PRT
<213> homo sapiens

<220>
<221> VARIANT
<222> (1)...(951)
<223> Xaa = Any Amino Acid

<400> 36
Met Leu Ala Ala Arg Cys Ile Val Gly Ser Ser Pro Leu Cys Arg Cys
1               5                   10                  15
Arg Arg Arg Arg Pro Arg Arg Ile Gly Ala Gly Pro Gly Arg Asp Asp
            20                  25                  30
Pro Gly Arg Lys Ala Ala Ala Ala Gly Gly Ser Gly Ser Pro Asn Ala
        35                  40                  45
Ala Leu Ser Arg Pro Arg Pro Ala Pro Ala Pro Gly Asp Ala Pro Pro
    50                  55                  60
Arg Ala Ala Ala Ser Ala Ala Ala Ala Ala Ala Ala Ala Ala Gly Thr
65                  70                  75                  80
Glu Gln Val Asp Gly Pro Leu Arg Ala Gly Pro Ala Asp Thr Pro Pro
                85                  90                  95
Ser Gly Trp Arg Met Gln Cys Leu Ala Ala Ala Leu Lys Asp Glu Thr
            100                 105                 110
Asn Met Ser Gly Gly Gly Glu Gln Ala Asp Ile Leu Pro Ala Asn Tyr
        115                 120                 125
Val Val Lys Asp Arg Trp Lys Val Leu Lys Lys Ile Gly Gly Gly Gly
    130                 135                 140
Phe Gly Glu Ile Tyr Glu Ala Met Asp Leu Leu Thr Arg Glu Asn Val
145                 150                 155                 160
Ala Leu Lys Val Glu Ser Ala Gln Gln Pro Lys Gln Val Leu Lys Met
                165                 170                 175
Glu Val Ala Val Leu Lys Lys Leu Gln Gly Lys Asp His Val Cys Arg
            180                 185                 190

Phe Ile Gly Cys Gly Arg Asn Glu Lys Phe Asn Tyr Val Val Met Gln
        195                 200                 205
Leu Gln Gly Arg Asn Leu Ala Asp Leu Arg Arg Ser Gln Pro Arg Gly
    210                 215                 220
Thr Phe Thr Leu Ser Thr Thr Leu Arg Leu Gly Lys Gln Ile Leu Glu
225                 230                 235                 240
Ser Ile Glu Ala Ile His Ser Val Gly Phe Leu His Arg Asp Ile Lys
                245                 250                 255
Pro Ser Asn Phe Ala Met Gly Arg Leu Pro Ser Thr Tyr Arg Lys Cys
            260                 265                 270
Tyr Met Leu Asp Phe Gly Leu Ala Arg Gln Tyr Thr Asn Thr Thr Gly
        275                 280                 285
Asp Val Arg Pro Pro Arg Asn Val Ala Gly Phe Arg Gly Thr Val Arg
    290                 295                 300
Tyr Ala Ser Val Asn Ala His Lys Asn Arg Glu Met Gly Arg His Asp
305                 310                 315                 320
Asp Leu Trp Ser Leu Phe Tyr Met Leu Val Glu Phe Ala Val Gly Gln
                325                 330                 335
Leu Pro Trp Arg Lys Ile Lys Asp Lys Glu Gln Val Gly Met Ile Lys
            340                 345                 350
Glu Lys Tyr Glu His Arg Met Leu Leu Lys His Met Pro Ser Glu Phe
        355                 360                 365
His Leu Phe Leu Asp His Ile Ala Ser Leu Asp Tyr Phe Thr Lys Pro
    370                 375                 380
Asp Tyr Gln Leu Ile Met Ser Val Phe Glu Asn Ser Met Lys Glu Arg
385                 390                 395                 400
Gly Ile Ala Glu Asn Glu Ala Phe Asp Trp Glu Lys Ala Gly Thr Asp
                405                 410                 415
Ala Leu Leu Ser Thr Ser Thr Ser Thr Pro Pro Gln Gln Asn Thr Arg
            420                 425                 430
Gln Thr Ala Ala Met Phe Gly Val Val Asn Val Thr Pro Val Pro Gly
        435                 440                 445
Asp Leu Leu Arg Glu Asn Thr Glu Asp Val Leu Gln Gly Glu His Leu
    450                 455                 460
Ser Asp Gln Glu Asn Ala Pro Pro Ile Leu Pro Gly Arg Pro Ser Glu
465                 470                 475                 480
Gly Leu Gly Pro Ser Pro His Leu Val Pro His Pro Gly Gly Pro Glu
                485                 490                 495
Ala Glu Val Trp Glu Glu Thr Asp Val Asn Arg Asn Lys Leu Arg Ile
            500                 505                 510
Asn Ile Gly Lys Val Thr Ala Ala Arg Ala Lys Gly Val Gly Gly Leu
        515                 520                 525
Phe Ser His Pro Arg Phe Pro Ala Leu Cys Pro Cys Pro Val Pro Pro
    530                 535                 540
Lys His Pro Val Pro Gly His Leu Pro Ala Cys Pro Ala Ser Val Ser
545                 550                 555                 560
Arg Ser Leu Pro Ala Leu Ala Ser Leu Cys Leu Pro Ser Ser Ser Ser
                565                 570                 575
Ser Val Ser Phe Thr Leu Arg Arg Pro Ser Ala His Ser Arg Leu Ile
            580                 585                 590
Ser Pro Ser Ser Trp His Ser Pro Leu Leu Gln Ser Pro Cys Val Glu
        595                 600                 605
Glu Glu Gln Ser Arg Gly Met Gly Val Pro Ser Ser Pro Val Arg Ala
    610                 615                 620
Pro Pro Asp Ser Pro Thr Thr Pro Val Arg Ser Leu Arg Tyr Arg Arg
625                 630                 635                 640
Val Asn Ser Pro Glu Ser Glu Arg Leu Ser Thr Ala Asp Gly Arg Val
                645                 650                 655
Glu Leu Pro Glu Arg Arg Ser Arg Met Asp Leu Pro Gly Ser Pro Ser
            660                 665                 670
Arg Gln Ala Cys Ser Ser Gln Pro Ala Gln Met Leu Ser Val Asp Thr
        675                 680                 685
Gly His Ala Asp Arg Gln Ala Ser Gly Arg Met Asp Val Ser Ala Ser
    690                 695                 700
Val Glu Gln Glu Ala Leu Ser Asn Ala Phe Arg Ser Val Pro Leu Ala
705                 710                 715                 720
Glu Glu Glu Asp Phe Asp Ser Lys Glu Trp Val Ile Ile Asp Lys Glu
                725                 730                 735

Thr Glu Leu Lys Asp Phe Pro Pro Gly Ala Glu Pro Ser Thr Ser Gly
            740                 745                 750
Thr Thr Asp Glu Glu Pro Glu Glu Leu Arg Pro Leu Pro Glu Glu Gly
        755                 760                 765
Glu Glu Arg Arg Arg Leu Gly Ala Glu Pro Thr Val Arg Pro Arg Gly
    770                 775                 780
Arg Ser Met Gln Ala Leu Ala Glu Glu Asp Leu Gln His Leu Pro Pro
785                 790                 795                 800
Gln Pro Leu Pro Pro Gln Leu Ser Gln Xaa Asp Gly Arg Ser Glu Thr
                805                 810                 815
Ser Gln Pro Pro Thr Pro Gly Ser Pro Ser His Ser Pro Leu His Ser
            820                 825                 830
Gly Pro Arg Pro Arg Arg Arg Glu Ser Asp Pro Thr Gly Pro Gln Arg
        835                 840                 845
Gln Leu Glu Glu Asp Arg Leu Ser Gly His Ser Leu Pro Arg Tyr Ser
    850                 855                 860
Pro Leu Arg Arg Leu Ala Ser Ser Val Phe Ser Ser Ser Thr Leu Glu
865                 870                 875                 880
Thr Glu His Tyr Pro His Pro Gly Gly Gly Gly Ser Ser Gly Ser Ser
                885                 890                 895
Gly Ser Leu Ile Gln Arg Ser Arg Ser Ala Glu Ser Ser Pro Val Arg
            900                 905                 910
Ala Pro His Arg Arg His Ala Pro Leu Ala Ala Gly Asn His Arg Leu
        915                 920                 925
Met Pro Ser Val Leu Arg Ile Ser Arg Ser Gln Leu Gln Gln Val Trp
    930                 935                 940
Ala Arg Phe Thr His Lys Thr
945                 950


<210> 37
<211> 2556
<212> DNA
<213> homo sapiens

<400> 37
atgcagtgcc tagcggccgc ccttaaggac gaaaccaaca tgagtggggg aggggagcag 60
gccgacatcc tgccggccaa ctacgtggtc aaggatcgct ggaaggtgct gaaaaagatc 120
gggggcgggg gctttggtga gatctacgag gccatggacc tgctgaccag ggagaatgtg 180
gccctcaagg tggagtcagc ccagcagccc aagcaggtcc tcaagatgga ggtggccgtg 240
ctcaagaagt tgcaagggaa ggaccatgtg tgcaggttca ttggctgtgg caggaacgag 300
aagtttaact atgtagtgat gcagctccag ggccggaacc tggccgacct gcgccgtagc 360
cagccgcgag gcaccttcac gctgagcacc acattgcggc tgggcaagca gatcttggag 420
tccatcgagg ccatccactc tgtgggcttc ctgcaccgtg acatcaagcc ttcaaacttt 480
gccatgggca ggctgccctc cacctacagg aagtgctata tgctggactt cgggctggcc 540
cggcagtaca ccaacaccac gggggatgtg cggccccctc ggaatgtggc cgggtttcga 600
ggaacggttc gctatgcctc agtcaatgcc cacaagaacc gggagatggg ccgccacgac 660
gacctgtggt ccctcttcta catgctggtg gagtttgcag tgggccagct gccctggagg 720
aagatcaagg acaaggaaca ggtagggatg atcaaggaga agtatgagca ccggatgctg 780
ctgaagcaca tgccgtcaga gttccacctc ttcctggacc acattgccag cctcgactac 840
ttcaccaagc ccgactacca gttgatcatg tcagtgtttg agaacagcat gaaggagagg 900
ggcattgccg agaatgaggc ctttgactgg gagaaggcag gcaccgatgc cctcctgtcc 960
acgagcacct ctaccccgcc ccagcagaac acccggcaga cggcagccat gtttggggtg 1020
gtcaatgtga cgccagtgcc tggggacctg ctccgggaga acaccgagga tgtgctacag 1080
ggagagcacc tgagtgacca ggagaatgca cccccaattc tgcccgggag gccctctgag 1140
gggctgggcc ccagtcccca ccttgtcccc caccccgggg gtcctgaggc tgaagtctgg 1200
gaggagacag atgtcaaccg gaacaaactc cggatcaaca tcggcaaagt aactgccgcc 1260
agggcgaagg gcgtgggtgg ccttttctct cacccccgat tcccagcctt gtgcccctgc 1320
cctgttcctc ctaagcaccc tgtccccggc catctgcctg cttgccctgc ctctgtttcc 1380
cggtccctcc ccgcactagc ctcgctgtgt cttccatcat catcatcctc tgtctccttc 1440
accctgagga gaccatccgc ccacagccgc ctcatcagcc ccagctcatg gcactcccct 1500
ctcctgcaga gcccctgtgt ggaggaggaa cagagccgag gcatgggggt ccccagctcc 1560
ccagtgcgtg cccccccaga ctcccccaca accccagtcc gttctctgcg ctaccggagg 1620
gtgaacagcc ctgagtcaga aaggctgtcc acggcggacg ggcgagtgga gctacctgag 1680
aggaggtcac ggatggatct gcctggctcg ccctcgcgcc aggcctgctc ctctcagcca 1740
gcccagatgc tgtcagtgga cacaggccac gctgaccgac aggccagtgg ccgcatggay 1800
gtgtcagcct ctgtggagca ggaggccctg agcaacgcct tccgctcggt gccgctggct 1860
gaggaggagg atttcgacag caaagagtgg gtcatcatcg acaaggagac ggagctcaag 1920

gacttccctc caggggctga gcccagcaca tcgggcacca cggatgagga gcccgaggag 1980
ctgcggccac tgcccgagga gggcgaagag cggcggcggc tgggggcaga gcccaccgtc 2040
cggccccggg gacgcagcat gcaggcgctg gcggaggagg acctgcagca tttgccgccc 2100
cagcccctgc caccccagct gagccaggsc gatggccgtt ccgagacgtc acagcccccc 2160
acgcctggca gcccttccca ctcacccctg cactcgggac cccgccctcg acggagagag 2220
tcggacccca caggcccaca gagacagttg gaggaggaca gactctcggg gcactccctc 2280
ccgcggtaca gccccctgcg acgactggcg tcctccgtgt tctcctcctc cacgctggag 2340
acggagcatt accctcaccc cggcggcggc ggctcctcgg gctcctccgg ttccctcatt 2400
cagcgcagcc gctcggctga gagcagccct gtgcgggcgc cccaccggcg ccacgcgccc 2460
ctcgctgctg gcaaccacag actcatgccc tcggtgctcc gcatctcgcg gtcccagctg 2520
cagcaggtgt gggcccggtt cacccacaag acctag 2556

<210> 38
<211> 851
<212> PRT
<213> homo sapiens

<220>
<221> VARIANT
<222> (1)...(851)
<223> Xaa = Any Amino Acid

<400> 38
Met Gln Cys Leu Ala Ala Ala Leu Lys Asp Glu Thr Asn Met Ser Gly
1               5                   10                  15
Gly Gly Glu Gln Ala Asp Ile Leu Pro Ala Asn Tyr Val Val Lys Asp
            20                  25                  30
Arg Trp Lys Val Leu Lys Lys Ile Gly Gly Gly Gly Phe Gly Glu Ile
        35                  40                  45
Tyr Glu Ala Met Asp Leu Leu Thr Arg Glu Asn Val Ala Leu Lys Val
    50                  55                  60
Glu Ser Ala Gln Gln Pro Lys Gln Val Leu Lys Met Glu Val Ala Val
65                  70                  75                  80
Leu Lys Lys Leu Gln Gly Lys Asp His Val Cys Arg Phe Ile Gly Cys
                85                  90                  95
Gly Arg Asn Glu Lys Phe Asn Tyr Val Val Met Gln Leu Gln Gly Arg
            100                 105                 110
Asn Leu Ala Asp Leu Arg Arg Ser Gln Pro Arg Gly Thr Phe Thr Leu
        115                 120                 125
Ser Thr Thr Leu Arg Leu Gly Lys Gln Ile Leu Glu Ser Ile Glu Ala
    130                 135                 140
Ile His Ser Val Gly Phe Leu His Arg Asp Ile Lys Pro Ser Asn Phe
145                 150                 155                 160
Ala Met Gly Arg Leu Pro Ser Thr Tyr Arg Lys Cys Tyr Met Leu Asp
                165                 170                 175
Phe Gly Leu Ala Arg Gln Tyr Thr Asn Thr Thr Gly Asp Val Arg Pro
            180                 185                 190
Pro Arg Asn Val Ala Gly Phe Arg Gly Thr Val Arg Tyr Ala Ser Val
        195                 200                 205
Asn Ala His Lys Asn Arg Glu Met Gly Arg His Asp Asp Leu Trp Ser
    210                 215                 220
Leu Phe Tyr Met Leu Val Glu Phe Ala Val Gly Gln Leu Pro Trp Arg
225                 230                 235                 240
Lys Ile Lys Asp Lys Glu Gln Val Gly Met Ile Lys Glu Lys Tyr Glu
                245                 250                 255
His Arg Met Leu Leu Lys His Met Pro Ser Glu Phe His Leu Phe Leu
            260                 265                 270
Asp His Ile Ala Ser Leu Asp Tyr Phe Thr Lys Pro Asp Tyr Gln Leu
        275                 280                 285
Ile Met Ser Val Phe Glu Asn Ser Met Lys Glu Arg Gly Ile Ala Glu
    290                 295                 300
Asn Glu Ala Phe Asp Trp Glu Lys Ala Gly Thr Asp Ala Leu Leu Ser
305                 310                 315                 320
Thr Ser Thr Ser Thr Pro Pro Gln Gln Asn Thr Arg Gln Thr Ala Ala
                325                 330                 335
Met Phe Gly Val Val Asn Val Thr Pro Val Pro Gly Asp Leu Leu Arg
            340                 345                 350
Glu Asn Thr Glu Asp Val Leu Gln Gly Glu His Leu Ser Asp Gln Glu

        355                 360                 365
Asn Ala Pro Pro Ile Leu Pro Gly Arg Pro Ser Glu Gly Leu Gly Pro
    370                 375                 380
Ser Pro His Leu Val Pro His Pro Gly Gly Pro Glu Ala Glu Val Trp
385                 390                 395                 400
Glu Glu Thr Asp Val Asn Arg Asn Lys Leu Arg Ile Asn Ile Gly Lys
                405                 410                 415
Val Thr Ala Ala Arg Ala Lys Gly Val Gly Gly Leu Phe Ser His Pro
            420                 425                 430
Arg Phe Pro Ala Leu Cys Pro Cys Pro Val Pro Pro Lys His Pro Val
        435                 440                 445
Pro Gly His Leu Pro Ala Cys Pro Ala Ser Val Ser Arg Ser Leu Pro
    450                 455                 460
Ala Leu Ala Ser Leu Cys Leu Pro Ser Ser Ser Ser Ser Val Ser Phe
465                 470                 475                 480
Thr Leu Arg Arg Pro Ser Ala His Ser Arg Leu Ile Ser Pro Ser Ser
                485                 490                 495
Trp His Ser Pro Leu Leu Gln Ser Pro Cys Val Glu Glu Glu Gln Ser
            500                 505                 510
Arg Gly Met Gly Val Pro Ser Ser Pro Val Arg Ala Pro Pro Asp Ser
        515                 520                 525
Pro Thr Thr Pro Val Arg Ser Leu Arg Tyr Arg Arg Val Asn Ser Pro
    530                 535                 540
Glu Ser Glu Arg Leu Ser Thr Ala Asp Gly Arg Val Glu Leu Pro Glu
545                 550                 555                 560
Arg Arg Ser Arg Met Asp Leu Pro Gly Ser Pro Ser Arg Gln Ala Cys
                565                 570                 575
Ser Ser Gln Pro Ala Gln Met Leu Ser Val Asp Thr Gly His Ala Asp
            580                 585                 590
Arg Gln Ala Ser Gly Arg Met Asp Val Ser Ala Ser Val Glu Gln Glu
        595                 600                 605
Ala Leu Ser Asn Ala Phe Arg Ser Val Pro Leu Ala Glu Glu Glu Asp
    610                 615                 620
Phe Asp Ser Lys Glu Trp Val Ile Ile Asp Lys Glu Thr Glu Leu Lys
625                 630                 635                 640
Asp Phe Pro Pro Gly Ala Glu Pro Ser Thr Ser Gly Thr Thr Asp Glu
                645                 650                 655
Glu Pro Glu Glu Leu Arg Pro Leu Pro Glu Glu Gly Glu Glu Arg Arg
            660                 665                 670
Arg Leu Gly Ala Glu Pro Thr Val Arg Pro Arg Gly Arg Ser Met Gln
        675                 680                 685
Ala Leu Ala Glu Glu Asp Leu Gln His Leu Pro Pro Gln Pro Leu Pro
    690                 695                 700
Pro Gln Leu Ser Gln Xaa Asp Gly Arg Ser Glu Thr Ser Gln Pro Pro
705                 710                 715                 720
Thr Pro Gly Ser Pro Ser His Ser Pro Leu His Ser Gly Pro Arg Pro
                725                 730                 735
Arg Arg Arg Glu Ser Asp Pro Thr Gly Pro Gln Arg Gln Leu Glu Glu
            740                 745                 750
Asp Arg Leu Ser Gly His Ser Leu Pro Arg Tyr Ser Pro Leu Arg Arg
        755                 760                 765
Leu Ala Ser Ser Val Phe Ser Ser Ser Thr Leu Glu Thr Glu His Tyr
    770                 775                 780
Pro His Pro Gly Gly Gly Gly Ser Ser Gly Ser Ser Gly Ser Leu Ile
785                 790                 795                 800
Gln Arg Ser Arg Ser Ala Glu Ser Ser Pro Val Arg Ala Pro His Arg
                805                 810                 815
Arg His Ala Pro Leu Ala Ala Gly Asn His Arg Leu Met Pro Ser Val
            820                 825                 830
Leu Arg Ile Ser Arg Ser Gln Leu Gln Gln Val Trp Ala Arg Phe Thr
        835                 840                 845
His Lys Thr
    850

<210> 39
<211> 2517
<212> DNA

<213> homo sapiens

<400> 39
   atgagtgggg gaggggagca ggccgacatc ctgccggcca actacgtggt caaggatcgc 60
   tggaaggtgc tgaaaaagat cgggggcggg ggctttggtg agatctacga ggccatggac 120
   ctgctgacca gggagaatgt ggccctcaag gtggagtcag cccagcagcc caagcaggtc 180
   ctcaagatgg aggtggccgt gctcaagaag ttgcaaggga aggaccatgt gtgcaggttc 240
   attggctgtg gcaggaacga gaagtttaac tatgtagtga tgcagctcca gggccggaac 300
   ctggccgacc tgcgccgtag ccagccgcga ggcaccttca cgctgagcac cacattgcgg 360
   ctgggcaagc agatcttgga gtccatcgag gccatccact ctgtgggctt cctgcaccgt 420
   gacatcaagc cttcaaactt tgccatgggc aggctgccct ccacctacag gaagtgctat 480
   atgctggact tcgggctggc ccggcagtac accaacacca cgggggatgt gcggccccct 540
   cggaatgtgg ccgggtttcg aggaacggtt cgctatgcct cagtcaatgc ccacaagaac 600
   cgggagatgg gccgccacga cgacctgtgg tccctcttct acatgctggt ggagtttgca 660
   gtgggccagc tgccctggag gaagatcaag gacaaggaac aggtagggat gatcaaggag 720
   aagtatgagc accggatgct gctgaagcac atgccgtcag agttccacct cttcctggac 780
   cacattgcca gcctcgacta cttcaccaag cccgactacc agttgatcat gtcagtgttt 840
   gagaacagca tgaaggagag gggcattgcc gagaatgagg cctttgactg ggagaaggca 900
   ggcaccgatg ccctcctgtc cacgagcacc tctaccccgc cccagcagaa cacccggcag 960
   acggcagcca tgtttggggt ggtcaatgtg acgccagtgc ctggggacct gctccgggag 1020
   aacaccgagg atgtgctaca gggagagcac ctgagtgacc aggagaatgc acccccaatt 1080
   ctgcccggga ggccctctga ggggctgggc cccagtcccc accttgtccc ccaccccggg 1140
   ggtcctgagg ctgaagtctg ggaggagaca gatgtcaacc ggaacaaact ccggatcaac 1200
   atcggcaaag taactgccgc cagggcgaag ggcgtgggtg gccttttctc tcacccccga 1260
   ttcccagcct tgtgcccctg ccctgttcct cctaagcacc ctgtccccgg ccatctgcct 1320
   gcttgccctg cctctgtttc ccggtccctc cccgcactag cctcgctgtg tcttccatca 1380
   tcatcatcct ctgtctcctt caccctgagg agaccatccg cccacagccg cctcatcagc 1440
   cccagctcat ggcactcccc tctcctgcag agcccctgtg tggaggagga acagagccga 1500
   ggcatggggg tccccagctc cccagtgcgt gcccccccag actcccccac aaccccagtc 1560
   cgttctctgc gctaccggag ggtgaacagc cctgagtcag aaaggctgtc cacggcggac 1620
   gggcgagtgg agctacctga gaggaggtca cggatggatc tgcctggctc gccctcgcgc 1680
   caggcctgct cctctcagcc agcccagatg ctgtcagtgg acacaggcca cgctgaccga 1740
   caggccagtg gccgcatgga ygtgtcagcc tctgtggagc aggaggccct gagcaacgcc 1800
   ttccgctcgg tgccgctggc tgaggaggag gatttcgaca gcaaagagtg ggtcatcatc 1860
   gacaaggaga cggagctcaa ggacttccct ccaggggctg agcccagcac atcgggcacc 1920
   acggatgagg agcccgagga gctgcggcca ctgcccgagg agggcgaaga gcggcggcgg 1980
   ctgggggcag agcccaccgt ccggccccgg ggacgcagca tgcaggcgct ggcggaggag 2040
   gacctgcagc atttgccgcc ccagcccctg ccaccccagc tgagccaggs cgatggccgt 2100
   tccgagacgt cacagccccc cacgcctggc agcccttccc actcacccct gcactcggga 2160
   ccccgccctc gacggagaga gtcggacccc acaggcccac agagacagtt ggaggaggac 2220
   agactctcgg ggcactccct cccgcggtac agccccctgc gacgactggc gtcctccgtg 2280
   ttctcctcct ccacgctgga gacggagcat taccctcacc ccggcggcgg cggctcctcg 2340
   ggctcctccg gttccctcat tcagcgcagc cgctcggctg agagcagccc tgtgcgggcg 2400
   ccccaccggc gccacgcgcc cctcgctgct ggcaaccaca gactcatgcc ctcggtgctc 2460
   cgcatctcgc ggtcccagct gcagcaggtg tgggcccggt tcacccacaa gacctag 2517

<210> 40
<211> 838
<212> PRT
<213> homo sapiens

<220>
<221> VARIANT
<222> (1)...(838)
<223> Xaa = Any Amino Acid

<400> 40
   Met Ser Gly Gly Gly Glu Gln Ala Asp Ile Leu Pro Ala Asn Tyr Val
    1               5                  10                  15
   Val Lys Asp Arg Trp Lys Val Leu Lys Lys Ile Gly Gly Gly Gly Phe
               20                  25                  30
   Gly Glu Ile Tyr Glu Ala Met Asp Leu Leu Thr Arg Glu Asn Val Ala
           35                  40                  45
   Leu Lys Val Glu Ser Ala Gln Gln Pro Lys Gln Val Leu Lys Met Glu
       50                  55                  60
   Val Ala Val Leu Lys Lys Leu Gln Gly Lys Asp His Val Cys Arg Phe
   65                  70                  75                  80
   Ile Gly Cys Gly Arg Asn Glu Lys Phe Asn Tyr Val Val Met Gln Leu
   85 90 95
   Gln Gly Arg Asn Leu Ala Asp Leu Arg Arg Ser Gln Pro Arg Gly Thr
   100 105 110
   Phe Thr Leu Ser Thr Thr Leu Arg Leu Gly Lys Gln Ile Leu Glu Ser
   115 120 125
   Ile Glu Ala Ile His Ser Val Gly Phe Leu His Arg Asp Ile Lys Pro
   130 135 140
   Ser Asn Phe Ala Met Gly Arg Leu Pro Ser Thr Tyr Arg Lys Cys Tyr
   145 150 155 160
   Met Leu Asp Phe Gly Leu Ala Arg Gln Tyr Thr Asn Thr Thr Gly Asp
   165 170 175
   Val Arg Pro Pro Arg Asn Val Ala Gly Phe Arg Gly Thr Val Arg Tyr
   180 185 190
   Ala Ser Val Asn Ala His Lys Asn Arg Glu Met Gly Arg His Asp Asp
   195 200 205
   Leu Trp Ser Leu Phe Tyr Met Leu Val Glu Phe Ala Val Gly Gln Leu
   210 215 220
   Pro Trp Arg Lys Ile Lys Asp Lys Glu Gln Val Gly Met Ile Lys Glu
   225 230 235 240
   Lys Tyr Glu His Arg Met Leu Leu Lys His Met Pro Ser Glu Phe His
   245 250 255
   Leu Phe Leu Asp His Ile Ala Ser Leu Asp Tyr Phe Thr Lys Pro Asp
   260 265 270
   Tyr Gln Leu Ile Met Ser Val Phe Glu Asn Ser Met Lys Glu Arg Gly
   275 280 285
   Ile Ala Glu Asn Glu Ala Phe Asp Trp Glu Lys Ala Gly Thr Asp Ala
   290 295 300
   Leu Leu Ser Thr Ser Thr Ser Thr Pro Pro Gln Gln Asn Thr Arg Gln
   305 310 315 320
   Thr Ala Ala Met Phe Gly Val Val Asn Val Thr Pro Val Pro Gly Asp
   325 330 335
   Leu Leu Arg Glu Asn Thr Glu Asp Val Leu Gln Gly Glu His Leu Ser
   340 345 350
   Asp Gln Glu Asn Ala Pro Pro Ile Leu Pro Gly Arg Pro Ser Glu Gly
   355 360 365
   Leu Gly Pro Ser Pro His Leu Val Pro His Pro Gly Gly Pro Glu Ala
   370 375 380
   Glu Val Trp Glu Glu Thr Asp Val Asn Arg Asn Lys Leu Arg Ile Asn
   385 390 395 400
   Ile Gly Lys Val Thr Ala Ala Arg Ala Lys Gly Val Gly Gly Leu Phe
   405 410 415
   Ser His Pro Arg Phe Pro Ala Leu Cys Pro Cys Pro Val Pro Pro Lys
   420 425 430
   His Pro Val Pro Gly His Leu Pro Ala Cys Pro Ala Ser Val Ser Arg
   435 440 445
   Ser Leu Pro Ala Leu Ala Ser Leu Cys Leu Pro Ser Ser Ser Ser Ser
   450 455 460
   Val Ser Phe Thr Leu Arg Arg Pro Ser Ala His Ser Arg Leu Ile Ser
   465 470 475 480
   Pro Ser Ser Trp His Ser Pro Leu Leu Gln Ser Pro Cys Val Glu Glu
   485 490 495
   Glu Gln Ser Arg Gly Met Gly Val Pro Ser Ser Pro Val Arg Ala Pro
   500 505 510
   Pro Asp Ser Pro Thr Thr Pro Val Arg Ser Leu Arg Tyr Arg Arg Val
   515 520 525
   Asn Ser Pro Glu Ser Glu Arg Leu Ser Thr Ala Asp Gly Arg Val Glu
   530 535 540
   Leu Pro Glu Arg Arg Ser Arg Met Asp Leu Pro Gly Ser Pro Ser Arg
   545 550 555 560
   Gln Ala Cys Ser Ser Gln Pro Ala Gln Met Leu Ser Val Asp Thr Gly
   565 570 575
   His Ala Asp Arg Gln Ala Ser Gly Arg Met Asp Val Ser Ala Ser Val
   580 585 590
   Glu Gln Glu Ala Leu Ser Asn Ala Phe Arg Ser Val Pro Leu Ala Glu
   595 600 605
   Glu Glu Asp Phe Asp Ser Lys Glu Trp Val Ile Ile Asp Lys Glu Thr
   610 615 620
   Glu Leu Lys Asp Phe Pro Pro Gly Ala Glu Pro Ser Thr Ser Gly Thr
   625                 630                 635                 640
   Thr Asp Glu Glu Pro Glu Glu Leu Arg Pro Leu Pro Glu Glu Gly Glu
                   645                 650                 655
   Glu Arg Arg Arg Leu Gly Ala Glu Pro Thr Val Arg Pro Arg Gly Arg
               660                 665                 670
   Ser Met Gln Ala Leu Ala Glu Glu Asp Leu Gln His Leu Pro Pro Gln
           675                 680                 685
   Pro Leu Pro Pro Gln Leu Ser Gln Xaa Asp Gly Arg Ser Glu Thr Ser
       690                 695                 700
   Gln Pro Pro Thr Pro Gly Ser Pro Ser His Ser Pro Leu His Ser Gly
   705                 710                 715                 720
   Pro Arg Pro Arg Arg Arg Glu Ser Asp Pro Thr Gly Pro Gln Arg Gln
                   725                 730                 735
   Leu Glu Glu Asp Arg Leu Ser Gly His Ser Leu Pro Arg Tyr Ser Pro
               740                 745                 750
   Leu Arg Arg Leu Ala Ser Ser Val Phe Ser Ser Ser Thr Leu Glu Thr
           755                 760                 765
   Glu His Tyr Pro His Pro Gly Gly Gly Gly Ser Ser Gly Ser Ser Gly
       770                 775                 780
   Ser Leu Ile Gln Arg Ser Arg Ser Ala Glu Ser Ser Pro Val Arg Ala
   785                 790                 795                 800
   Pro His Arg Arg His Ala Pro Leu Ala Ala Gly Asn His Arg Leu Met
                   805                 810                 815
   Pro Ser Val Leu Arg Ile Ser Arg Ser Gln Leu Gln Gln Val Trp Ala
               820                 825                 830
   Arg Phe Thr His Lys Thr
           835

## Description

The present application claims the benefit of U.S. Provisional Application Numbers 60/255,103 and 60/289,422 which were filed on December 12, 2000 and May 8, 2001 and are herein incorporated by reference in their entirety.

### 1. INTRODUCTION

The present invention relates to the discovery, identification, and characterization of novel human polynucleotides encoding proteins sharing sequence similarity with animal kinases. The invention encompasses the described polynucleotides, host cell expression systems, the encoded proteins, fusion proteins, polypeptides and peptides, antibodies to the encoded proteins and peptides, and genetically engineered animals that either lack or overexpress the disclosed genes, antagonists and agonists of the proteins, and other compounds that modulate the expression or activity of the proteins encoded by the disclosed genes, which can be used for diagnosis, drug screening, clinical trial monitoring, the treatment of diseases and disorders, and cosmetic or nutriceutical applications.

### 2. BACKGROUND OF THE INVENTION

Kinases mediate the phosphorylation of a wide variety of proteins and compounds in the cell. Along with phosphatases, kinases are involved in a range of regulatory pathways. Given the physiological importance of kinases, they have been subject to intense scrutiny and are proven drug targets.

### 3. SUMMARY OF THE INVENTION

The present invention relates to the discovery, identification, and characterization of nucleotides that encode novel human proteins and the corresponding amino acid sequences of these proteins. The novel human proteins (NHPs) described for the first time herein share structural similarity with animal kinases, including, but not limited to, serine-threonine kinases, casein kinases, calcium/calmodulin-dependent protein kinases, and mitogen activated kinases. Accordingly, the described NHPs encode novel kinases having homologues and orthologs across a range of phyla and species.

The novel human polynucleotides described herein, encode open reading frames (ORFs) encoding proteins of 870, 864, 764, 751, 654, 648, 548, 535, 895, 889, 789, 776, 982, 976, 876, 863, 957, 951, 851, and 838 amino acids in length (see respectively SEQ ID NOS: 2, 4, 6, 8, 10, 12, 14, 16, 18, 20, 22, 24, 26, 28, 30, 32, 34, 36, 38, and 40).

The invention also encompasses agonists and antagonists of the described NHPs, including small molecules, large molecules, mutant NHPs, or portions thereof, that compete with native NHP, peptides, and antibodies, as well as nucleotide sequences that can be used to inhibit the expression of the described NHPs (e.g., antisense and ribozyme molecules, and open reading frame or regulatory sequence replacement constructs) or to enhance the expression of the described NHPs (e.g., expression constructs that place the described polynucleotide under the control of a strong promoter system), and transgenic animals that express a NHP sequence, or "knock-outs" (which can be conditional) that do not express a functional NHP. Knock-out mice can be produced in several ways, one of which involves the use of mouse embryonic stem cells ("ES cells") lines that contain gene trap mutations in a murine homolog of at least one of the described NHPs. When the unique NHP sequences described in SEQ ID NOS:1-40 are "knocked-out" they provide a method of identifying phenotypic expression of the particular gene as well as a method of assigning function to previously unknown genes. In addition, animals in which the unique NHP sequences described in SEQ ID NOS:1-40 are "knocked-out" provide a unique source in which to elicit antibodies to homologous and orthologous proteins which would have been previously viewed by the immune system as "self" and therefore would have failed to elicit significant antibody responses. To these ends, gene trapped knockout ES cells have been generated in murine homologs of the described NHPs.

Additionally, the unique NHP sequences described in SEQ ID NOS:1-40 are useful for the identification of protein coding sequence and mapping a unique gene to a particular chromosome. These sequences identify actual, biologically verified, and therefore relevant, exon splice junctions as opposed to those that may have been bioinformatically predicted from genomic sequence alone. The sequences of the present invention are also useful as additional DNA markers for restriction fragment length polymorphism (RFLP) analysis, and in forensic biology.

Further, the present invention also relates to processes for identifying compounds that modulate, i.e., act as agonists or antagonists, of NHP expression and/or NHP activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequence of the novel human ORFs encoding the described novel human kinase proteins.

### 5. DETAILED DESCRIPTION OF THE INVENTION

The NHPs described for the first time herein are novel proteins that are expressed in, inter alia, human cell lines and human fetal brain, brain, pituitary, cerebellum, and fetal lung, kidney, and embryo cells. The described sequences were compiled from sequences available in GENBANK, and cDNAs generated from human brain and cerebellum mRNAs (Edge Biosystems, Gaithersburg, MD) that were identified using primers generated from human genomic DNA.

The present invention encompasses the nucleotides presented in the Sequence Listing, host cells expressing such nucleotides, the expression products of such nucleotides, and: (a) nucleotides that encode mammalian homologs of the described genes, including the specifically described NHPs, and the NHP products; (b) nucleotides that encode one or more portions of an NHP that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of a NHP, or one of its domains (e.g., a receptor/ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing. As discussed above, the present invention includes: (a) the human DNA sequences presented in the Sequence Listing (and vectors comprising the same) and additionally contemplates any nucleotide sequence encoding a contiguous NHP open reading frame (ORF) that hybridizes to a complement of a DNA sequence presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. *et al.,* eds., 1989, Current Protocols in Molecular Biology, Vol. I, Green Publishing Associates, Inc., and John Wiley & Sons, Inc., NY, at p. 2.10.3) and encodes a functionally equivalent expression product.

Additionally, contemplated are any nucleotide sequences that hybridize to the complement of the DNA sequence that encode and express an amino acid sequence presented in the Sequence Listing under moderately stringent conditions, e.g., washing in 0.2xSSC/0.1% SDS at 42°C (Ausubel et al., 1989, *supra),* yet still encode a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent Nos. 5,837,458 or 5,723,323 both of which are herein incorporated by reference). The invention also includes degenerate nucleic acid variants of the disclosed NHP polynucleotide sequences.

Additionally contemplated are polynucleotides encoding NHP ORFs, or their functional equivalents, encoded by polynucleotide sequences that are about 99, 95, 90, or about 85 percent similar to corresponding regions of SEQ ID NO:1 (as measured by BLAST sequence comparison analysis using, for example, the GCG sequence analysis package using default parameters).

The invention also includes nucleic acid molecules, preferably DNA molecules, that hybridize to, and are therefore the complements of, the described NHP encoding polynucleotides. Such hybridization conditions can be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-40 can be used as a hybridization probe in conjunction with a solid support matrix/substrate (resins, beads, membranes, plastics, polymers, metal or metallized substrates, crystalline or polycrystalline substrates, *etc*.). Of particular note are spatially addressable arrays (i.e., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-40, or an amino acid sequence encoded thereby. Methods for attaching biopolymers to, or synthesizing biopolymers on, solid support matrices, and conducting binding studies thereon are disclosed in, inter alia, U.S. Patent Nos. 5,700,637, 5,556,752, 5,744,305, 4,631,211, 5,445,934, 5,252,743, 4,713,326, 5,424,186, and 4,689,405. the disclosures of which are herein incorporated by reference in their entirety.

Addressable arrays comprising sequences first disclosed in SEQ ID NOS:1-40 can be used to identify and characterize the temporal and tissue specific expression of a gene. These addressable arrays incorporate oligonucleotide sequences of sufficient length to confer, the required specificity, yet be within the limitations of the production technology. The length of these probes is within a range of between about 8 to about 2000 nucleotides. Preferably the probes consist of 60 nucleotides and more preferably 25 nucleotides from the sequences first disclosed in SEQ ID NOS:1-40.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-40 provides detailed information about transcriptional changes involved in a specific pathway, potentially leading to the identification of novel components or gene functions that manifest themselves as novel phenotypes.

Probes consisting of sequences first disclosed in SEQ ID NOS:1-40 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-40 can be utilized in microarrays or other assay formats, to screen collections of genetic material from patients who have a particular medical condition. These investigations can also be carried out using the sequences first disclosed in SEQ ID NOS:1-40 *in silico* and by comparing previously collected genetic databases and the disclosed sequences using computer software known to those in the art.

Thus the sequences first disclosed in SEQ ID NOS:1-40 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-40. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (e.g., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relative to one or more additional sequence(s) or one or more restriction sites present in the disclosed sequence.

For oligonucleotide probes, highly stringent conditions may refer, e.g., to washing in 6x SSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation and/or as antisense primers in amplification reactions of NHP gene nucleic acid sequences. With respect to NHP gene regulation, such techniques can be used to regulate biological functions. Further, such sequences can be used as part of ribozyme and/or triple helix sequences that are also useful for NHP gene regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group including but not limited to arabinose, 2-fluoroarabinose, xylulose, and hexose.

In yet another embodiment, the antisense oligonucleotide will comprise at least one modified phosphate backbone selected from the group including, but not limited to, a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. *15*:6625-6641). The oligonucleotide is a 2'-0-methylribonucleotide (Inoue et *al.,* 1987, Nucl. Acids Res. *15*:6131-6148), or a chimeric RNA-DNA analogue (Inoue *et al*., 1987, FEBS Lett. 215:327-330). Alternatively, double stranded RNA can be used to disrupt the expression and function of a targeted NHP.

Oligonucleotides of the invention can be synthesized by standard methods known in the art, e.g. by use of an automated DNA synthesizer (such as are commercially available from Biosearch, Applied Biosystems, etc.). As examples, phosphorothioate oligonucleotides can be synthesized by the method of Stein *et al.* (1988, Nucl. Acids Res. 16:3209), and methylphosphonate oligonucleotides can be prepared by use of controlled pore glass polymer supports (Sarin et al., 1988, Proc. Natl. Acad. Sci. U.S.A. 85:7448-7451), etc.

Low stringency conditions are well-known to those of skill in the art, and will vary predictably depending on the specific organisms from which the library and the labeled sequences are derived. For guidance regarding such conditions see, for example, Sambrook *et al.,* 1989, Molecular Cloning, A Laboratory Manual (and periodic updates thereof), Cold Spring Harbor Press, NY; and Ausubel *et al.,* 1989, Current Protocols in Molecular Biology, Green Publishing Associates and Wiley Interscience, NY.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), etc., that can be used in diagnostics and pharmacogenomics.

For example, the present sequences can be used in restriction fragment length polymorphism (RFLP) analysis to identify specific individuals. In this technique, an individual's genomic DNA is digested with one or more restriction enzymes, and probed on a Southern blot to yield unique bands for identification (as generally described in U.S. Patent No. 5,272,057, incorporated herein by reference). In addition, the sequences of the present invention can be used to provide polynucleotide reagents, e.g., PCR primers, targeted to specific loci in the human genome, which can enhance the reliability of DNA-based forensic identifications by, for example, providing another "identification marker" (i.e., another DNA sequence that is unique to a particular individual). Actual base sequence information can be used for identification as an accurate alternative to patterns formed by restriction enzyme generated fragments.

Further, a NHP gene homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from, for example, human or non-human cell lines or tissue known or suspected to express an allele of a NHP gene.

The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (i.e., one known, or suspected, to express a NHP gene). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see e.g., Sambrook *et al.,* 1989, *supra.*

A cDNA encoding a mutant NHP sequence can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal sequence. Using these two primers, the product is then amplified via PCR, optionally cloned into a suitable vector, and subjected to DNA sequence analysis through methods well-known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of or known to carry a mutant NHP allele (e.g., a person manifesting a NHP-associated phenotype such as, for example, behavioral disorders, immune disorders, obesity, high blood pressure, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP sequences can then be purified and subjected to sequence analysis according to methods well-known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue may be expressed and screened using standard antibody screening techniques in conjunction with antibodies raised against a normal NHP product, as described below. (For screening techniques, see, for example, Harlow, E. and Lane, eds., 1988, "Antibodies: A Laboratory Manual", Cold Spring Harbor Press, Cold Spring Harbor, NY).

Additionally, screening can be accomplished by screening with labeled NHP fusion proteins, such as, for example, alkaline phosphatase-NHP or NHP-alkaline phosphatase fusion proteins. In cases where a NHP mutation results in an expression product with altered function (e.g., as a result of a missense or a frameshift mutation), polyclonal antibodies to NHP are likely to cross-react with a corresponding mutant NHP expression product. Library clones detected via their reaction with such labeled antibodies can be purified and subjected to sequence analysis according to methods well-known in the art.

An additional application of the described novel human polynucleotide sequences is their use in the molecular mutagenesis/evolution of proteins that are at least partially encoded by the described novel sequences using, for example, polynucleotide shuffling or related methodologies. Such approaches are described in U.S. Patent Nos. 5,830,721, 5,837,458, 6,117,679, and 5,723,323 which are herein incorporated by reference in their entirety.

The invention also encompasses (a) DNA vectors that contain any of the foregoing NHP coding sequences and/or their complements (i.e., antisense); (b) DNA expression vectors that contain any of the foregoing NHP coding sequences operatively associated with a regulatory element that directs the expression of the coding sequences (for example, baculovirus as described in U.S. Patent No. 5,869,336 herein incorporated by reference); (c) genetically engineered host cells that contain any of the foregoing NHP coding sequences operatively associated with a regulatory element that directs the expression of the coding sequences in the host cell; and (d) genetically engineered host cells that express an endogenous NHP sequence under the control of an exogenously introduced regulatory element (i.e., gene activation). As used herein, regulatory elements include, but are not limited to, inducible and non-inducible promoters, enhancers, operators and other elements known to those skilled in the art that drive and regulate expression. Such regulatory elements include but are not limited to the cytomegalovirus (hCMV) immediate early gene, regulatable, viral elements (particularly retroviral LTR promoters), the early or late promoters of SV40 adenovirus, the *lac* system, the trp system, the *TAC* system, the *TRC system,* the major operator and promoter regions of phage lambda, the control regions of fd coat protein, the promoter for 3-phosphoglycerate kinase (PGK), the promoters of acid phosphatase, and the promoters of the yeast α-mating factors.

Where, as in the present instance, some of the described NHP peptides or polypeptides are thought to be cytoplasmic or nuclear proteins (although processed forms or fragments can be secreted or membrane associated), expression systems can be engineered that produce soluble derivatives of a NHP (corresponding to a NHP extracellular and/or intracellular domains, or truncated polypeptides lacking one or more hydrophobic domains) and/or NHP fusion protein products (especially NHP-Ig fusion proteins, i.e., fusions of a NHP domain to an IgFc), NHP antibodies, and anti-idiotypic antibodies (including Fab fragments) that can be used in therapeutic applications. Preferably, the above expression systems are engineered to allow the desired peptide or polypeptide to be recovered from the culture media.

The present invention also encompasses antibodies and anti-idiotypic antibodies (including Fab fragments), antagonists and agonists of a NHP, as well as compounds or nucleotide constructs that inhibit expression of a NHP sequence (transcription factor inhibitors, antisense and ribozyme molecules, or open reading frame sequence or regulatory sequence replacement constructs), or promote the expression of a NHP (e.g., expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of a NHP in the body. The use of engineered host cells and/or animals can offer an advantage in that such systems allow not only for the identification of compounds that bind to the endogenous receptor/ligand of a NHP, but can also identify compounds that trigger NHP-mediated activities or pathways.

Finally, the NHP products can be used as therapeutics. For example, soluble derivatives such as NHP peptides/domains corresponding to NHPs, NHP fusion protein products (especially NHP-Ig fusion proteins, i.e., fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics the NHP could activate or effectively antagonize the endogenous NHP or a protein interactive therewith. Nucleotide constructs encoding such NHP products can be used to genetically engineer host cells to express such products in vivo; these genetically engineered cells function as "bioreactors" in the body delivering a continuous supply of a NHP, a NHP peptide, or a NHP fusion protein to the body. Nucleotide constructs encoding functional NHPs, mutant NHPs, as well as antisense and ribozyme molecules can also be used in "gene therapy" approaches for the modulation of NHP expression. Thus, the invention also encompasses pharmaceutical formulations and methods for treating biological disorders.

Various aspects of the invention are described in greater detail in the subsections below.

### 5.1 THE NHP SEQUENCES

The cDNA sequences and corresponding deduced amino acid sequences of the described NHPs are presented in the Sequence Listing.

Expression analysis has provided evidence that the described NHPs can be expressed in a relatively narrow range of human tissues. In addition to serine-threonine kinases, the described NHPs also share significant similarity to a range of additional kinase families, including kinases associated with signal transduction, from a variety of phyla and species. Several polymorphisms were detected in regions of sequence common to several of the described NHPs including a C/G polymorphism at the region represented by nucleotide position number 2166 of, for example, SEQ ID NO:1 which can result in a gly or ala being present at corresponding amino acid (aa) position 729 of, for example, SEQ ID NO:2, and a C/G polymorphism at the region represented by nucleotide position number 1901 of, for example, SEQ ID NO:9 which can result in a arg or thr being present at corresponding amino acid (aa) position 634 of, for example, SEQ ID NO:10.

The gene encoding the described NHPs is apparently encoded on human chromosome 6. Accordingly, the described sequences are useful for mapping and identifying the coding regions of the human genome, and for defining exon splice junctions.

The described novel human polynucleotide sequences can be used, among other things, in the molecular mutagenesis/evolution of proteins that are at least partially encoded by the described novel sequences using, for example, polynucleotide shuffling or related methodologies. Such approaches are described in U.S. Patent Nos. 5,830,721 and 5,837,458 which are herein incorporated by reference in their entirety.

NHP gene products can also be expressed in transgenic animals. Animals of any species, including, but not limited to, worms, mice, rats, rabbits, guinea pigs, pigs, micro-pigs, birds, goats, and non-human primates, e.g., baboons, monkeys, and chimpanzees may be used to generate NHP transgenic animals.

Any technique known in the art may be used to introduce a NHP transgene into animals to produce the founder lines of transgenic animals. Such techniques include, but are not limited to pronuclear microinjection (Hoppe, P.C. and Wagner, T.E., 1989, U.S. Patent No. 4,873,191); retrovirus-mediated gene transfer into germ lines (Van der Putten *et al*., 1985, Proc. Natl. Acad. Sci., USA 82:6148-6152); gene targeting in embryonic stem cells (Thompson *et al*., 1989, Cell *56*:313-321); electroporation of embryos (Lo, 1983, Mol Cell. Biol. 3:1803-1814); and sperm-mediated gene transfer (Lavitrano *et al.,* 1989, Cell 57:717-723); *etc*. For a review of such techniques, see Gordon, 1989, Transgenic Animals, Intl. Rev. Cytol. 115:171-229, which is incorporated by reference herein in its entirety.

The present invention provides for transgenic animals that carry the NHP transgene in all their cells, as well as animals which carry the transgene in some, but not all their cells, i.e., mosaic animals or somatic cell transgenic animals. The transgene may be integrated as a single transgene or in concatamers, e.g., head-to-head tandems or head-to-tail tandems. The transgene may also be selectively introduced into and activated in a particular cell-type by following, for example, the teaching of Lasko *et al*., 1992, Proc. Natl. Acad. Sci. USA 89:6232-6236. The regulatory sequences required for such a cell-type specific activation will depend upon the particular cell-type of interest, and will be apparent to those of skill in the art.

When it is desired that a NHP transgene be integrated into the chromosomal site of the endogenous NHP gene, gene targeting is preferred. Briefly, when such a technique is to be utilized, vectors containing some nucleotide sequences homologous to the endogenous NHP gene are designed for the purpose of integrating, via homologous recombination with chromosomal sequences, into and disrupting the function of the nucleotide sequence of the endogenous NHP gene (i.e., "knockout" animals).

The transgene can also be selectively introduced into a particular cell-type, thus inactivating the endogenous NHP gene in only that cell-type, by following, for example, the teaching of Gu et al., 1994, Science, 265:103-106. The regulatory sequences required for such a cell-type specific inactivation will depend upon the particular cell-type of interest, and will be apparent to those of skill in the art.

Once transgenic animals have been generated, the expression of the recombinant NHP gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques which include but are not limited to Northern blot analysis of tissue samples obtained from the animal, in situ hybridization analysis, and RT-PCR. Samples of NHP gene-expressing tissue, may also be evaluated immunocytochemically using antibodies specific for the NHP transgene product.

### 5.2 NHPS AND NHP POLYPEPTIDES

NHP products, polypeptides, peptide fragments, mutated, truncated, or deleted forms of the NHPs, and/or NHP fusion proteins can be prepared for a variety of uses. These uses include, but are not limited to, the generation of antibodies, as reagents in diagnostic assays, the identification of other cellular gene products related to the NHP, as reagents in assays for screening for compounds that can be used as pharmaceutical reagents useful in the therapeutic treatment of mental, biological, or medical disorders and disease.

The Sequence Listing discloses the amino acid sequences encoded by the described NHP-encoding polynucleotides. The NHPs display initiator methionines that are present in DNA sequence contexts consistent with eucaryotic translation initiation sites. The NHPs do not display consensus signal sequences which indicates that they may be cytoplasmic or possibly nuclear proteins, although they may also be secreted or membrane associated.

The NHP amino acid sequences of the invention include the amino acid sequences presented in the Sequence Listing as well as analogues and derivatives thereof. Further, corresponding NHP homologues from other species are encompassed by the invention. In fact, any NHP protein encoded by the NHP nucleotide sequences described above are within the scope of the invention, as are any novel polynucleotide sequences encoding all or any novel portion of an amino acid sequence presented in the Sequence Listing. The degenerate nature of the genetic code is well-known, and, accordingly, each amino acid presented in the Sequence Listing, is generically representative of the well-known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell et al. eds., Scientific American Books, New York, NY, herein incorporated by reference) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and modify a NHP substrate, or the ability to effect an identical or complementary downstream pathway, or a change in cellular metabolism (e.g., proteolytic activity, ion flux, tyrosine phosphorylation, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent expression product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where the NHP peptide or polypeptide can exist, or has been engineered to exist, as a soluble or secreted molecule, the soluble NHP peptide or polypeptide can be recovered from the culture media. Such expression systems also encompass engineered host cells that express a NHP, or functional equivalent, in situ. Purification or enrichment of a NHP from such expression systems can be accomplished using appropriate detergents and lipid micelles and methods well-known to those skilled in the art. However, such engineered host cells themselves may be used in situations where it is important not only to retain the structural and functional characteristics of the NHP, but to assess biological activity, e.g., in certain drug screening assays.

The expression systems that may be used for purposes of the invention include, but are not limited to, microorganisms such as bacteria (e.g., *E. coli, B. subtilis*) transformed with recombinant bacteriophage DNA, plasmid DNA or cosmid DNA expression vectors containing NHP nucleotide sequences; yeast *(e.g., Saccharomyces, Pichia)* transformed with recombinant yeast expression vectors containing NHP nucleotide sequences; insect cell systems infected with recombinant virus expression vectors (e.g., baculovirus) containing NHP nucleotide sequences; plant cell systems infected with recombinant virus expression vectors (*e*.*g*., cauliflower mosaic virus, CaMV; tobacco mosaic virus, TMV) or transformed with recombinant plasmid expression vectors (e.g., Ti plasmid) containing NHP nucleotide sequences; or mammalian cell systems (e.g., COS, CHO, BHK, 293, 3T3) harboring recombinant expression constructs containing NHP nucleotide sequences and promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, or for raising antibodies to a NHP, vectors that direct the expression of high levels of fusion protein products that are readily purified may be desirable. Such vectors include, but are not limited, to the *E. coli* expression vector pUR278 (Ruther *et* al., 1983, EMBO J. 2:1791), in which a NHP coding sequence may be ligated individually into the vector in frame with the *lacZ* coding region so that a fusion protein is produced; pIN vectors (Inouye & Inouye, 1985, Nucleic Acids Res. 13:3101-3109; Van Heeke & Schuster, 1989, J. Biol. Chem. *264*:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target expression product can be released from the GST moiety.

In an insect system, Autographa *californica* nuclear polyhedrosis virus (AcNPV) is used as a vector to express foreign polynucleotide sequences. The virus grows in *Spodoptera frugiperda* cells. A NHP coding sequence can be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (i.e., virus lacking the proteinaceous coat coded for by the polyhedrin gene). These recombinant viruses are then used to infect Spodoptera frugiperda cells in which the inserted sequence is expressed (e.g., see Smith et al., 1983, J. Virol. 46: 584; Smith, U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, e.g., the late promoter and tripartite leader sequence. This chimeric sequence may then be inserted in the adenovirus genome by in vitro or in vivo recombination. Insertion in a non-essential region of the viral genome (e.g., region E1 or E3) will result in a recombinant virus that is viable and capable of expressing a NHP product in infected hosts (e.g., See Logan & Shenk, 1984, Proc. Natl. Acad. Sci. USA 81:3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter *et al.,* 1987, Methods in Enzymol. 153:516-544).

In addition, a host cell strain may be chosen that modulates the expression of the inserted sequences, or modifies and processes the expression product in the specific fashion desired. Such modifications (*e.g*., glycosylation) and processing (e.g., cleavage) of protein products may be important for the function of the protein. Different host cells have characteristic and specific mechanisms for the post-translational processing and modification of proteins and expression products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the expression product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines that stably express the NHP sequences described above can be engineered. Rather than using expression vectors which contain viral origins of replication, host cells can be transformed with DNA controlled by appropriate expression control elements (e.g., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler *et al*., 1977, Cell *11*:223), hypoxanthine-guanine phosphoribosyltransferase (Szybalska and Szybalski, 1962, Proc. Natl. Acad. Sci. USA 48:2026), and adenine phosphoribosyltransferase (Lowy *et al.,* 1980, Cell *22*:817) genes, which can be employed in tk⁻, hgprt⁻ or aprt⁻ cells, respectively. Also, antimetabolite resistance can be used as the basis of selection for the following genes: dhfr, which confers resistance to methotrexate (Wigler *et al*., 1980, Proc. Natl. Acad. Sci. USA *77*:3567; O'Hare *et al*., 1981, Proc. Natl. Acad. Sci. USA *78*:1527); gpt, which confers resistance to mycophenolic acid (Mulligan and Berg, 1981, Proc. Natl. Acad. Sci. USA 78:2072); neo, which confers resistance to the aminoglycoside G-418 (Colberre-Garapin *et al*., 1981, J. Mol. Biol. 150:1); and hygro, which confers resistance to hygromycin (Santerre *et al*., 1984, Gene 30:147).

Alternatively, any fusion protein can be readily purified by utilizing an antibody specific for the fusion protein being expressed. For example, a system described by Janknecht et al. allows for the ready purification of non-denatured fusion proteins expressed in human cell lines (Janknecht, et *al.,* 1991, Proc. Natl. Acad. Sci. USA 88:8972-8976). In this system, the sequence of interest is subcloned into a vaccinia recombination plasmid such that the sequence's open reading frame is translationally fused to an amino-terminal tag consisting of six histidine residues, Extracts from cells infected with recombinant vaccinia virus are loaded onto Ni²⁺-nitriloacetic acid-agarose columns and histidine-tagged proteins are selectively eluted with imidazole-containing buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively targeting of NHP or its nucleic acid sequence might be achieved using liposome or lipid complex based delivery systems. Such technologies are described in "Liposomes:A Practical Approach", New, R.R.C., ed., Oxford University Press, New York and in U.S. Patent Nos. 4,594,595, 5,459,127, 5,948,767 and 6,110,490 and their respective disclosures which are herein incorporated by reference in their entirety. Additionally embodied are novel protein constructs engineered in such a way that they facilitate transport of the NHP to the target site or desired organ, where they cross the cell membrane and/or the nucleus where the NHP can exert its functional activity. This goal may be achieved by coupling of the NHP to a cytokine or other ligand that provides targeting specificity, and/or to a protein transducing domain (see generally U.S. applications Ser. No. 60/111,701 and 60/056,713, both of which are herein incorporated by reference, for examples of such transducing sequences) to facilitate passage across cellular membranes and can optionally be engineered to include nuclear localization.

### 5.3 ANTIBODIES TO NHP PRODUCTS

Antibodies that specifically recognize one or more epitopes of a NHP, or epitopes of conserved variants of a NHP, or peptide fragments of a NHP are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention can be used, for example, in the detection of NHP in a biological sample and may, therefore, be utilized as part of.a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP expression product. Additionally, such antibodies can be used in conjunction gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with the NHP, a NHP peptide (e.g., one corresponding to a functional domain of a NHP), truncated NHP polypeptides (NHP in which one or more domains have been deleted), functional equivalents of the NHP or mutated variant of the NHP. Such host animals may include but are not limited to pigs, rabbits, mice, goats, and rats, to name but a few. Various adjuvants may be used to increase the immunological response, depending on the host species, including, but not limited to, Freund's adjuvant (complete and incomplete), mineral salts such as aluminum hydroxide or aluminum phosphate, chitosan, surface active substances such as lysolecithin, pluronic polyols, polyanions, peptides, oil emulsions, and potentially useful human adjuvants such as BCG (bacille Calmette-Guerin) and *Corynebacterium parvum*. Alternatively, the immune response could be enhanced by combination and or coupling with molecules such as keyhole limpet hemocyanin, tetanus toxoid, diphtheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature 256:495-497; and U.S. Patent No. 4,376,110), the human B-cell hybridoma technique (Kosbor *et al.,* 1983, Immunology Today *4*:72; Cole *et al.,* 1983, Proc. Natl. Acad. Sci. USA 80:2026-2030), and the EBV-hybridoma technique (Cole *et al*., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or *in vivo.* Production of high titers of mAbs in vivo makes this the presently preferred method of production.

In addition, techniques developed for the production of "chimeric antibodies" (Morrison *et al.,* 1984, Proc. Natl. Acad. Sci., *81*:6851-6855; Neuberger *et al*., 1984, Nature, *312*:604-608; Takeda *et al.,* 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patent Nos. 6,075,181 and 5,877,397 and their respective disclosures which are herein incorporated by reference in their entirety. Also encompassed by the present invention is the use of fully humanized monoclonal antibodies as described in US Patent No. 6,150,584 and respective disclosures which are herein incorporated by reference in their entirety.

Alternatively, techniques described for.the production of single chain antibodies (U.S. Patent 4,946,778; Bird, 1988, Science *242*:423-426; Huston *et al*., 1988, Proc. Natl. Acad. Sci. USA *85*:5879-5883; and Ward *et al*., 1989, Nature *341*:544-546) can be adapted to produce single chain antibodies against NHP expression products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse *et al.,* 1989, Science, 246:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well-known to those skilled in the art. (See, e.g., Greenspan & Bona, 1993, FASEB J 7(5):437-444; and Nissinoff, 1991, J. Immunol. 147(8):2429-2438). For example antibodies which bind to a NHP domain and competitively inhibit the binding of NHP to its cognate receptor/ligand can be used to generate anti-idiotypes that "mimic" the NHP and, therefore, bind, activate, or neutralize a NHP, NHP receptor, or NHP ligand. Such anti-idiotypic antibodies or Fab fragments of such anti-idiotypes can be used in therapeutic regimens involving a NHP-mediated pathway.

Additionally given the high degree of relatedness of mammalian NHPs, the presently described knock-out mice (having never seen NHP, and thus never been tolerized to NHP) have a unique utility, as they can be advantageously applied to the generation of antibodies against the disclosed mammalian NHP (i.e., NHP will be immunogenic in NHP knock-out animals).

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims. All cited publications, patents, and patent applications are herein incorporated by reference in their entirety.

## Claims

1. An isolated nucleic acid molecule comprising a nucleotide sequence encoding an amino acid sequence drawn from the group consisting of: SEQ ID NOS: 2, 4, 6, 8, 10, 12, 14, 16, 18, 20, 22, 24, 26, 28, 30, 32, 34, 36, 38 and 40.

2. An isolated nucleic acid molecule comprising a nucleotide sequence that:
(a) encodes the amino acid sequence shown in SEQ ID NO: 2; and
(b) hybridizes under stringent conditions to the nucleotide sequence of SEQ ID NO: 1 or the complement thereof.

3. An isolated nucleic acid molecule comprising a nucleotide sequence encoding the amino acid sequence shown in SEQ ID NO: 2.

4. An isolated nucleic acid molecule comprising a nucleotide sequence encoding the amino acid sequence shown in SEQ ID NO: 8.

5. A polypeptide encoded by a nucleic acid as claimed in any one of claims 1 to 4.

6. A polypeptide comprising at least 12 amino acids in a sequence first disclosed in SEQ ID NO: 2, 4, 6, 8, 10, 12, 14, 16, 18, 20, 22, 24, 26, 28, 30, 32, 34, 36, 38 and 40.

7. An antibody that binds to a polypeptide as claimed in claim 5 or claim 6, or a fragment or a derivative of said antibody, for example, a polyclonal or monoclonal antibody, a single chain antibody, a Fab fragment, a F(ab')2, a fragment produced by a Fab expression library, or an anti-idiotypic (anti-Id) antibody, or an epitope binding fragment of any of said antibodies or fragments.

8. A host cell that comprises a nucleic acid as claimed in any one of claims 1 to 4 and is capable of expressing the polypeptide encoded by said nucleic acid, optionally on the surface of said host cell.

9. An animal that is capable of expressing a nucleic acid as claimed in any one of claims 1 to 4, or that is an optionally conditional "knock-out" animal that does not express a polypeptide as claimed in claim 5 or claim 6.

10. Use of a nucleic acid as claimed in any one of claims 1 to 4, a polypeptide as claimed in claim 5 or claim 6, an antibody as claimed in claim 7, a host cell as claimed in claim 8 or an animal as claimed in claim 9, in a method for screening compounds for the ability to modulate the expression of a nucleic acid as claimed in any one of claims 1 to 4 or a polypeptide as claimed in claim 5 or claim 6, or that modulates the activity of a polypeptide as claimed in claim 5 or claim 6, for example, for high throughput screening of combinatorial libraries.
